(19) Europäisches Patentamt
European Patent Office
Office européen des brevets

(11) EP 1 731 523 A1

(12) EUROPEAN PATENT APPLICATION
published in accordance with Art. 158(3) EPC

(43) Date of publication:
13.12.2006 Bulletin 2006/50

(21) Application number: 05728421.8

(22) Date of filing: 31.03.2005

(51) Int Cl.:
*C07D 513/04* (2006.01) *A61K 31/519* (2006.01)
*A61P 13/08* (2006.01) *A61P 35/00* (2006.01)
*A61P 43/00* (2006.01)

(86) International application number:
PCT/JP2005/006832

(87) International publication number:
WO 2005/095419 (13.10.2005 Gazette 2005/41)

(84) Designated Contracting States:
AT BE BG CH CY CZ DE DK EE ES FI FR GB GR HU IE IS IT LI LT LU MC NL PL PT RO SE SI SK TR

(30) Priority: 01.04.2004 JP 2004109393

(71) Applicant: Takeda Pharmaceutical Company Limited
Osaka-shi,
Osaka 541-0045 (JP)

(72) Inventors:
- SUGIHARA, Yoshihiro
TAKEDA PHARMACEUTICAL COMP LTD
Osaka-shi, Osaka 5328686 (JP)
- KAWAKITA, Youichi,
TAKEDA PHARMACEUTICAL COMP. LTD
Osaka-shi, Osaka 5328686 (JP)

(74) Representative: Rickard, Timothy Mark Adrian
Takeda Euro IP Department
Arundel Great Court
2 Arundel Street
London
WC2R 3DA (GB)

# (54) THIAZOLOPYRIMIDINE DERIVATIVE

(57) The present invention provides a compound represented by the following Formula:

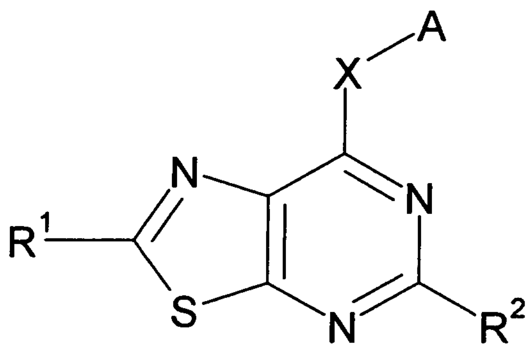

(I)

wherein A is an aryl group or a heteroaryl group, each of which may be substituted; $R^1$ is a group which is bonded through carbon; $R^2$ is hydrogen or an aliphatic hydrocarbon group; and X is $-NR^3-$, -O-, -S-, -SO-, $-SO_2-$, or $-CHR^3-$(wherein $R^3$ is hydrogen or an aliphatic hydrocarbon group), or a salt thereof, or a prodrug thereof, which has growth factor receptor tyrosine kinase inhibitory activity and low toxicity, and is useful for prevention and treatment of cancer, and thus can be sufficiently used as a medicine.

EP 1 731 523 A1

## Description

### Technical Field

The present invention relates to a thiazolo[5,4-d]pyrimidine derivative which has growth factor receptor tyrosine kinase inhibitory activity and is useful for prevention and treatment of cancer, a process for producing the same, and use thereof.

### Background Art

Cell growth factor and growth factor receptor genes are known as proto-oncogene, and take important role in the pathology of human tumors. Epidermal growth factor receptor family (erbB) including EGFR, HER2, HER3, and HER4, is a type I receptor tyrosine kinase. The erbB family is expressed in the various cell groups, and deeply involved in the regulation of cell proliferation, cell differentiation, and cell-death inhibition (apoptosis suppression). For example, it has been realized that overexpression of EGFR and HER2, and constitutive activation of receptor, experimentally transform cells.

Further, it has been clearly understood that overexpression and simultaneous expression of both receptors, are adverse prognostic factors in various cancer patients.

Many of peptide ligand such as EGF, TGFα, or the like, is conjugated with these receptors, and homo or hetero dimerization of the receptor is promoted by the ligand conjugation. This induces an increase in kinase activity from auto-phosphorylation, or trans-phosphorylation of the receptor, and generates an activation of downstream signaling pathway (MAPK, Akt) through a conjugated protein to specific phosphorylated tyrosine residues. This is the main part of the receptor activity in the above-mentioned cell proliferation, cell differentiation, cell-death inhibition or the like, and also is considered to be the reason for a overexpression of receptor and development of malignant cancer by the local increase in the ligand concentration, in cancers.

It has been realized that EGRF and HER2 are overexpressed in many cancers such as, breast cancer (20-30%), ovarian cancer (20-40%), nonsmall cell lung cancer (30-60%), colon cancer (40-80%), prostate cancer (10-60%), bladder cancer (30-60%), kidney cancer (20-40%), or the like. Further, there is a mutuality between the expression of receptor and prognosis, and the expression of receptor is the adverse prognostic factor in breast cancer, nonsmall cell lung cancer, or the like.

Recently, a clinical use of humanized anti-HER2 antibody (Trastuzumab) for HER2 overexpression breast cancer, a clinical trial of anti-EGFR antibody, and clinical trials of several inhibitors for low molecular receptor enzyme, are showing a possible cancer therapeutic agent of drug for the HER2 receptor or EGFR receptors. The drug shows a tumor proliferation inhibitory action in clinical or non-clinical trials, and at this time, it has been realized to induce the inhibition of receptor enzyme activity and suppression of downstream signaling pathway. Therefore, inhibition of EGFR or HER2 kinase, or inhibition of activation thereof, is effective as a therapy for cancers.

As a compound that inhibits receptor type tyrosine kinases including HER2/EGFR kinase, fused heterocyclic compounds (for example, refer to WO97/13771, WO98/02437, WO96/40142, WO00/44728, US2004/0242604), quinazoline derivatives (for example, refer to WO02/02552, WO01/98277, WO03/049740, WO03/050108), thienopyrimidine derivatives (for example, refer to WO03/053446), aromatic azole derivatives (for example, refer to WO98/003648, WO01/077107, WO03/031442), or the like, has been realized. However, there is no HER2 kinase inhibitory substance for a cancer therapeutic agent which is placed on the market. About thiazolo[5,4-d]pyrimidine derivatives, there are several documents for synthesis of the compound (for example, refer to Chemical & Pharmaceutical Bulletin 6, 352-355, 1958, Liebigs Annalen der Chemie, 1255-1261, 1986), however there are no documents disclosing the tyrosine kinase inhibitory action or use of prophylactic and/or therapeutic agent for cancer.

### Disclosure of the Invention

It is an object of the present invention to provide a compound which has excellent tyrosine kinase inhibitory action, and low toxicity, and thus can be sufficiently used as a medicine.

The inventors of the invention conducted keen investigation, and as a result, found that a compound represented by the following Formula (Ia) or a salt thereof (hereinafter, may be simply referred to as Compound (Ia) in the present specification) has excellent tyrosine kinase inhibitory action, and in the further investigation, completed the invention. Further, in the Compound (Ia), a compound represented by the following Formula (I) or a salt thereof (hereinafter, may be simply referred to as Compound (I) in the present specification) is a novel compound.

Thus, the invention provides:

[1] a compound represented by Formula:

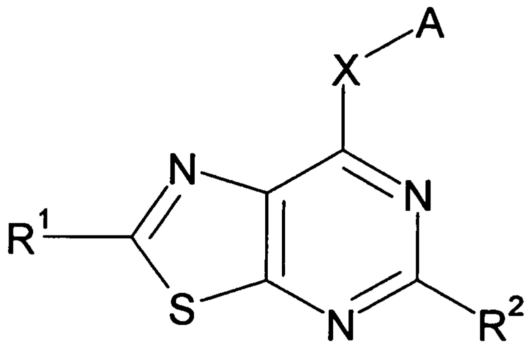

(I)

wherein A is an aryl group or a heteroaryl group, each of which may be substituted; $R^1$ is a group which is bonded through carbon; $R^2$ is a hydrogen atom or an aliphatic hydrocarbon group; and X is $-NR^3-$, -O-, -S-, -SO-, $-SO_2-$, or $-CHR^3-$ (wherein $R^3$ is a hydrogen atom or an aliphatic hydrocarbon group),
or a salt thereof (provided that 2-methyl-N-phenyl[1,3]thiazolo[5,4-d]pyrimidin-7-amine, N-(4-fluorophenyl)-2-methyl [1,3]thiazolo[5,4-d]pyrimidin-7-amine, 2,5-dimethyl-N-phenyl[1,3]thiazolo[5,4-d]pyrimidin-7-amine, N-(4-chlorophenyl)-2,5-dimethyl[1,3]thiazolo[5,4-d]pyrimidin-7-amine, N-(4-fluorophenyl)-2,5-dimethyl[1,3]thiazolo[5,4-d]pyrimidin-7-amine, 2,5-dimethyl-N-(4-methylphenyl)[1,3]thiazolo[5,4-d]pyrimidin-7-amine, 2,5-dimethyl-N-[3-(trifluoromethyl)phenyl][1,3]thiazolo[5,4-d]pyrimidin-7-amine, N-(3,4-dimethylphenyl)-2,5-dimethyl[1,3]thiazolo[5,4-d]pyrimidin-7-amine, N-(3,5-dimethylphenyl)-2,5-dimethyl[1,3]thiazolo[5,4-d]pyrimidin-7-amine, and N-(2,6-dimethylphenyl)-2,5-dimethyl[1,3]thiazolo[5,4-d]pyrimidin-7-amine are excluded);
[2] a prodrug of the compound according to [1] above;
[3] the compound according to [1] above, wherein A is an aryl group which is substituted with a group represented by Formula: -Y-B (wherein, Y is a single bond or a spacer, and B is an aryl group or a heterocyclic group, each of which may be substituted), and which may be further substituted;
[4] the compound according to [1] above, wherein $R^1$ is a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, or a heterocyclyl-$C_{1-4}$ alkyl group, each of which may be substituted;
[5] the compound according to [1] above, wherein $R^2$ is a hydrogen atom;
[6] the compound according to [1] above, wherein A is an aryl group which is substituted with a group represented by Formula: -Y-B (wherein, Y is a single bond or a spacer, and B is an aryl group or a heterocyclic group, each of which may be substituted), and which may be further substituted; $R^1$ is a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, or a heterocyclyl-$C_{1-4}$ alkyl group, each of which may be substituted; and $R^2$ is a hydrogen atom;
[7] the compound according to [1] above, wherein A is:

(i) an aryl group which is substituted with a group having an aromatic ring, or (ii) a heteroaryl group which may be substituted;

[8] a compound represented by Formula:

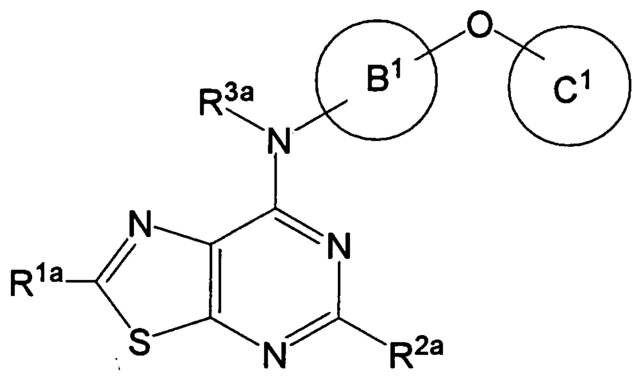

(I-I)

wherein $B^1$ is a $C_{6-18}$ aryl ring which may be substituted; $C^1$ is a $C_{6-18}$ aryl group which may be substituted; $R^{1a}$ is a group which is bonded through carbon; $R^{2a}$ is a hydrogen atom or an aliphatic hydrocarbon group; and $R^{3a}$ is a hydrogen atom or an aliphatic hydrocarbon group,

or a salt thereof;

[9] a compound represented by Formula:

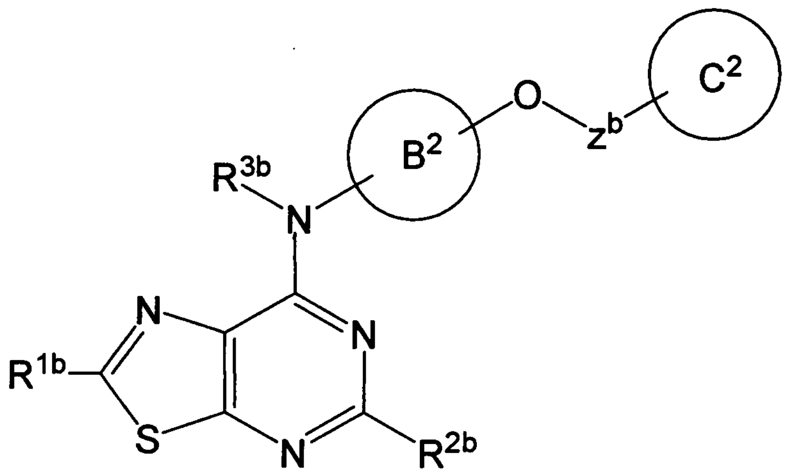

(I-II)

wherein $B^2$ is a $C_{6-18}$ aryl ring which may be substituted; $C^2$ is a $C_{6-18}$ aryl group which may be substituted; $R^{1b}$ is a group which is bonded through carbon; $R^{2b}$ is a hydrogen atom or an aliphatic hydrocarbon group; $R^{3b}$ is a hydrogen atom or an aliphatic hydrocarbon group; and $Z^b$ is a $C_{1-4}$ alkylene group which may be substituted,

or a salt thereof;

[10] a compound represented by Formula:

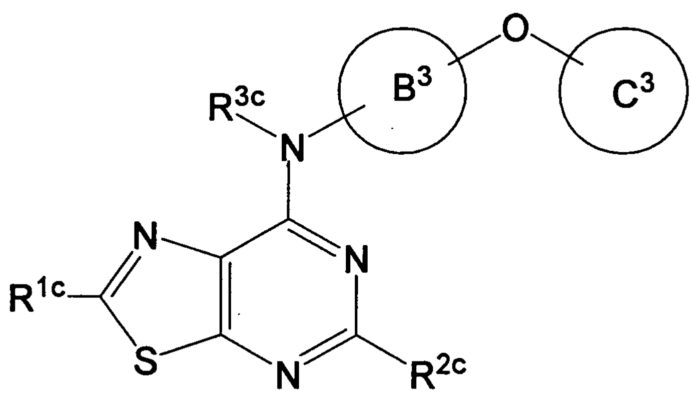

(I-III)

wherein $B^3$ is a $C_{6-18}$ aryl ring which may be substituted; $C^3$ is a heterocyclic group which may be substituted; $R^{1c}$ is a group which is bonded through carbon; $R^{2c}$ is a hydrogen atom or an aliphatic hydrocarbon group; and $R^{3c}$ is a hydrogen atom or an aliphatic hydrocarbon group,
or a salt thereof;
[11] a compound represented by Formula:

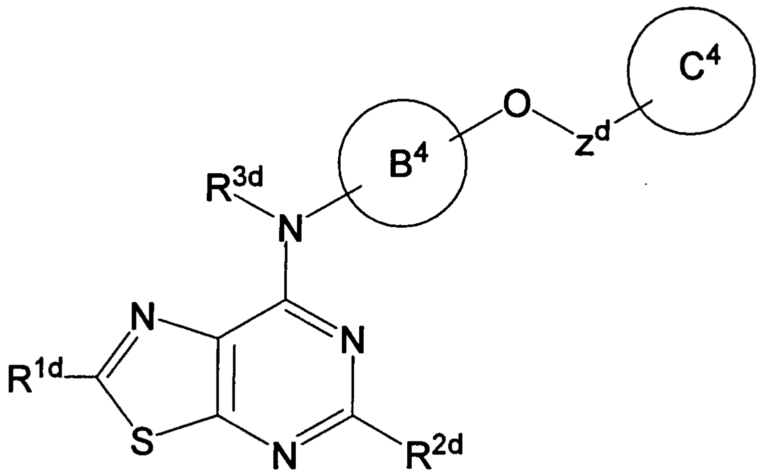

(I-IV)

wherein $B^4$ is a $C_{6-18}$ aryl ring which may be substituted; $C^4$ is a heterocyclic group which may be substituted; $R^{1d}$ is a group which is bonded through carbon; $R^{2d}$ is a hydrogen atom or an aliphatic hydrocarbon group; $R^{3d}$ is a hydrogen atom or an aliphatic hydrocarbon group; and $Z^d$ is a $C_{1-4}$ alkylene group which may be substituted,
or a salt thereof;
[12] the compound according to [1] above, wherein $R^1$ is a $C_{6-18}$ aryl group which may be substituted, or a $C_{6-18}$ aryl-$C_{1-4}$ alkyl group which may be substituted;
[13] the compound according to [1] above, wherein
A is a $C_{6-18}$ aryl group which may be substituted with 1 to 5 substituents selected from the group consisting of:

(i) a $C_{6-18}$ aryl-oxy group which may be substituted with substituent(s) selected from the group consisting of:

(a) a halogen atom,
(b) $C_{1-4}$alkyl which may be halogenated, and
(c) $C_{1-4}$ alkyloxy which may be halogenated,

(ii) a $C_{6-18}$ aryl-$C_{1-4}$ alkyl-oxy group (said $C_{1-4}$ alkyl may be substituted with $C_{1-4}$ alkyl which may be halogenated) which may be substituted with substituent(s) selected from the group consisting of:

(a) a halogen atom,
(b) $C_{1-4}$ alkyl which may be halogenated, and
(c) cyano,

(iii) a 5- to 8- membered heterocyclyl-oxy group having 1 to 3 heteroatoms selected from nitrogen, oxygen and sulfur atoms, which may be substituted with $C_{1-4}$ alkyl,
(iv) a 5- to 8- membered heterocyclyl-$C_{1-3}$ alkyl-oxy group having 1 to 3 heteroatoms selected from nitrogen, oxygen and sulfur atoms,
(v) a $C_{6-8}$ aryl-$C_{1-4}$ alkyl group,
(vi) a halogen atom,
(vii) a $C_{1-4}$ alkyl group which may be substituted with substituent(s) selected from the group consisting of:

(a) a halogen atom,
(b) hydroxy, and
(c) $C_{1-4}$ alkyloxy, and

(viii) a $C_{1-4}$ alkyloxy group,

$R^1$ is:

(i) a $C_{1-8}$ alkyl group which may be substituted with substituent(s) selected from the group consisting of:

(a) hydroxy,
(b) $-NR^8-(CH_2)_n-O-C_{1-4}$ alkyl,
(C) $-NR^8-CO-C_{1-4}$ alkyl, and
(d) $-NR^8-CO-(CH_2)_n-O-C_{1-4}$ alkyl,
wherein n is an integer from 1 to 4, and $R^8$ is a hydrogen atom or a $C_{1-4}$ alkyl group,

(ii) a $C_{3-8}$ cycloalkyl group,
(iii) a $C_{6-18}$ aryl group which may be substituted with substituent(s) selected from the group consisting of:

(a) a halogen atom,
(b) nitro,
(c) $-NR^6R^7$,
(d) $-O-C_{1-4}$ alkyl,
(e) $-(CH_2)_m-NR^8-(CH_2)_n-OH$,
(f) $-(CH_2)_m-NR^8-(CH_2)_n-O-C_{1-4}$ alkyl,
(g)$-(CH_2)_m-NR^8-(CH_2)_n-SO_2-C_{1-4}$alkyl,
(h) $-(CH_2)_m-NR^8-(CH_2)_n-NR^8-CO-C_{1-4}$ alkyl,
(i)$-(CH_2)_m-O-(CH_2)_n-O-C_{1-4}$ alkyl,
(j) $-(CH_2)_m-NR^8-CO-(CH_2)_n-OH$,
(k) $-(CH_2)_m-NR^8-CO-(CH_2)_n-O-C_{1-4}$ alkyl,
(l) $-(CH_2)_m-NR^8-CO-(CH_2)_n-SO_2-C_{1-4}$ alkyl,
(m) $-(CH_2)_m-NR^8-CO-NR^8-(CH_2)_n-SO_2-C_{1-4}$ alkyl,
(n) $-(CH_2)_m-NR^8-SO_2-C_{1-4}$ alkyl,
(o) $-(CH_2)_m-NR^8-CO-(CH_2)_n-O-(CH_2)_n-O-C_{1-4}$ alkyl,
(p) $-(CH_2)_m-NR^8-CO-(CH_2)_n-NR^6R^7$, and
(q) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$,
wherein m is an integer from 0 to 4, n is an integer from 1 to 4, and when m or n is two or more, a part of $-CH_2-CH_2-$ moieties thereof may be replaced by -CH=CH-; $R^6$ and $R^7$ are identical or different and are each a hydrogen atom or a $C_{1-4}$ alkyl group; and $R^8$ is a hydrogen atom, a $C_{1-4}$ alkyl group, or a $C_{1-4}$ alkyl-carbonyl group,

(iv) a $C_{6-18}$ aryl-$C_{1-4}$ alkyl group which may be substituted with substituent(s) selected from the group consisting of:

(a) nitro, and
(b) $-O-C_{1-4}$ alkyl,

(v) a carbamoyl group which may be substituted with substituent(s) selected from the group consisting of:

(a) $-(CH_2)_n-OH$,
(b) $-(CH_2)_n-NR^6R^7$,
(c) $-(CH_2)_n-O-C_{1-4}$ alkyl,
(d) $-(CH_2)_m-O-(CH_2)_n-OH$,
(d) $-(CH_2)_n-NR^8-(CH_2)_n-SO_2-C_{1-4}$ alkyl,
(e) $-(CH_2)_n-NR^8-CO-(CH_2)_n-OH$,
(f) $-(CH_2)_n-NR^8-CO-(CH_2)_n-NR^6R^7$, and
(g) $-(CH_2)_n-NR^8-CO-(CH_2)_n$-heterocyclic group (for example, a 5- to 8- membered heterocyclic group having 1 to 3 heteroatoms selected from nitrogen, oxygen and sulfur atoms),

wherein m is an integer from 0 to 4, n is an integer from 1 to 4, and when m or n is two or more, a part of

$-CH_2-CH_2-$ moieties thereof may be replaced by -CH=CH-; $R^6$ and $R^7$ identical or different and are each are a hydrogen atom or a $C_{1-4}$ alkyl group which may have hydroxy; and
$R^8$ is a hydrogen atom or a $C_{1-4}$ alkyl group,
(vi) a 5- to 8- membered cyclic amino-carbonyl group which may be substituted with substituent(s) selected from the group consisting of:

(a) hydroxyl,
(b) $-(CH_2)_n-NR^6R^7$, and
(c) -CO- $(CH_2)_n-O-C_{1-4}$ alkyl,

wherein n is an integer from 1 to 4, and when n is two or more, a part of $-CH_2-CH_2-$ moieties thereof may be replaced by -CH=CH-; and $R^6$ and
$R^7$ are identical or different and are each a hydrogen atom or a $C_{1-4}$ alkyl group,
(vii) a 5- to 8- membered heterocyclic group having 1 to 3 heteroatoms selected from nitrogen, oxygen and sulfur atoms, which may be substituted with substituent(s) selected from the group consisting of:

(a) $-(CH_2)_m-NR^8-CO-(CH_2)_n-OH$, and
(b) $-(CH_2)_m-NR^8-CO-(CH_2)_n-SO_2-C_{1-4}$ alkyl,

wherein m is an integer from 0 to 4, n is an integer from 1 to 4, and $R^8$ is a hydrogen atom
or a $C_{1-4}$ alkyl group, or
(viii) a 5- to 8- membered heterocyclyl-$C_{1-4}$ alkyl group having 1 to 3 heteroatoms selected from nitrogen, oxygen and sulfur atoms (e.g., piperidinomethyl), which may be substituted with hydroxy,

$R^2$ is a hydrogen atom,
X is $-NR^3-$, and
$R^3$ is a hydrogen atom or a $C_{1-3}$ alkyl group;
[14] the compound according to [8] above, wherein
$B^1$ is a benzene ring which may be substituted with halogen atom(s),
$C^1$ is a $C_{6-18}$ aryl group (e.g., a phenyl group) which may be substituted with substituent(s) selected from the group consisting of:

(a) a halogen atom,
(b) $C_{1-4}$ alkyl which may be halogenated, and
(c) $C_{1-4}$ alkyloxy which may be halogenated,

$R^{1a}$ is:

(i) a $C_{6-18}$ aryl group which may be substituted with $-(CH_2)_m-NR^8-(CH_2)_n-OH$ [wherein m is an integer from 0 to 4 (preferably, 1 to 3), n is an integer from 1 to 4, and $R^8$ is a hydrogen atom or a $C_{1-4}$ alkyl group], or
(ii) a carbamoyl group which may be substituted with $-(CH_2)_m-O-(CH_2)_n-OH$ [wherein m is an integer from 0 to 4 (preferably, 1 to 3), and n is an integer from 1 to 4],

$R^{2a}$ is a hydrogen atom, and
$R^{3a}$ is a hydrogen atom;
[15] the compound according to [9] above, wherein
$B^2$ is a benzene ring which may be substituted with substituent(s) selected from the group consisting of:

(a) a halogen atom,
(b) $C_{1-4}$ alkyl which may be substituted with hydroxy or $C_{1-4}$ alkyloxy, and
(c) $C_{1-4}$ alkyloxy,

$C^2$ is a $C_{6-18}$ aryl group (e.g., a phenyl group) which may be substituted with substituent(s) selected from the group consisting of:

(a) a halogen atom,
(b) $C_{1-4}$ alkyl which may be halogenated, and
(c) cyano,

$R^{1b}$ is :

(i) a $C_{1-8}$ alkyl group which may be substituted with substituent(s) selected from the group consisting of:

(a) $-NR^8-CO-C_{1-4}$ alkyl, and
(b) $-NR^8-CO-(CH_2)_n-O-C_{1-4}$ alkyl,

wherein n is an integer from 1 to 4, and $R^8$ is a hydrogen atom or a $C_{1-4}$ alkyl group,
(ii) a $C_{3-8}$ cycloalkyl group,
(iii) a $C_{6-18}$ aryl group which may be substituted with substituent(s) selected from the group consisting of:

(a) a halogen atom,
(b) $-O-C_{1-4}$ alkyl,
(C)$-(CH_2)_m-NR^8-(CH_2)_n-OH$,
(d)$-(CH_2)_m-NR^8-(CH_2)_n-O-C_{1-4}$alkyl,
(e)$-(CH_2)_m-NR^8-(CH_2)_n-SO_2-C_{1-4}$ alkyl,
(f)$-(CH_2)m-NR^8-(CH_2)_n-NR^8-CO-C_{1-4}$ alkyl,
(g)$-(CH_2)_m-O-(CH_2)_n-O-C_{1-4}$ alkyl,
(h) $-(CH_2)_m-NR^8-CO-(CH_2)_n-OH$,
(i) $-(CH_2)_m-NR^8-CO-(CH_2)_n-O-C_{1-4}$ alkyl,
(j) $-(CH_2)_m-NR^8-CO-(CH_2)_n-SO_2-C_{1-4}$ alkyl,
(k) $-(CH_2)_m-NR^8-CO-NR^8-(CH_2)_n-SO_2-C_{1-4}$ alkyl,
(1) $-(CH_2)_m-NR^8-SO_2-C_{1-4}$ alkyl,
(m)$-(CH_2)_m-NR^8-CO-(CH_2)_n-O-(CH_2)_n-O-C_{1-4}$ alkyl, and
(n) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$,

wherein m is an integer from 0 to 4 (preferably 1 to 3), n is an integer from 1 to 4, and $R^8$ is a hydrogen atom or a $C_{1-4}$ alkyl group,
(iv) a $C_{6-18}$ aryl-$C_{1-4}$ alkyl group which may be substituted with $-O-C_{1-4}$ alkyl,
(v) a carbamoyl group which may be substituted with substituent(s) selected from the group consisting of:

(a) $-(CH_2)_n-OH$,
(b) $-(CH_2)_n-NR^6R^7$,
(c) $-(CH_2)_n-O-C_{1-4}$ alkyl,
(d) $-(CH_2)_n-NR^8-(CH_2)_n-SO_2-C_{1-4}$ alkyl,
(e) $-(CH_2)_n-NR^8-CO-(CH_2)_n-OH$,
(f) $-(CH_2)_n-NR^8-CO-(CH_2)_n-NR^6R^7$, and
(g) $-(CH_2)_n-NR^8-CO-(CH_2)_n$-heterocyclic group (for example, a 5- to 8- membered heterocyclic group having 1 to 3 heteroatoms selected from nitrogen, oxygen and sulfur atoms),

wherein n is an integer from 1 to 4, and when n is two or more, a part of $-CH_2-CH_2-$ moieties thereof may be replaced by $-CH=CH-$; $R^6$ and $R^7$ are identical or different and are each a hydrogen atom or a $C_{1-4}$ alkyl group which may have hydroxy; and $R^8$ is a hydrogen atom or a $C_{1-4}$ alkyl group,
(vi) a 5- to 8- membered cyclic amino-carbonyl group which may be substituted with substituent(s) selected from the group consisting of:

(a) hydroxy,
(b) $-(CH_2)_n-NR^6R^7$, and
(c) $-CO-(CH_2)_n-O-C_{1-4}$ alkyl,

wherein n is an integer from 1 to 4, and when n is two or more, a part of $-CH_2-CH_2-$ moieties thereof may be replaced by $-CH=CH-$; and $R^6$ and $R^7$ are identical or different and are each a hydrogen atom or a $C_{1-4}$ alkyl group,
(vii) a 5- to 8- membered heterocyclic group having 1 to 3 heteroatoms selected from nitrogen, oxygen and sulfur atoms, which may be substituted with substituent(s) selected from the group consisting of:

(a) - $(CH_2)_m$-$NR^8$-CO-$(CH_2)_n$-OH,
(b) - $(CH_2)_m$-$NR^8$-CO-$(CH_2)_n$-$SO_2$-$C_{1-4}$ alkyl, and
(C) -$(CH_2)_m$-$NR^8$-$(CH_2)_n$-OH,

wherein m is an integer from 0 to 4 (preferably, 1 to 3), n is an integer from 1 to 4, and $R^8$ is a hydrogen atom or a $C_{1-4}$ alkyl group, or
(viii) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with -$(CH_2)_n$-OH [wherein n is an integer from 1 to 4],

$R^{2b}$ is a hydrogen atom,
$R^{3b}$ is a hydrogen atom, and
$Z^b$ is a $C_{1-4}$ alkylene group which may be substituted with $C_{1-4}$ alkyl which may be halogenated;
[16] the compound according to [10] above, wherein
$B^3$ is a benzene ring which may be substituted with $C_{1-4}$ alkyl,
$C^3$ is a 5- to 8- membered heterocyclic group having 1 to 3 heteroatoms selected from nitrogen, oxygen and sulfur atoms, which may be substituted with $C_{1-4}$ alkyl,
$R^{1c}$ is:

(i) a $C_{1-8}$ alkyl group which may be substituted with substituent(s) selected from the group consisting of:

(a) hydroxy,
(b) -$NR^8$- $(CH_2)_n$-O-$C_{1-4}$ alkyl, and
(c) -$NR^8$-CO-$(CH_2)_n$-O-$C_{1-4}$ alkyl,

wherein n is an integer from 1 to 4, and $R^8$ is a hydrogen atom or a $C_{1-4}$ alkyl group,
(ii) a $C_{6-18}$ aryl group which may be substituted with substituent(s) selected from the group consisting of:

(a) -O-$C_{1-4}$ alkyl,
(b) -$(CH_2)_m$-$NR^8$-$(CH_2)_n$-OH,
(c) -$(CH_2)_m$-$NR^8$-$(CH_2)_n$-O-$C_{1-4}$ alkyl,
(d) -$(CH_2)_m$-$NR^8$-$(CH_2)_n$-$NR^8$-CO-$C_{1-4}$ alkyl,
(e) -$(CH_2)_m$-$NR^8$-$(CH_2)_n$-$SO_2$-$C_{1-4}$ alkyl,
(f) -$(CH_2)_m$-$NR^8$-CO-$(CH_2)_n$-O-$C_{1-4}$ alkyl,
(g) - $(CH_2)_m$-$NR^8$-CO-$(CH_2)_n$-$NR^6R^7$,
(h) -$NR^6R^7$, and
(i) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with -$(CH_2)_n$-OH,

wherein m is an integer from 0 to 4, n is an integer from 1 to 4, and when m or n (particularly, n) is two or more, a part of - $CH_2$-$CH_2$- moieties thereof may be replaced by - CH=CH-; $R^6$ and $R^7$ are identical or different and are each a hydrogen atom or a $C_{1-4}$ alkyl group; and $R^8$ is a hydrogen atom, a $C_{1-4}$ alkyl group, or a $C_{1-4}$ alkyl-carbonyl group,
(iii) a $C_{6-18}$ aryl-$C_{1-4}$ alkyl group which may be substituted with substituent(s) selected from the group consisting of:

(a) nitro, and
(b) -O-$C_{1-4}$ alkyl,

(iv) a carbamoyl group which may be substituted with -$(CH_2)_n$-O-$C_{1-4}$ alkyl [wherein n is an integer from 1 to 4],
(v) a 5- to 8- membered cyclic amino-carbonyl group which may be substituted with hydroxy, or
(vi) a 5- to 8- membered heterocyclyl-$C_{1-4}$ alkyl group having 1 to 3 heteroatoms selected from nitrogen, oxygen and sulfur atoms (e.g., piperidinomethyl), which may be substituted with hydroxy,

$R^{2c}$ is a hydrogen atom, and
$R^{3c}$ is a hydrogen atom;
[17] the compound according to [11] above, wherein
$B^4$ is a benzene ring which may be substituted with halogen atom(s),

$C^4$ is a 5- to 8- membered heterocyclic group having 1 to 3 heteroatoms selected from nitrogen, oxygen and sulfur atoms,
$R^{1d}$ is a $C_{6-18}$ aryl group which may be substituted with - $(CH_2)_m$-$NR^8$-CO- $(CH_2)_n$-OH [wherein m is an integer from 0 to 4 (preferably, 1 to 3), n is an integer from 1 to 4, and $R^8$ is a hydrogen atom or $C_{1-4}$ alkyl],
$R^{2d}$ is a hydrogen atom,
$R^{3d}$ is a hydrogen atom, and
$Z^d$ is a $C_{1-4}$ alkylene group;
[18] the compound according to [1] above, which is N-{3-chloro-4-[(3-fluorobenzyl)oxy]phenyl}-2-[3-({[2-(methylsulfonyl)ethyl]amino}methyl)phenyl][1,3]thiazolo[5,4-d]pyrimidin-7-amine,
7-({3-chloro-4-[(3-fluorobenzyl)oxy]phenyl}amino)-N-(2-hydroxyethyl)[1,3]thiazolo[5,4-d]pyrimidine-2-carboxamide,
2-({4-[7-({3-chloro-4-[(3-fluorobenzyl)oxy]phenyl}amino)[1,3]thiazolo[5,4-d]pyrimidin-2-yl]benzyl}amino)ethanol,
N-{3-chloro-4-[(3-fluorobenzyl)oxy]phenyl}-2-(4-{[(2-methoxyethyl)amino]methyl}phenyl)[1,3]thiazolo[5,4-d]pyrimidin-7-amine,
N-{3-chloro-4-[(3-fluorobenzyl)oxy]phenyl}-2-(4-{[(3-methoxypropyl)amino]methyl}phenyl)[1,3]thiazolo[5,4-d]pyrimidin-7-amine,
N-[2-({4-[7-({3-chloro-4-[(3-fluorobenzyl)oxy]phenyl}amino)[1,3]thiazolo[5,4-d]pyrimidin-2-yl]benzyl}amino)ethyl]acetamide,
N-{3-chloro-4-[(3-fluorobenzyl)oxy]phenyl}-2-[4-({[2-(methylsulfonyl)ethyl]amino}methyl)phenyl][1,3]thiazolo[5,4-d]pyrimidin-7-amine,
N-{2-[{4-[7-({3-chloro-4-[(3-fluorobenzyl)oxy]phenyl}amino)[1,3]thiazolo[5,4-d]pyrimidin-2-yl]benzyl}(methyl)amino]ethyl}acetamide,
2-methoxy-N-{4-[7-({3-methyl-4-[(6-methylpyridin-3-yl)oxy]phenyl}amino)[1,3]thiazolo[5,4-d]pyrimidin-2-yllbenzyllacetamide,
7-({3-chloro-4-[(3-fluorobenzyl)oxy]phenyl}amino)-N-(2-{[(2E)-4-(dimethylamino)but-2-enoyl]amino}ethyl)[1,3]thiazolo[5,4-d]pyrimidine-2-carboxamide,
N-{2-[bis(2-hydroxyethyl)amino]ethyl}-7-({3-chloro-4-[(3-fluorobenzyl)oxy]phenyl}amino)[1,3]thiazolo[5,4-d]pyrimidine-2-carboxamide,
7-({3-chloro-4-[(3-fluorobenzyl)oxy]phenyl}amino)-N-{2-[(3-hydroxypropanoyl)amino]ethyl}[1,3]thiazolo[5,4-d]pyrimidine-2-carboxamide,
N-{3-chloro-4-[(3-fluorobenzyl)oxy]phenyl}-2-({4-[(2E)-4-methoxybut-2-enoyl]piperazin-1-yl}carbonyl)[1,3]thiazolo[5,4-d]pyrimidin-7-amine,
N-{3-[7-({3-chloro-4-[(3-fluorobenzyl)oxy]phenyl}amino)[1,3]thiazolo[5,4-d]pyrimidin-2-yl]benzyl}-2-hydroxyacetamide, or
N-{4-[7-({3-chloro-4-[(3-fluorobenzyl)oxy]phenyl}amino)[1,3]thiazolo[5,4-d]pyrimidin-2-yl]benzyl}-N'-[2-(methylsulfonyl)ethyl]urea,
or a salt thereof;
[19] a process for producing the compound represented by Formula (I) or a salt thereof, comprising reacting a compound represented by Formula:

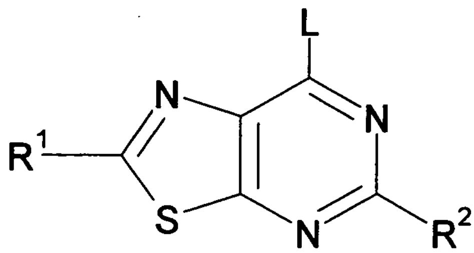

(II)

wherein L is a leaving group, and other symbols have the same meanings as defined in the above [1], or a salt thereof, with a compound represented by Formula: G-X-A
wherein G is a hydrogen atom or a metal atom, and other symbols have the same meanings as defined in the above

[1], provided that when X is $-CHR^3-$ (the symbols in the formula have the same meanings as defined in the above [1]), and G is a metal atom, or a salt thereof;
[20] a medicine containing a compound represented by Formula:

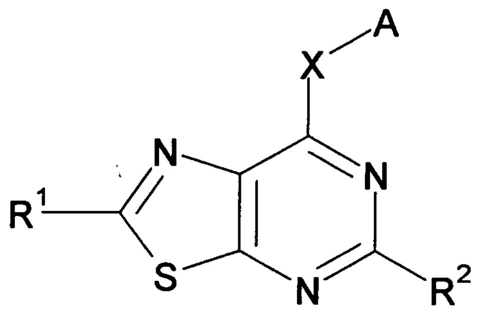
(Ia)

wherein A is aryl or heteroaryl, each of which may be substituted; $R^1$ is a group which is bonded through carbon; $R^2$ is hydrogen or an aliphatic hydrocarbon group; and X is $-NR^3-$, -O-, -S-, -SO-, $-SO_2-$, or $-CHR^3-$ (wherein $R^3$ is hydrogen or an aliphatic hydrocarbon group),
or a salt thereof or a prodrug thereof;
[21] the medicine according to [20] above, which is a tyrosine kinase inhibitor;
[22] the medicine according to [20] above, which is a prophylactic and/or therapeutic agent for cancer;
[23] the medicine according to [22] above, wherein the cancer is breast cancer, prostate cancer, lung cancer, pancreatic cancer, or kidney cancer;
[24] a method of preventing and/or treating cancer by administering an effective amount of a compound represented by Formula:

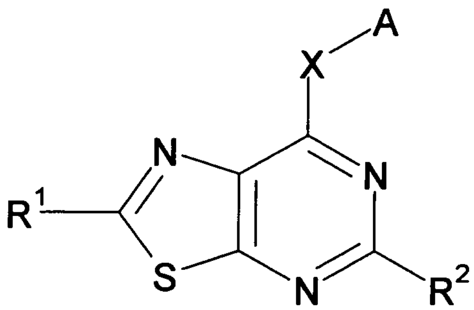
(Ia)

wherein A is aryl or heteroaryl, each of which may be substituted; $R^1$ is a group which is bonded through carbon; $R^2$ is hydrogen or an aliphatic hydrocarbon group; and X is $-NR^3-$, -O-, -S-, -SO-, $-SO_2-$, or $-CHR^3-$ (wherein $R^3$ is hydrogen or an aliphatic hydrocarbon group),
or a salt thereof or a prodrug thereof, to a mammal;
[25] use of a compound represented by Formula:

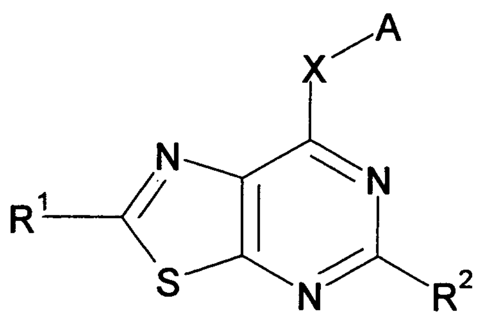
(Ia)

wherein A is aryl or heteroaryl, each of which may be substituted; $R^1$ is a group which is bonded through carbon; $R^2$ is hydrogen or an aliphatic hydrocarbon group; and X is - $NR^3$-, -O-, -S-, -SO-, -$SO_2$-, or -$CHR^3$- (wherein $R^3$ is hydrogen or an aliphatic hydrocarbon group),
or a salt thereof, or a prodrug thereof, for the preparation of a prophylactic and/or therapeutic agent for cancer; and the like.

Furthermore, the invention provides:

[26] the compound according to [1] above, wherein
A is:

(i) a $C_{6-18}$ aryl group, or

(ii) a 5- or 6- membered aromatic monocyclic heterocyclic group or a 8- to 12- membered aromatic fused heterocyclic group, each of which has 1 to 4 heteroatoms selected from oxygen, sulfur and nitrogen atoms, as an atom constituting the ring system (ring atoms) (for example, a heterocyclic ring in which the above-mentioned 5- or 6- membered aromatic monocyclic heterocyclic group is fused with a benzene ring, or a heterocyclic ring in which two heterocyclic rings of the above-mentioned 5- or 6- membered aromatic monocyclic heterocyclic group that may be identical or different, are fused with each other),
each of which may be substituted with 1 to 5 substituents selected from a group represented by Formula: -Y-B, halogen, $C_{1-4}$ alkyl which may be halogenated, hydroxy, $C_{1-4}$ alkyloxy which may be halogenated, $C_{1-4}$ alkyloxy-$C_{1-4}$ alkyl, hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino and $C_{1-4}$ alkylsulfonylamino,
Y is:

(i) a single bond, or
(ii) a spacer selected from -O-, -$OCH_2$-, -$CH_2O$-, - $CH_2$-, -$(CH_2)_2$-, -CO-, -C(OH)$R^4$ -, -C(=N-$OR^4$)-, -S-, - SO-, -$SO_2$-, -$NR^4$-, -N($COR^4$)-, -N($CO_2R^5$)-, -N($SO_2R^5$)-, -CO-$NR^4$-, -$NR^4$-CO-, -$NR^4$-$CO_2$-, -$NR^4$-CO-NH-, -$NR^4$-$SO_2$-and -$SO_2$-$NR^4$- [wherein $R^4$ is a hydrogen atom or a $C_{1-4}$ alkyl group, and $R^5$ is a $C_{1-4}$ alkyl group],

B is:

(i) a $C_{6-18}$ aryl group,
(ii) a 8- to 12- membered heteroaryl group having 1 to 4 heteroatoms selected from oxygen, sulfur and nitrogen atoms, as an atom constituting the ring system (ring atoms), or
(iii) a 3- to 8- membered saturated or unsaturated aliphatic heterocyclic group having 1 to 4 heteroatoms selected from oxygen, sulfur and nitrogen atoms, as an atom constituting the ring system (ring atoms),
each of which may be substituted with 1 to 5 substituents selected from halogen, $C_{1-4}$ alkyl which may be halogenated, hydroxy, $C_{1-4}$ alkyloxy which may be halogenated, $C_{1-4}$ alkyloxy-$C_{1-4}$ alkyl, hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino and $C_{1-4}$ alkylsulfonylamino,

$R^1$ is:

(i) a $C_{1-8}$ alkyl group,
(ii) a $C_{2-8}$ alkenyl group,
(iii) a $C_{2-8}$ alkynyl group,
(iv) a carbamoyl group (two substituents may form, together with the adjacent nitrogen atom, a 3- to 8- membered saturated or unsaturated aliphatic heterocyclic ring),
(v) a $C_{3-8}$ cycloalkyl group,
(vi) a $C_{4-12}$ bridged cyclic hydrocarbon group,
(vii) a $C_{6-18}$ aryl group,
(viii) a $C_{6-18}$ aryl-$C_{1-4}$ alkyl group,
(ix) a heterocyclic group, or
(x) heterocyclyl-$C_{1-4}$ alkyl group,
each of which may be substituted with
substituent(s) selected from the group (hereinafter, simply referred to as Substituent Group T) consisting of:

(a) halogen,
(b) oxo,
(c) $C_{1-4}$ alkyl which may be halogenated,
(d) $-(CH_2)_m-Q$,
(e) $-(CH_2)_m-Z^1-C_{1-4}$ alkyl,
(f) $-(CH_2)_m-Z^1$-heterocyclic group,
(g) $-(CH_2)_m-Z^2-(CH_2)_n-Q$,
(h) $-(CH_2)_m-Z^2-(CH_2)_n-Z^1-C_{1-4}$ alkyl,
(i) $-(CH_2)_m-Z^2-(CH_2)_n-Z^1$-heterocyclic group,
(j) $-(CH_2)_m-Z^2-(CH_2)_n-Z^1-(CH_2)_n-Z^3-C_{1-4}$ alkyl, and (k) $-(CH_2)_m-Z^2-(CH_2)_n-Z^1-(CH_2)_n-Z^3$-heterocyclic group,
wherein m is an integer from 0 to 4, n is an integer from 1 to 4;
Q is hydroxy, carboxy, cyano, nitro, $-NR^6R^7$, - $CONCONR^6R^7$ or $-SO_2NR^6R^7$;
$Z^1$ and $Z^3$ are identical or different and are each -O-, -CO-, $-C(OH)R^8-$, $-C(=N-OR^8)-$, -S-, - SO-, $-SO_2-$, $-N(COR^8)-$, $-N(CO_2R^9)-$, $-N(SO_2R^9)-$, - CO-O-, -O-CO-, $-NR^8-CO-$, $NR^8-CO_2-$, $-NR^8-CO-NH-$, or $-NR^8-SO_2-$;
$Z^2$ is -O-, -CO-, $-C(OH)R^8-$, $-C(=N-OR^8)-$, -S-, - SO-, $-SO_2-$, $-NR^8-$, $-N(COR^8)-$, $-N(CO_2R^9)-$, - $N(SO_2R^9)-$, -CO-O-, -O-CO-, $-CO-NR^8-$, $-NR^8-CO-$, - $NR^8-CO_2-$, $-NR^8-CO-NH-$, $-NR^8-SO_2-$, or $-SO_2-NR^8-$;
$(CH_2)_m$ and $(CH_2)_n$ may be substituted with substituent(s) selected from halogen and $C_{1-4}$ alkyl, and when m or n is two or more, a part of $-CH_2-CH_2-$ moieties thereof may be replaced by -CH=CH-;
$R^6$ and $R^7$ are identical or different and are each a hydrogen atom or a $C_{1-4}$ alkyl group which may have hydroxy, or $R^6$ and $R^7$ form a 3- to 8-membered saturated or unsaturated aliphatic heterocyclic ring together with a nitrogen atom;
$R^8$ is a hydrogen atom, a $C_{1-4}$ alkyl group, or a
$C_{1-4}$ alkyl-carbonyl group; and
$R^9$ is a $C_{1-4}$ alkyl group,

$R^2$ is a hydrogen atom, or a straight or branched chain aliphatic hydrocarbon group having 1 to 15 (preferably, 1 to 8) carbon atoms (for example, a $C_{1-8}$ alkyl group, a $C_{2-8}$ alkenyl group, a $C_{2-8}$ alkynyl group, a carbamoyl group or a $C_{3-8}$ cycloalkyl group), and
X is $-NR^3-$, -O-, -S-, -SO-, $-SO_2-$, or $-CHR^3-$ (wherein $R^3$ is a hydrogen atom or a straight or branched chain aliphatic hydrocarbon group having 1 to 15 (preferably, 1 to 8) carbon atoms (for example, a $C_{1-8}$ alkyl group, a $C_{2-8}$ alkenyl group, a $C_{2-8}$alkynyl group, a carbamoyl group or a $C_{3-8}$cycloalkyl group);
[27] the compound according to [8] above, wherein $R^{1a}$ is a $C_{6-18}$ aryl group which may be substituted, or a $C_{6-18}$ aryl-$C_{1-4}$ alkyl group which may be substituted;
[28] the compound according to [9] above, wherein $R^{1b}$ is a $C_{6-18}$ aryl group which may be substituted, or a $C_{6-18}$ aryl-$C_{1-4}$ alkyl group which may be substituted;
[29] the compound according to [10] above, wherein $R^{1c}$ is a $C_{6-18}$ aryl group which may be substituted, or a $C_{6-18}$ aryl-$C_{1-4}$ alkyl group which may be substituted; and
[30] the compound according to [11] above, wherein $R^{1d}$ is a $C_{6-18}$ aryl group which may be substituted, or a $C_{6-18}$ aryl-$C_{1-4}$ alkyl group which may be substituted.

The present invention provides a thiazolo[5,4-d]pyrimidine derivative which has excellent tyrosine kinase inhibitory action, and low toxicity, and thus can be sufficiently used as a medicine, a process for producing the same, and use thereof.

## Best Mode for Carrying Out the Invention

In the present specification, the "aryl group" includes a monocyclic aryl group and a fused polycyclic aryl group, unless otherwise specified. The "aryl group" is exemplified by a $C_{6-18}$ aryl group. The "$C_{6-18}$ aryl group" is exemplified by phenyl, biphenylyl, naphthyl, anthryl, phenanthryl, and acenaphthylenyl.
In the present specification, the "heterocyclic group (and "heterocyclyl-" in the substituents)" is exemplified by a 8- to 12- membered heteroaryl group or a 8- to 12-membered saturated or unsaturated aliphatic heterocyclic group, each of which has one or more heteroatoms (preferably 1 to 4, more preferably 1 to 2) selected from oxygen, sulfur, and nitrogen atoms and the like as the atom constituting the ring system (ring atoms). In the present specification, the "aliphatic hydrocarbon group" is exemplified by a straight or branched chain aliphatic hydrocarbon group having 1 to 15 (preferably, 1 to 8) carbon atoms, unless otherwise specified. Examples of such the "aliphatic hydrocarbon group" include a $C_{1-8}$ alkyl group, a $C_{2-8}$ alkenyl group, a $C_{2-8}$ alkynyl group, a carbamoyl group, and a $C_{3-8}$ cycloalkyl group.
In the present specification, the "heteroaryl group" is exemplified by an aromatic monocyclic heterocyclic group (for example, a 5- or 6- membered aromatic monocyclic heterocyclic group such as, furyl, thienyl, pyrrolyl, oxazolyl,

isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like), an aromatic fused heterocyclic group (for example, a 8- to 12- membered aromatic fused heterocyclic group such as, benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like), and the like, unless otherwise specified. The aromatic fused heterocyclic group is preferably a heterocyclic ring in which the above-mentioned 5- or 6- membered aromatic monocyclic heterocyclic group is fused with a benzene ring, or a heterocyclic ring in which two heterocyclic rings of the above-mentioned 5- or 6- membered aromatic monocyclic heterocyclic group that may be identical or different are fused with each other.

In the present specification, the "aliphatic heterocyclic group" is exemplified by a 3- to 8- membered (preferably, 5- to 8- membered) saturated or unsaturated (preferably, saturated) aliphatic heterocyclic group, such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like, unless otherwise specified.

In the present specification, the "$C_{1-8}$ alkyl group" is exemplified by methyl, ethyl, n-propyl, i-propyl, n-butyl, i-butyl, s-butyl, t-butyl, n-pentyl, i-pentyl, t-pentyl, neopentyl, n-hexyl, i-hexyl, n-heptyl, n-octyl and the like, unless otherwise specified. Among these, a $C_{1-6}$ alkyl group is preferred. In the present specification, the "$C_{1-4}$ alkyl group" is exemplified by methyl, ethyl, n-propyl, i-propyl, n-butyl, and i-butyl, unless otherwise specified.

In the present specification, the "$C_{2-8}$ alkenyl group" is exemplified by vinyl, (1- or 2-) propenyl, (1-, 2- or 3-) butenyl, pentenyl, octenyl and (1,3-) butadienyl, unless otherwise specified. Among these, a $C_{2-4}$ alkenyl group is preferred.

In the present specification, the "$C_{2-8}$ alkynyl group" is exemplified by ethynyl, (1- or 2-) propynyl, (1-, 2- or 3-) butynyl, pentynyl and octynyl, unless otherwise specified. Among these, a $C_{2-4}$alkynyl group is preferred.

In the present specification, the "$C_{3-8}$ cycloalkyl group" is exemplified by cyclopropyl, cyclobutyl, cyclopentyl, cyclohexyl, cycloheptyl and cyclooctyl, unless otherwise specified. Among these, a $C_{3-6}$ cycloalkyl group is preferred.

In the present specification, the "$C_{4-12}$ bridged cyclic hydrocarbon group is exemplified by 1-adamantyl, 2-adamantyl, 2-norbornanyl and 5-norbornen-2-yl, unless otherwise specified.

In the present specification, the "halogen" is exemplified by fluorine, chlorine, bromine and iodine, unless otherwise specified.

In the above-mentioned formulas, the "aryl group" represented by A is preferably a $C_{6-18}$ aryl group, more preferably phenyl.

The "aryl group" or the "heteroaryl group" represented by A may be substituted with a group represented by Formula: -Y-B (wherein Y is a single bond or a spacer, and B is an aryl group or a heterocyclic group, each of which may be substituted).

The spacer represented by Y is exemplified by -O-, -O-Z- (preferably, $-OCH_2-$), -Z-O- (preferably, $-CH_2O-$), -Z-(preferably, $-CH_2-$), $-(CH_2)_2-$, -CO-, $-C(OH)R^4-$, $-C(=N-OR^4)-$, - S-, -SO-, $-SO_2-$, $-NR^4-$, $-N(COR^4)-$, $-N(CO_2R^5)-$, $-N(SO_2R^5)-$, - $CO-NR^4-$, $-NR^4-CO-$, $-NR^4-CO_2-$, $-NR^4-CO-NH-$, $-NR^4-SO_2-$ and $-SO_2-NR^4-$ [wherein $R^4$ is a hydrogen atom, or a $C_{1-4}$ alkyl group, $R^5$ is a $C_{1-4}$ alkyl group, and Z is a $C_{1-4}$ alkylene group which may be substituted].

As the "$C_{1-4}$ alkylene group" of the "$C_{1-4}$ alkylene group which may be substituted" represented by Z, methylene, ethylene, propylene, butylene or the like, can be used. Among these, methylene is preferred.

As the substituent of the "$C_{1-4}$ alkylene group", for example, halogen, $C_{1-4}$ alkyl which may be halogenated, hydroxy, $C_{1-4}$ alkyloxy which may be halogenated, $C_{1-4}$ alkyloxy-$C_{1-4}$ alkyl (e.g., $C_{1-4}$ alkyloxymethyl), hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino, $C_{1-4}$ alkylsulfonylamino or the like, can be used. Among these, $C_{1-4}$ alkyl which may be halogenated, or the like is preferred.

The spacer represented by Y is preferably, -O-, $-OCH_2-$, or the like.

The "aryl group" represented by B is preferably a $C_{6-18}$ aryl group, and among these, phenyl is preferred.

The "aryl group" or the "heterocyclic group" represented by B may have 1 to 5 substituents, which may be identical or different, selected from halogen, $C_{1-4}$ alkyl which may be halogenated (e.g., methyl, trifluoromethyl, ethyl, propyl, isopropyl, n-butyl, tert-butyl), hydroxy, $C_{1-4}$ alkyloxy which may be halogenated (e.g., methoxy, trifluoromethoxy, ethoxy, propoxy, butoxy), $C_{1-4}$ alkyloxy-$C_{1-4}$ alkyl (e.g., $C_{1-4}$ alkyloxymethyl such as, methoxymethyl, ethoxymethyl, propoxymethyl and the like), hydroxy $C_{1-4}$ alkyl group (e.g., hydroxymethyl, hydroxyethyl), $C_{1-4}$ alkyl-carbonyl (e.g., methylcarbonyl, ethylcarbonyl), carboxy, $C_{1-4}$ alkoxy-carbonyl (e.g., methoxycarbonyl, ethoxycarbonyl), cyano, carbamoyl, sulfamoyl, nitro, amino, $C_{1-4}$alkyl-carbonylamino (e.g., methylcarbonylamino, ethylcarbonylamino), $C_{1-4}$ alkoxy-carbonylamino (e.g., methoxycarbonylamino, ethoxycarbonylamino) and $C_{1-4}$ alkylsulfonylamino (e.g., methylsulfo-

nylamino, ethylsulfonylamino), at substitutable arbitrary positions.

Instead of the group represented by the above-mentioned formula: -Y-B, or in addition to the group represented by the above-mentioned formula: -Y-B, the "aryl group" or the "heteroaryl group" represented by A may have 1 to 5 substituents which may be identical or different, at substitutable arbitrary positions. Examples of such substituents include the same one exemplified for the "aryl group" or the "heterocyclic group" represented by B.

The A is preferably, for example, (i) an aryl group which is substituted with a group having an aromatic ring, or (ii) a heteroaryl group which may be substituted.

The "group having an aromatic ring" may be any groups containing an aromatic ring in the molecule, and the aromatic ring may be substituted with substituent(s) which the "aryl group" represented by B may has, or the like. Specifically, as the "group having an aromatic ring", a group represented by formula: $-Y^1-B^1$ (wherein $Y^1$ is a single bond or a spacer, and $B^1$ is an aryl group or a heteroaryl group, each of which may be substituted), or the like, can be used.

As the "spacer" represented by $Y^1$, the same one as the "spacer" represented by Y can be used.

As the "aryl group which may be substituted" represented by $B^1$, the same one as the "aryl group which may be substituted" represented by B can be used.

As the "heteroaryl group" of the "heteroaryl group which may be substituted" represented by $B^1$, the same one as the "heteroaryl group" represented by A can be used.

As the substituent of the "heteroaryl group" represented by $B^1$, the same one as the substituent of the "heterocyclic group" represented by B can be used.

The "group which is bonded through carbon" represented by $R^1$ is exemplified by a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, and a heterocyclyl-$C_{1-4}$ alkyl group, each of which may be substituted. Among these, a $C_{6-18}$ aryl group (preferably, a phenyl group) which may be substituted, or a $C_{6-18}$ aryl-$C_{1-4}$ alkyl group (preferably, a benzyl group) which may be substituted, is preferred.

The "$C_{1-8}$ alkyl group", "$C_{2-8}$ alkenyl group", "$C_{2-8}$ alkynyl group", "$C_{3-8}$ cycloalkyl group", "$C_{4-12}$ bridged cyclic hydrocarbon group", "$C_{6-18}$ aryl group", "$C_{6-18}$ aryl-$C_{1-4}$ alkyl group", "heterocyclic group", and "heterocyclyl-$C_{1-4}$ alkyl group" may be substituted with one or more substituents selected from the group (hereinafter, may be referred to as Substituent Group T) consisting of, for example, halogen, oxo, $C_{1-4}$ alkyl which may be halogenated, $-(CH_2)_m-Q$, $-(CH_2)_m-Z^1-C_{1-4}$ alkyl, $-(CH_2)_m-Z^1$-heterocyclic group, $-(CH_2)_m-Z^2-(CH_2)_n-Q$, $-(CH_2)_m-Z^2-(CH_2)_n-Z^1-C_{1-4}$ alkyl, $-(CH_2)_m-Z^2-(CH_2)_n-Z^1$-heterocyclic group, $-(CH_2)_m-Z^2-(CH_2)_n-Z^1-(CH_2)_n-Z^3-C_{1-4}$ alkyl, and $-(CH_2)_m-Z^2-(CH_2)_n-Z^1-(CH_2)_n-Z^3$-heterocyclic group.

In the formulas, m is an integer from 0 to 4, n is an integer from 1 to 4, Q is hydroxy, carboxy, cyano, nitro, $-NR^6R^7$, $-CONR^6R^7$ or $-SO_2NR^6R^7$, $Z^1$ and $Z^3$ are identical or different and are each -O-, -CO-, $-C(OH)R^8-$, $-C(=N-OR^8)-$, -S-, -SO-, $-SO_2-$, $-N(COR^8)$ -, $-N(CO_2R^9)-$, $N(SO_2R^9)-$, -CO-O-, -O-CO-, $-NR^8-CO-$, $-NR^8-CO_2-$, $NR^8-CO-NH-$, or $-NR^8-SO_2-$, and $Z^2$ is -O-, -CO-, $-C(OH)R^8-$, $-C(=N-OR^8)-$, -S-, -SO-, $-SO_2-$, $-NR^8-$, $- N(COR^8)-$, $-N(CO_2R^9)$ -, $N(SO_2R^9)-$, -CO-O-, -O-CO-, $-CO-NR^8-$, $- NR^8-CO-$, $-NR^8-CO_2-$, $-NR^8-CO-NH-$, $-NR^8-SO_2-$, or $-SO_2-NR^8$. The $(CH_2)_m$ and $(CH_2)_n$ in the formulas may be substituted with one or more substituents selected from, for example, halogen and $C_{1-4}$ alkyl, and when m or n is two or more, a part of $-CH_2-CH_2-$ moieties thereof may be replaced by -CH=CH-.

In the formulas, $R^6$ and $R^7$ are identical or different and are each a hydrogen atom or a $C_{1-4}$ alkyl group optionally having hydroxy, or $R^6$ and $R^7$ form a ring together with a nitrogen atom. Further, $R^8$ is a hydrogen atom, $C_{1-4}$ alkyl, or a $C_{1-4}$ alkyl-carbonyl group, and $R^9$ is $C_{1-4}$ alkyl, in the formulas.

As the $C_{1-4}$ alkyl-carbonyl group, methylcarbonyl, ethylcarbonyl or the like, can be used.

When $R^6$ and $R^7$ form a ring together with a nitrogen atom, the nitrogen-containing heterocyclic group is exemplified by a 3- to 8- membered (preferably, 5- or 6- membered) saturated or unsaturated (preferably, saturated) aliphatic heterocyclic group such as, azetidinyl, pyrrolidinyl, piperidinyl, homopiperidinyl, heptamethylenimino, morpholinyl, thiomorpholinyl, piperazinyl, homopiperazinyl and the like.

As the "heterocyclic group", the same one as the above-mentioned "heterocyclic group" can be used. Among these, a 5- or 6- membered aromatic monocyclic heterocyclic group having one or more (preferably 1 to 4, more preferably 1 to 2) heteroatoms selected from oxygen, sulfur and nitrogen atoms and the like, such as thienyl, is preferred.

When m or n is two or more and a part of $-CH_2-CH_2-$moieties thereof is replaced by -CH=CH-, $(CH_2)_m$ or $(CH_2)_n$ is -CH=CH-, $-CH_2-CH=CH-$, $-CH=CH-CH_2-$, $-CH_2-CH_2-CH=CH-$, $-CH_2-CH=CH-CH_2-$, $-CH=CH-CH_2-CH_2-$ or the like. Among these, $- CH=CH-CH_2-$ is preferred.

The above-mentioned "carbamoyl" may have 1 or 2 substituents selected from a hydrogen atom and a $C_{1-8}$ alkyl group which may be substituted. Alternatively the "carbamoyl" may have two substituents, and form a ring which may be substituted, together with the adjacent nitrogen atom. The "ring" of the "ring which may be substituted" is exemplified by the same ring as the above-mentioned case where $R^6$ and $R^7$ form a ring together with a nitrogen atom. The "substituent" of the "$C_{1-8}$ alkyl group which may be substituted" and the "substituent" of the "ring which may be substituted" are exemplified by the same group as the substituent of the above-mentioned Substituent Group T.

For the compound represented by Formula (I), compounds (I-I) to (I-V) of the below, and the like are preferred.

[Compound (I-I)]

A compound represented by Formula:

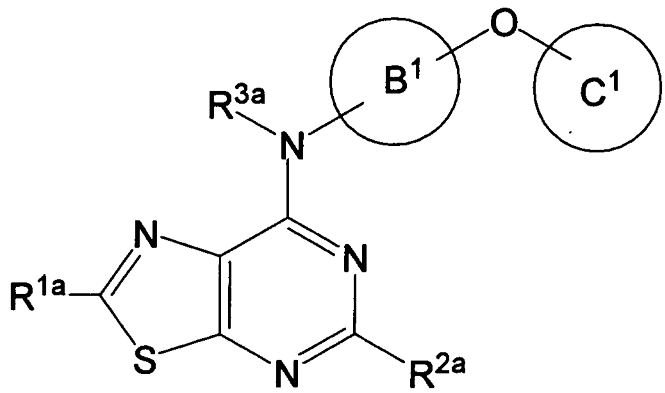

(I-I)

wherein $B^1$ is a $C_{6-18}$ aryl ring which may be substituted; $C^1$ is a $C_{6-18}$ aryl group which may be substituted; $R^{1a}$ is a group which is bonded through carbon; $R^{2a}$ is a hydrogen atom or an aliphatic hydrocarbon group; and $R^{3a}$ is a hydrogen atom or an aliphatic hydrocarbon group.

As the "$C_{6-18}$ aryl ring" of the "$C_{6-18}$ aryl ring which may be substituted" represented by $B^1$, for example, a benzene ring, a naphthalene ring, or the like, can be used. Among these, a benzene ring is preferred.

As the substituent of the "$C_{6-18}$ aryl ring", 1 to 5 substituents, which may be identical or different, selected from halogen, $C_{1-4}$ alkyl which may be halogenated, hydroxy, $C_{1-4}$ alkyloxy which may be halogenated, $C_{1-4}$ alkyloxy-$C_{1-4}$ alkyl (e.g., $C_{1-4}$ alkyloxymethyl), hydroxy $C_{1-4}$ alkyl, $C_{1-4}$ alkyl-carbonyl, carboxyl, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino, $C_{1-4}$ alkylsulfonylamino and the like, can be used. The substituents may substitute at substitutable arbitrary positions.

As the "$C_{6-18}$ aryl group" of the "$C_{6-18}$ aryl group which may be substituted" represented by $C^1$, for example, phenyl, biphenyl, naphthyl, anthryl, phenanthryl, acenaphthylenyl or the like, can be used. Among these, phenyl is preferred.

As the substituent of the "$C_{6-18}$ aryl group", 1 to 5 substituents, which may be identical or different, selected from halogen, $C_{1-4}$ alkyl which may be halogenated, hydroxy, $C_{1-4}$ alkyloxy which may be halogenated, $C_{1-4}$ alkyloxy-$C_{1-4}$ alkyl (e.g., $C_{1-4}$ alkyloxymethyl), hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino, $C_{1-4}$ alkylsulfonylamino and the like, can be used. The substituents may substitute at substitutable arbitrary positions.

As the "group which is bonded through carbon" represented by $R^{1a}$, the same one as the "group which is bonded through carbon" represented by $R^1$ can be used.

The $R^{1a}$ is preferably a $C_{6-18}$ aryl group which may be substituted, or a $C_{6-18}$ aryl-$C_{1-4}$ alkyl group which may be substituted.

As the "aliphatic hydrocarbon group" represented by $R^{2a}$, the same one as the "aliphatic hydrocarbon group" represented by $R^2$ can be used.

As the "aliphatic hydrocarbon group" represented by $R^{3a}$, the same one as the "aliphatic hydrocarbon group" represented by $R^3$ can be used.

The compound (I-I) is preferably a compound (I-Ia) described below, in which $B^1$ is a benzene ring which may be substituted, and $C^1$ is a phenyl group which may be substituted. As the substituent of the benzene ring represented by $B^{1a}$, the same one as the substituent of the $C_{6-18}$ aryl ring represented by $B^1$ can be used. As the substituent of a phenyl group represented by $C^{1a}$, the same one as the substituent of the $C_{6-18}$ aryl group represented by $C^1$ can be used.

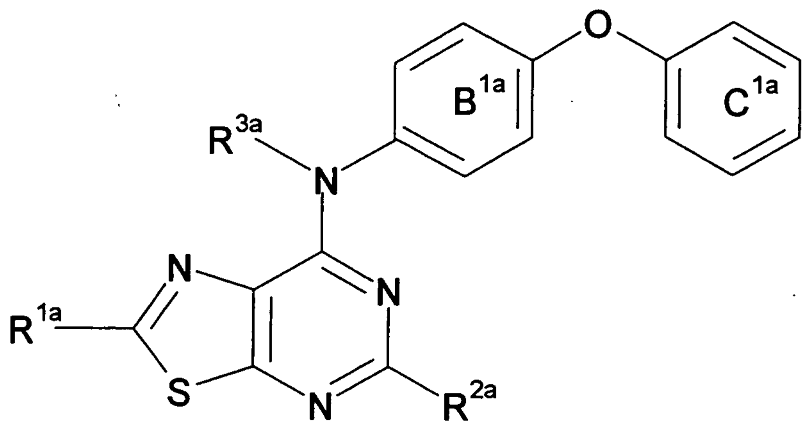

(I-Ia)

In particular, the compound (I-I) is preferably a compound in which
$B^1$ is a benzene ring which may be substituted with halogen atom(s) (e.g., a chlorine atom),
$C^1$ is a $C_{6-18}$ aryl group (e.g., a phenyl group) which may be substituted with substituent(s) selected from the group consisting of:

(a) a halogen atom (e.g., a fluorine atom, a chlorine atom),
(b) $C_{1-4}$ alkyl which may be halogenated (e.g., methyl, trifluoromethyl), and
(c) $C_{1-4}$ alkyloxy which may be halogenated (e.g., methoxy, trifluoromethoxy),

$R^{1a}$ is :

(i) a $C_{6-18}$ aryl group (e.g., a phenyl group) which may be substituted with $-(CH_2)_m-NR^8-(CH_2)_n-OH$ [wherein m is an integer from 0 to 4 (preferably, 1 to 3, specifically preferably, 1), n is an integer from 1 to 4 (preferably, 2), and $R^8$ is a hydrogen atom or $C_{1-4}$ alkyl (preferably, a hydrogen atom)], or
(ii) a carbamoyl group which may be substituted with $-(CH_2)_m-O-(CH_2)_n-OH$ [wherein m is an integer from 0 to 4 (preferably, 1 to 3, specifically preferably, 2), and n is an integer from 1 to 4 (preferably, 2)],

$R^{2a}$ is a hydrogen atom, and
$R^{3a}$ is a hydrogen atom.

[Compound (I-II)]

A compound represented by Formula:

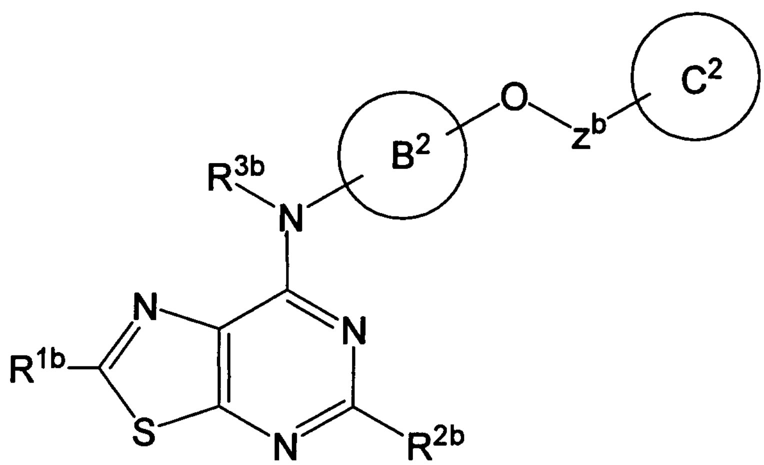

(I-II)

wherein $B^2$ is a $C_{6-18}$ aryl ring which may be substituted; $C^2$ is a $C_{6-18}$ aryl group which may be substituted; $R^{1b}$ is a group which is bonded through carbon; $R^{2b}$ is a hydrogen atom or an aliphatic hydrocarbon group; $R^{3b}$ is a hydrogen atom or an aliphatic hydrocarbon group; and $Z^b$ is a $C_{1-4}$ alkylene group which may be substituted.

As the "$C_{6-18}$ aryl ring which may be substituted" represented by $B^2$, the same one as the "$C_{6-18}$ aryl ring which may be substituted" represented by $B^1$ can be used.

As the "$C_{6-18}$ aryl group which may be substituted" represented by $C^2$, the same one as the "$C_{6-18}$ aryl group which may be substituted" represented by $C^1$ can be used.

As the "$C_{1-4}$ alkylene group" of the "$C_{1-4}$ alkylene group which may be substituted" represented by $Z^b$, methylene, ethylene, propylene, butylene or the like, can be used. Among these, methylene is preferred.

As the substituent of the "$C_{1-4}$ alkylene group", for example, halogen, $C_{1-4}$ alkyl which may be halogenated, hydroxy, $C_{1-4}$ alkyloxy which may be halogenated, $C_{1-4}$ alkyloxy-$C_{1-4}$ alkyl (e.g., $C_{1-4}$ alkyloxymethyl), hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino, $C_{1-4}$ alkylsulfonylamino or the like, can be used. Among these, a $C_{1-4}$ alkyl which may be halogenated, or the like is preferred.

As the "group which is bonded through carbon" represented by $R^{1b}$, the same one as the "group which is bonded through carbon" represented by $R^1$ can be used.

The $R^{1b}$ is preferably a $C_{6-18}$ aryl group which may be substituted, or a $C_{6-18}$ aryl-$C_{1-4}$ alkyl group which may be substituted.

As the "aliphatic hydrocarbon group" represented by $R^{2b}$, the same one as the "aliphatic hydrocarbon group" represented by $R^2$ can be used.

As the "aliphatic hydrocarbon group" represented by $R^{3b}$, the same one as the "aliphatic hydrocarbon group" represented by $R^3$ can be used.

The compound (I-II) is preferably a compound (I-IIa) described below, in which $B^2$ is a benzene ring which may be substituted, and $C^2$ is a phenyl group which may be substituted. As the substituent of the benzene ring represented by $B^{2a}$, the same one as the substituent of the $C_{6-18}$ aryl ring represented by $B^2$ can be used. As the substituent of the phenyl group represented by $C^{2a}$, the same one as the substituent of the $C_{6-18}$ aryl group represented by $C^2$ can be used.

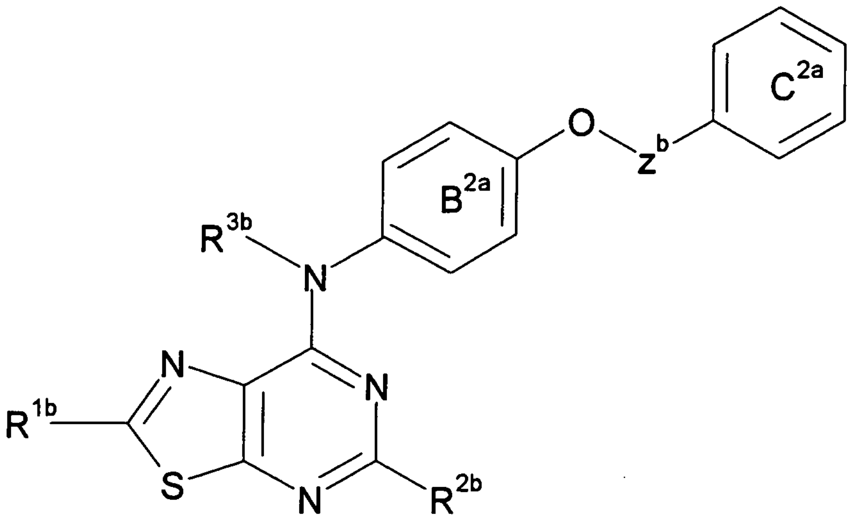

(I-IIa)

In particular, the compound (I-II) is preferably a compound in which
$B^2$ is a benzene ring which may be substituted with substituent(s) selected from the group consisting of:

(a) a halogen atom (e.g., a chlorine atom),
(b) $C_{1-4}$ alkyl (e.g., methyl) which may be substituted with hydroxy or $C_{1-4}$ alkyloxy (e.g., methoxy), and
(c) $C_{1-4}$ alkyloxy (e.g., methoxy) ,
$C^2$ is a $C_{6-18}$ aryl group (e.g., a phenyl group) which may be substituted with substituent(s) selected from the group consisting of:

(a) a halogen atom (e.g., a fluorine atom, a chlorine atom),
(b) $C_{1-4}$ alkyl which may be halogenated (e.g., methyl, trifluoromethyl), and
(c) cyano,

$R^{1b}$ is:

(i) a $C_{1-8}$ alkyl group (preferably, a $C_{1-5}$ alkyl group) which may be substituted with substituent(s) selected from the group consisting of:

(a) $-NR^8-CO-C_{1-4}$ alkyl, and
(b) $-NR^8-CO-(CH_2)_n-O-C_{1-4}$ alkyl,

wherein n is an integer from 1 to 4, $R^8$ is a hydrogen atom or a $C_{1-4}$ alkyl group (preferably, a hydrogen atom), and the $C_{1-4}$ alkyl is preferably methyl,
(ii) a $C_{3-8}$ cycloalkyl group (e.g., a cyclopropyl group),
(iii) a $C_{6-18}$ aryl group (e.g., a phenyl group) which may be substituted with substituent(s) selected from the group consisting of:

(a) a halogen atom (e.g., a fluorine atom),
(b) $-O-C_{1-4}$ alkyl (e.g., methoxy),
(c) $-(CH_2)_m-NR^8-(CH_2)_n-OH$,
(d) $-(CH_2)_m-NR^8-(CH_2)_n-O-C_{1-4}$ alkyl,
(e) $-(CH_2)_m-NR^8-(CH_2)_n-SO_2-C_{1-4}$ alkyl,
(f) $-(CH_2)_m-NR^8-(CH_2)_n-NR^8-CO-C_{1-4}$ alkyl,
(g) $-(CH_2)_m-O-(CH_2)_n-O-C_{1-4}$ alkyl,
(h) $-(CH_2)_m-NR^8-CO-(CH_2)_n-OH$,
(i) $-(CH_2)_m-NR^8-CO-(CH_2)_n-O-C_{1-4}$ alkyl,
(j) $-(CH_2)_m-NR^8-CO-(CH_2)_n-SO_2-C_{1-4}$ alkyl,
(k) $-(CH_2)_m-NR^8-CO-NR^8-(CH_2)_n-SO_2-C_{1-4}$ alkyl,
(l) $-(CH_2)_m-NR^8-SO_2-C_{1-4}$ alkyl,
(m) $-(CH_2)_m-NR^8-CO-(CH_2)_n-O-(CH_2)_n-O-C_{1-4}$ alkyl, and
(n) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$ wherein m is an integer from 0 to 4 (preferably 1 to 3, specifically preferably 1 to 2), n is an integer from 1 to 4 (preferably 1 to 3), $R^8$ is a hydrogen atom or a $C_{1-4}$ alkyl group (preferably, a hydrogen atom or a methyl group), and the $C_{1-4}$ alkyl is preferably methyl, or isopropyl,

(iv) a $C_{6-18}$ aryl-$C_{1-4}$ alkyl group (e.g., a benzyl group) which may be substituted with $-O-C_{1-4}$ alkyl (e.g., methoxy),
(v) a carbamoyl group which may be substituted with substituent(s) selected from the group consisting of:

(a) $-(CH_2)_n-OH$,
(b) $-(CH_2)_n-NR^6R^7$,
(c) $-(CH_2)_n-O-C_{1-4}$ alkyl,
(d) $-(CH_2)_n-NR^8-(CH_2)_n-SO_2-C_{1-4}$ alkyl,
(e) $-(CH_2)_n-NR^8-CO-(CH_2)_n-OH$,
(f) $-(CH_2)_n-NR^8-CO-(CH_2)_n-NR^6R^7$, and
(g) $-(CH_2)_n-NR^8-CO-(CH_2)_n$-heterocyclic group (for example, a 5- to 8- membered heterocyclic group having 1 to 3 heteroatoms selected from nitrogen, oxygen and sulfur atoms, such as thienyl and the like),

wherein n is an integer from 1 to 4 (preferably, 1 to 3), when n is two or more, a part of $-CH_2-CH_2-$ moieties thereof may be replaced by -CH=CH-; $R^6$ and $R^7$ are identical or different and are each a hydrogen atom or a $C_{1-4}$ alkyl group which may have hydroxy (e.g., methyl, hydroxyethyl); $R^8$ is a hydrogen atom or a $C_{1-4}$ alkyl group; and the $C_{1-4}$ alkyl is preferably methyl,
(vi) a 5- to 8- membered cyclic amino-carbonyl group (e.g., a piperidinocarbonyl group, piperazinylcarbonyl) which may be substituted with substituent(s) selected from the group consisting of:

(a) hydroxy,
(b) $-(CH_2)_n-NR^6R^7$, and
(c) $-CO-(CH_2)_n-O-C_{1-4}$ alkyl,

wherein n is an integer from 1 to 4 (preferably, 1 to 3), when n is two or more, a part of $-CH_2-CH_2-$ moieties thereof

may be replaced by -CH=CH-; $R^6$ and $R^7$ are identical or different and are each a hydrogen atom or a $C_{1-4}$ alkyl group (e.g., methyl), and the $C_{1-4}$ alkyl is preferably methyl,

(vii) a 5- to 8- membered heterocyclic group having 1 to 3 heteroatoms selected from nitrogen, oxygen and sulfur atoms (e.g., a thiazole group, an oxadizole group, a furyl group), which may be substituted with a substituent selected from the group consisting of:

(a) $-(CH_2)_m-NR^8-CO-(CH_2)_n-OH$,
(b) $-(CH_2)_m-NR^8-CO-(CH_2)_n-SO_2-C_{1-4}$ alkyl, and
(c) $-(CH_2)_m-NR^8-(CH_2)_n-OH$,

wherein m is an integer from 0 to 4 (preferably, 1 to 3, specifically preferably, 1), n is an integer from 1 to 4 (preferably, 1), $R^8$ is a hydrogen atom or a $C_{1-4}$ alkyl group (preferably, a hydrogen atom), and the $C_{1-4}$ alkyl is preferably, for example, methyl or

(viii) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$ [wherein n is an integer from 1 to 4 (preferably, 1)],

$R^{2b}$ is a hydrogen atom,
$R^{3b}$ is a hydrogen atom, and
$Z^b$ is a $C_{1-4}$ alkylene group (preferably, methylene) which may be substituted with $C_{1-4}$ alkyl which may be halogenated (e.g., methyl, trifluoromethyl).

[Compound (I-III)]

A compound represented by Formula:

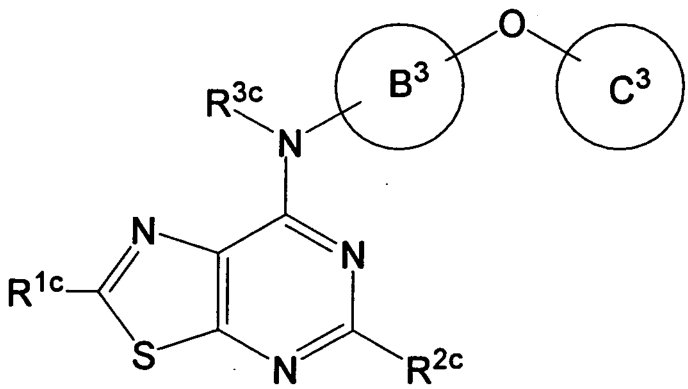

(I-III)

wherein $B^3$ is a $C_{6-18}$ aryl ring which may be substituted; $C^3$ is a heterocyclic group which may be substituted; $R^{1c}$ is a group which is bonded through carbon; $R^{2c}$ is a hydrogen atom or an aliphatic hydrocarbon group; and $R^{3c}$ is a hydrogen atom or an aliphatic hydrocarbon group.

As the "$C_{6-18}$ aryl ring which may be substituted" represented by $B^3$, the same one as the "$C_{6-18}$ aryl ring which may be substituted" represented by $B^1$ can be used.

As the "heterocyclic group" of the "heterocyclic group which may be substituted" represented by $C^3$, the same one as the above-mentioned heterocyclic group can be used. Among these, a 5- or 6- membered aromatic monocyclic heterocyclic group having one or more (preferably 1 to 4, more preferably 1 to 2) heteroatoms selected from oxygen, sulfur and nitrogen atoms and the like, such as thienyl (e.g., 3-thienyl), pyridyl (3-pyridyl) and the like, is preferred.

As the substituent of the "heterocyclic group", for example, 1 to 5 substituents, which may be identical or different, selected from halogen, $C_{1-4}$ alkyl which may be halogenated, hydroxy, $C_{1-4}$ alkyloxy which may be halogenated, $C_{1-4}$ alkyloxy-$C_{1-4}$ alkyl (e.g., $C_{1-4}$ alkyloxymethyl), hydroxy $C_{1-4}$ alkyl, $C_{1-4}$ alkyl-carbonyl, carboxy, $C_{1-4}$ alkoxy-carbonyl, cyano, carbamoyl, sulfamoyl, nitro, amino, $C_{1-4}$ alkyl-carbonylamino, $C_{1-4}$ alkoxy-carbonylamino, $C_{1-4}$ alkylsulfonylamino and the like, can be used. The substituents may substitute at substitutable arbitrary positions.

As the "group which is bonded through carbon" represented by $R^{1c}$, the same one as the "group which is bonded through carbon" represented by $R^1$ can be used.

The $R^{1c}$ is preferably a $C_{6-18}$ aryl group which may be substituted, or a $C_{6-18}$ aryl-$C_{1-4}$ alkyl group which may be substituted.

As the "aliphatic hydrocarbon group" represented by $R^{2c}$, the same one as the "aliphatic hydrocarbon group" represented by $R^2$can be used.

As the "aliphatic hydrocarbon group" represented by $R^{3c}$, the same one as the "aliphatic hydrocarbon group" represented by $R^3$ can be used.

The compound (I-III) is preferably a compound (I-IIIa) described below, in which $B^3$ is a benzene ring which may be substituted. As the substituent of the benzene ring represented by $B^{3a}$, the same one as the substituent of the $C_{6-18}$ aryl ring represented by $B^3$ can be used.

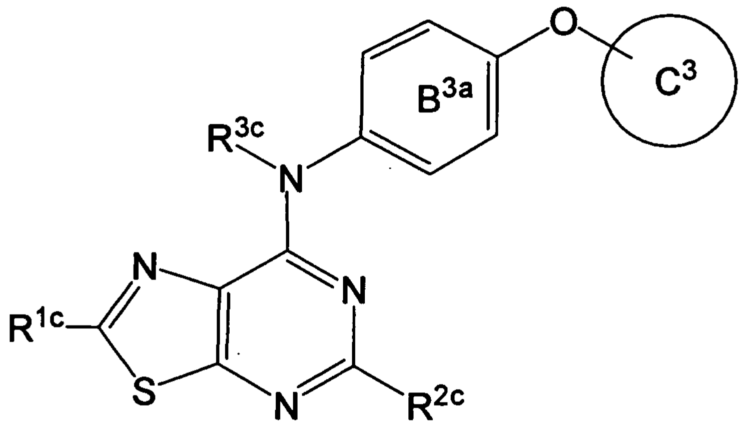

(I-IIIa)

In particular, the compound (I-III) is preferably a compound, in which
$B^3$ is a benzene ring which may be substituted with $C_{1-4}$ alkyl (e.g., methyl),

$C^3$ is a 5- to 8- membered heterocyclic group having 1 to 3 heteroatoms selected from nitrogen, oxygen and sulfur atoms (e.g., a pyridyl group), which may be substituted with $C_{1-4}$ alkyl (e.g., methyl),
$R^{1c}$ is:

(i) a $C_{1-8}$ alkyl group (preferably, a $C_{1-5}$ alkyl group) which may be substituted with substituent(s) selected from the group consisting of:

(a) hydroxy,
(b) $-NR^8-(CH_2)_n-O-C_{1-4}$ alkyl, and
(c) $-NR^8-CO-(CH_2)_n-O-C_{1-4}$ alkyl,

wherein n is an integer from 1 to 4 (preferably, 1 to 3); $R^8$ is a hydrogen atom or a $C_{1-4}$ alkyl group (preferably, a hydrogen atom); and the $C_{1-4}$ alkyl is preferably methyl,

(ii) a $C_{6-18}$ aryl group (e.g., a phenyl group) which may be substituted with substituent(s) selected from the group consisting of:

(a) $-O-C_{1-4}$ alkyl (e.g., methoxy),
(b) $-(CH_2)_m-NR^8-(CH_2)_n-OH$,
(c) $-(CH_2)_m-NR^8-(CH_2)_n-O-C_{1-4}$ alkyl,
(d) $-(CH_2)_m-NR^8-(CH_2)_n-NR^8-CO-C_{1-4}$ alkyl,
(e) $-(CH_2)_m-NR^8-(CH_2)_n-SO_2-C_{1-4}$ alkyl,
(f) $-(CH_2)_m-NR^8-CO-(CH_2)_n-O-C_{1-4}$ alkyl,
(g) $-(CH_2)_m-NR^8-CO-(CH_2)_n-NR^6R^7$,
(h) $-NR^6R^7$, and
(i) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$,

wherein m is an integer from 0 to 4 (preferably, 1 to 3), n is an integer from 1 to 4 (preferably, 1 to 3), and when m or n (particularly, n) is two or more, a part of $-CH_2-CH_2-$ moieties thereof may be replaced by $-CH=CH-$; $R^6$ and $R^7$ are identical or different and are each a hydrogen atom or a $C_{1-4}$ alkyl group (preferably, a hydrogen atom or a methyl group); $R^8$ is a hydrogen atom, a $C_{1-4}$ alkyl group (e.g., a methyl group) or a $C_{1-4}$ alkyl-carbonyl group (e.g., a methylcarbonyl group); and the $C_{1-4}$ alkyl is preferably methyl,

(iii) a $C_{6-18}$ aryl-$C_{1-4}$ alkyl group (e.g., a benzyl group) which may be substituted with substituent(s) selected from the group consisting of:

(a) nitro, and
(b) -O-$C_{1-4}$ alkyl (e.g., methoxy),

(iv) a carbamoyl group which may be substituted with $-(CH_2)_n$-O-$C_{1-4}$ alkyl [wherein n is an integer from 1 to 4 (preferably, 1 to 2) and the $C_{1-4}$ alkyl is preferably methyl],
(v) a 5- to 8- membered cyclic amino-carbonyl group (e.g., piperidinomethyl) which may be substituted with hydroxy, or
(vi) a 5- to 8- membered heterocyclyl-$C_{1-4}$ alkyl group having 1 to 3 heteroatoms selected from nitrogen, oxygen and sulfur atoms (e.g., piperidinomethyl), which may be substituted with hydroxy,
$R^{2c}$ is a hydrogen atom, and

$R^{3c}$ is a hydrogen atom.

[Compound (I-IV)]

A compound represented by Formula:

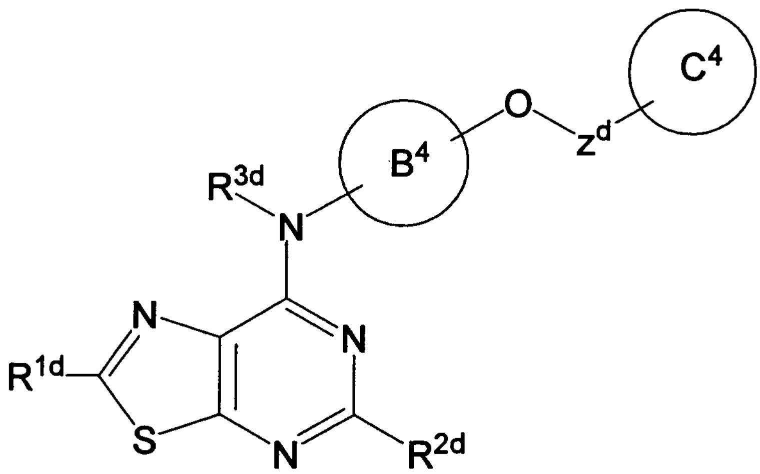

(I-IV)

wherein $B^4$ is a $C_{6-18}$ aryl ring which may be substituted; $C^4$ is a heterocyclic group which may be substituted; $R^{1d}$ is a group which is bonded through carbon; $R^{2d}$ is a hydrogen atom or an aliphatic hydrocarbon group; $R^{3d}$ is a hydrogen atom or an aliphatic hydrocarbon group; and $Z^d$ is a $C_{1-4}$ alkylene group which may be substituted.
As the "$C_{6-18}$ aryl ring which may be substituted" represented by $B^4$, the same one as the "$C_{6-18}$ aryl ring which may be substituted" represented by $B^1$ can be used.
As the "heterocyclic group which may be substituted" represented by $C^4$, the same one as the "heterocyclic group which may be substituted" represented by $C^3$ can be used.
As the "$C_{1-4}$ alkylene group which may be substituted" represented by $Z^d$, the same one as the "$C_{1-4}$ alkylene group which may be substituted" represented by $Z^b$ can be used.
As the "group which is bonded through carbon" represented by $R^{1d}$, the same one as the "group which is bonded through carbon" represented by $R^1$ can be used.
The $R^{1d}$ is preferably a $C_{6-18}$ aryl group which may be substituted, or a $C_{6-18}$ aryl-$C_{1-4}$ alkyl group which may be substituted.
As the "aliphatic hydrocarbon group" represented by $R^{2d}$, the same one as the "aliphatic hydrocarbon group" represented by $R^2$ can be used.
As the "aliphatic hydrocarbon group" represented by $R^{3d}$, the same one as the "aliphatic hydrocarbon group" represented by $R^3$ can be used.
The compound (I-IV) is preferably a compound (I-IVa) described below, in which $B^4$ is a benzene ring which may be substituted. As the substituent of the benzene ring represented by $B^{4a}$, the same one as the substituent of the $C_{6-18}$ aryl ring represented by $B^4$ can be used.

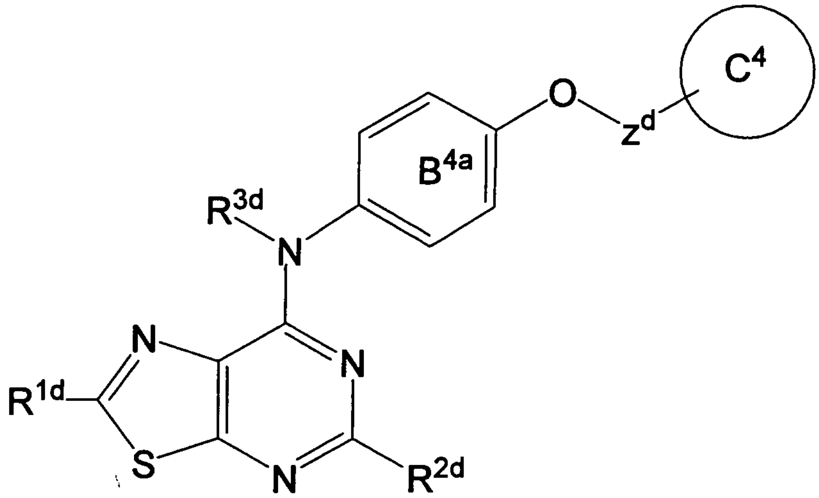

(I-IVa)

In particular, the compound (I-IV) is preferably a compound in which
$B^4$ is a benzene ring which may be substituted with halogen atom(s) (e.g., a chlorine atom),
$C^4$ is a 5- to 8- membered heterocyclic group having 1 to 3 heteroatoms selected from nitrogen, oxygen and sulfur atoms (e.g., a thienyl group),
$R^{1d}$ is a $C_{6-18}$ aryl group (e.g., a phenyl group) which may be substituted with $-(CH_2)_m-NR^8-CO-(CH_2)_n-OH$ [wherein m is an integer from 0 to 4 (preferably, 1 to 3, specifically preferably, 1), n is an integer from 1 to 4 (preferably, 1), and $R^8$ is a hydrogen atom or $C_{1-4}$alkyl (preferably, a hydrogen atom)],
$R^{2d}$ is a hydrogen atom,
$R^{3d}$ is a hydrogen atom, and
$Z^d$ is a $C_{1-4}$ alkylene group (preferably, methylene).

[Compound (I-V)]

In the above-mentioned formula (I), the compound (I-V) is preferably a compound, in which
A is a $C_{6-18}$ aryl group (e.g., a phenyl group) which may be substituted with 1 to 5 substituents selected from the group consisting of:

(i) a $C_{6-18}$ aryl-oxy group (preferably, a phenyloxy group) which may be substituted with substituent(s) selected from the group consisting of:

(a) a halogen atom,
(b) $C_{1-4}$alkyl which may be halogenated, and
(c) $C_{1-4}$ alkyloxy which may be halogenated,

(ii) a $C_{6-18}$ aryl-$C_{1-4}$ alkyl-oxy group (said $C_{1-4}$ alkyl may be substituted with $C_{1-4}$ alkyl which may be halogenated) (preferably, a benzyloxy group), which may be substituted with substituent(s) selected from the group consisting of:

(a) a halogen atom,
(b) $C_{1-4}$ alkyl which may be halogenated, and
(c) cyano,

(iii) a 5- to 8- membered heterocyclyl-oxy group having 1 to 3 heteroatoms selected from nitrogen, oxygen and sulfur atoms, which may be substituted with $C_{1-4}$ alkyl,
(iv) a 5- to 8- membered heterocyclyl-$C_{1-3}$ alkyl-oxy group having 1 to 3 heteroatoms selected from nitrogen, oxygen and sulfur atoms (e.g., said heterocyclyl-methyl-oxy),
(v) a $C_{6-18}$aryl-$C_{1-4}$ alkyl group (e.g., a phenyl-$C_{1-4}$ alkyl group),
(vi) a halogen atom,
(vii) a $C_{1-4}$ alkyl group which may be substituted with substituent(s) selected from the group consisting of:

(a) a halogen atom,

(b) hydroxy, and
(c) $C_{1-4}$alkyloxy, and

(viii) a $C_{1-4}$ alkyloxy group,

$R^1$ is :

(i) a $C_{1-8}$ alkyl group (e.g., a $C_{1-5}$ alkyl group) which may be substituted with substituent(s) selected from the group consisting of:

(a) hydroxy,
(b) $-NR^8-(CH_2)_n-O-C_{1-4}$ alkyl,
(c) $-NR^8-CO-C_{1-4}$ alkyl, and
(d) $-NR^8-CO-(CH_2)_n-O-C_{1-4}$ alkyl,

wherein n is an integer from 1 to 4, and $R^8$ is a hydrogen atom or a $C_{1-4}$alkyl group,
(ii) a $C_{3-8}$ cycloalkyl group (e.g., a cyclopropyl group),
(iii) a $C_{6-18}$ aryl group (e.g., a phenyl group) which may be substituted with substituent(s) selected from the group consisting of:

(a) a halogen atom,
(b) nitro,
(c) $-NR^6R^7$,
(d) $-O-C_{1-4}$ alkyl,
(e) $-(CH_2)_m-NR^8-(CH_2)_n-OH$,
(f) $-(CH_2)_m-NR^8-(CH_2)_n-O-C_{1-4}$ alkyl,
(g) $-(CH_2)_m-NR^8-(CH_2)_n-SO_2-C_{1-4}$ alkyl,
(h) $-(CH_2)_m-NR^8-(CH_2)_n-NR^8-CO-C_{1-4}$ alkyl,
(i) $-(CH_2)_m-O-(CH_2)_n-O-C_{1-4}$ alkyl,
(j) $-(CH_2)_m-NR^8-CO-(CH_2)_n-OH$,
(k) $-(CH_2)_m-NR^8-CO-(CH_2)_n-O-C_{1-4}$ alkyl,
(1) $-(CH_2)_m-NR^8-CO-(CH_2)_n-SO_2-C_{1-4}$ alkyl,
(m) $-(CH_2)_m-NR^8-CO-NR^8-(CH_2)_n-SO_2-C_{1-4}$ alkyl,
(n) $-(CH_2)_m-NR^8-SO_2-C_{1-4}$ alkyl,
(O) $-(CH_2)_m-NR^8-CO-(CH_2)_n-O-(CH_2)_n-O-C_{1-4}$ alkyl,
(p)$-(CH_2)_m-NR^8-CO-(CH_2)_n-NR^6R^7$, and
(q) a 5- to 8- membered heterocyclyl-$C_{1-4}$ alkyl group having 1 to 3 heteroatoms selected from nitrogen, oxygen and sulfur atoms (e.g., azabicyclo[3.1.0]hex-6-yl-methyl), which may be substituted with $-(CH_2)_n-OH$,

wherein m is an integer from 0 to 4, n is an integer from 1 to 4, and when m or n is two or more, a part of $-CH_2-CH_2-$ moieties thereof may be replaced by -CH=CH-; $R^6$ and $R^7$ are identical or different and are each a hydrogen atom or a $C_{1-4}$ alkyl group; and $R^8$ is a hydrogen atom, a $C_{1-4}$ alkyl group, or a $C_{1-4}$ alkyl-carbonyl group,
(iv) a $C_{6-18}$ aryl-$C_{1-4}$ alkyl group (e.g., a benzyl group) which may be substituted with substituent(s) selected from the group consisting of:

(a) nitro, and
(b) $-O-C_{1-4}$ alkyl,

(v) a carbamoyl group which may be substituted with substituent(s) selected from the group consisting of:

(a) $-(CH_2)_n-OH$,
(b) $-(CH_2)_n-NR^6R^7$,
(c) $-(CH_2)_n-O-C_{1-4}$ alkyl,
(d) $-(CH_2)_m-O-(CH_2)_n-OH$,
(d) $-(CH_2)_n-NR^8-(CH_2)_n-SO_2-C_{1-4}$ alkyl,
(e) $-(CH_2)_n-NR^8-CO-(CH_2)_n-OH$,
(f) $-(CH_2)_n-NR^8-CO-(CH_2)_n-NR^6R^7$, and
(g) $-(CH_2)_n-NR^8-CO-(CH_2)_n$-heterocyclic group (for example, a 5- to 8- membered heterocyclic group having 1

to 3 heteroatoms selected from nitrogen, oxygen and sulfur atoms),

wherein m is an integer from 0 to 4, n is an integer from 1 to 4, and when m or n is two or more, a part of $-CH_2-CH_2-$ moieties thereof may be substituted with -CH=CH-; $R^6$ and $R^7$ are identical or different and are each a hydrogen atom or a $C_{1-4}$ alkyl group which has hydroxy;
and $R^8$ is a hydrogen atom or a $C_{1-4}$ alkyl group,
(vi) a 5- to 8- membered cyclic amino-carbonyl group (e.g., a piperidinocarbonyl group) which may be substituted with substituent(s) selected from the group consisting of:

(a) hydroxy,
(b) $-(CH_2)_n-NR^6R^7$, and
(c) $-CO-(CH_2)_n-O-C_{1-4}$ alkyl,

wherein n is an integer from 1 to 4, when n is two or more, a part of $-CH_2-CH_2-$ moieties thereof may be replaced by -CH=CH-; and $R^6$ and
$R^7$ are identical or different and are each a hydrogen atom or a $C_{1-4}$ alkyl group,
(vii) a 5- to 8- membered heterocyclic group having 1 to 3 heteroatoms selected from nitrogen, oxygen and sulfur atoms, which may be substituted with substituent(s) selected from the group consisting of:

(a) $-(CH_2)_m-NR^8-CO-(CH_2)_n-OH$, and
(b) $-(CH_2)_m-NR^8-CO-(CH_2)_n-SO_2-C_{1-4}$ alkyl,

wherein m is an integer from 0 to 4, n is an integer from 1 to 4, and $R^8$ is a hydrogen atom or a $C_{1-4}$ alkyl group, or
(viii) a 5- to 8- membered heterocyclyl-$C_{1-4}$ alkyl group having 1 to 3 heteroatoms selected from nitrogen, oxygen and sulfur atoms (e.g., a piperidinomethyl group), which may be substituted with hydroxy,

$R^2$ is a hydrogen atom,
X is $-NR^3-$, and
$R^3$ is a hydrogen atom or a $C_{1-3}$ alkyl group (preferably, a hydrogen atom).

The salt of the compound represented by Formula (I) or Formula (Ia) is exemplified by metal salts, ammonium salt, salts with organic bases, salts with inorganic acids, salts with organic acids, salts with basic or acidic amino acids, and the like. Suitable examples of the metal salt include, for example, alkali metal salts such as sodium salt, potassium salt and the like; alkaline earth metal salts such as calcium salt, magnesium salt, barium salt and the like; aluminum salt; and the like. Suitable examples of the salt with organic base include, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like. Suitable examples of the salt with inorganic acid include, for example, salts with hydrochloric acid, hydrobromic acid, nitric acid, sulfuric acid, phosphoric acid and the like. Suitable examples of salt with organic acid include, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Suitable examples of the salt with basic amino acid include, for example, salts with arginine, lysine, ornithine and the like. Suitable examples of the salt with acidic amino acid include, for example, salts with aspartic acid, glutamic acid and the like.

Among these, pharmaceutically acceptable salts are preferred. For example, when an acidic functional group is present in the compound, inorganic salts such as alkali metal salts (e.g., sodium salt, potassium salt, etc.), alkaline earth metal salts (e.g., calcium salt, magnesium salt, barium salt, etc.) and the like, ammonium salt, and the like may be mentioned. Also, when a basic functional group is present in the compound, for example, salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like, may be mentioned.

As Compound (Ia), specifically preferably is a compound in which A is an aryl group which is substituted with a group represented by Formula -Y-B (wherein, Y is a single, bond or a spacer, and B is an aryl group or a heterocyclic group, each of which may be substituted), and which may be further substituted; $R^1$ is a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, or a heterocyclyl-$C_{1-4}$ alkyl group, each of which may be substituted; and $R^2$ is a hydrogen atom.

[Production Method]

Hereinafter, a method of producing Compound (Ia) of the present invention will be described.

Compound (Ia) of the present invention can be obtained, for example, according to a method represented by the following Reaction scheme or a method analogous thereto.

Compounds (II) to (VIII) in Reaction Scheme include salts thereof, and the salts are, for example, ones as defined in Compound (Ia).

The compounds obtained in each process may be isolated from the reaction mixture by an ordinary method, and easily purified by separation means such as recrystallization, distillation, chromatography, etc., though it may be used in next reaction as the reaction solution as it is, or as the crude product.

Reaction Schemes are schematically shown below, and each symbol of the compounds has the same meaning as defined above.

Compound (Ia) of the present invention can be prepared by, for example, reacting a compound represented by Formula:

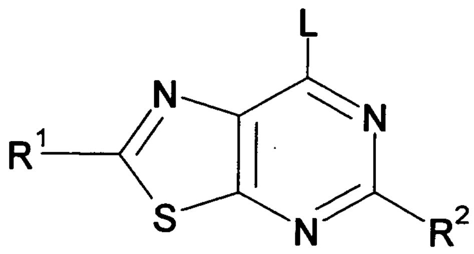

(II)

wherein each symbol has the same meaning as defined above, or a salt thereof, with a compound represented by Formula:

G-X-A (III)

wherein each symbol has the same meaning as defined above, or a salt thereof.

When X is $-NR^3-$, -O-, or -S-, G is mainly a hydrogen atom, but it may also be an alkali metal such as lithium, sodium, potassium, cesium, etc. When X is $-CHR^3-$, G is preferably a metal such as lithium, halogenated magnesium, copper, etc.

The compound (III) or a salt thereof is used in an amount of 1 to 5 moles equivalent and preferably 1 to 2 moles equivalent, to the compound (II), and the reaction is preferably carried out in a solvent. In addition, a base may be used in an amount of about 1 to 10 moles equivalent and preferably 1 to 2 moles equivalent.

As the leaving group represented by L in the above-mentioned formula, a halogen atom such as chlorine, bromine, iodine, etc.; a group represented by formula $-S(O)_kR^{10}$ [wherein, k is 0, 1 or 2, and $R^{10}$ is a lower ($C_{1-4}$) alkyl group such as methyl, ethyl, propyl, etc., a $C_{6-10}$ aryl group such as a benzyl group, phenyl, tolyl, etc., or the like]; or a group represented by formula $-OR^{10}$ [wherein, $R^{10}$ represents the same meaning as defined above], can be used.

Examples of the solvent for the above-mentioned reaction include halogenated hydrocarbons such as dichloromethane, chloroform, carbon tetrachloride and 1,2-dichloroethane, aromatic hydrocarbons such as benzene, toluene, and xylene, alcohols such as methanol, ethanol, isopropanol, and t-butanol, ethers such as diethyl ether, tetrahydrofuran, and dioxane, acetone, acetonitrile, ethyl acetate, N,N-dimethylformamide, N,N-dimethylacetamide, 1-methyl-2-pyrrolidone, dimethylsulfoxide, hexamethylphosphoramide, and water, a mixed solvent thereof and the like.

As the base for the above-mentioned reaction, inorganic bases, organic bases, and the like can be used, and specific examples include sodium hydroxide, potassium hydroxide, sodium carbonate, potassium carbonate, sodium hydrogencarbonate, potassium hydrogencarbonate, triethylamine, pyridine, N,N-dimethylaminopyridine, sodium methoxide, sodium ethoxide, potassium t-butoxide, sodium hydride, sodium amide, diazabicycloundecene (DBU), and the like.

The above-mentioned reaction can be carried out under a cooling condition, a room temperature, or a heating condition (at about 40 to 200°C, preferably about 40 to 160°C), and the reaction time is generally about 1 to 20 hours, preferably about 1 to 10 hours.

In Compound (Ia), ones in which X is -SO- or $-SO_2-$ can be produced by subjecting ones in which X is -S- to an oxidation reaction. Examples of an oxidizing agent for use in such reaction include m-chloroperbenzoic acid, hydrogen peroxide, peracetic acid, t-butylhydroperoxide, potassium peroxysulfate, potassium permanganate, sodium perborate, sodium periodate, sodium hypochlorite, halogen, and the like. The oxidizing agent is used in an amount of about 1 to 1.5 moles equivalent to produce the compound in which X is -SO-, and about 2 to 3 moles equivalent to produce the

compound in which X is $-SO_2-$, to the raw material compound. Such reaction solvent is not particularly limited if it does not react with the oxidizing agent, and examples include halogenated hydrocarbons such as dichloromethane, chloroform, carbon tetrachloride and 1,2-dichloroethane, aromatic hydrocarbons such as benzene, toluene, and xylene, alcohols such as methanol, ethanol, isopropanol, and t-butanol, ethers such as diethyl ether, tetrahydrofuran, and dioxane, carboxylic acids such as acetic acid and trifluoroacetic acid, acetonitrile, ethyl acetate, N,N-dimethylformamide, N,N-dimethylacetamide, 1-methyl-2-pyrrolidone, dimethylsulfoxide, and water, a mixed solvent thereof and the like. The reaction can be carried out under a cooling condition, a room temperature, or a heating condition, and the reaction time is generally about 1 to 20 hours, preferably about 1 to 10 hours.

The obtained compound (Ia) of the present invention is subjected to a further introduction of substituent or conversion of functional groups by appropriate means of a method known *per se* to produce a compound within the range of the present invention. For the conversion of substituent, generally known methods can be used, and examples include a conversion to a carboxy group by hydrolysis of ester, a conversion to a carbamoyl group by amidation of a carboxy group, a conversion to a hydroxymethyl group by reduction of a carboxy group, a conversion to alcohol by reduction or alkylation of a carbonyl group, oximation of a carbonyl group, acylation of an amino group, alkylation of an amino group, substitution/amination of active halogen by amine, alkylation of a hydroxy group, substitution/amination of a hydroxy group, and the like. In the case where a reactive substituent which generates a reaction other than the aimed reaction is present when processing the introduction of substituent or conversion of functional group, the compound within the range of the present invention can also be produced by initially introducing a protective group to the reactive substituent, if necessary, by means known *per se*, and then removing the protective group by means known *per se* after the aimed reaction.

Compound (Ia), which is the product obtained from the reaction, may be produced as an single compound or a mixture.

For thus-obtained compound (Ia) of the present invention, target product can be isolated and purified with high purity from a reaction solution by means known *per se,* for example, solvent extraction, concentration, neutralization, filtration, crystallization, recrystallization, column chromatography, high-performance liquid chromatography, recrystallization and the like, from the reaction mixture.

As the raw material compound (III) of the present production method, commercially available one is used, or it can be produced according to means known *per se.*

The raw material compound (II) of the present production method can be produced, for example, according to a method represented by the following scheme. Herein, compounds (IIa), (IIb), (IIc), and (IId) are encompassed in the compound (II).

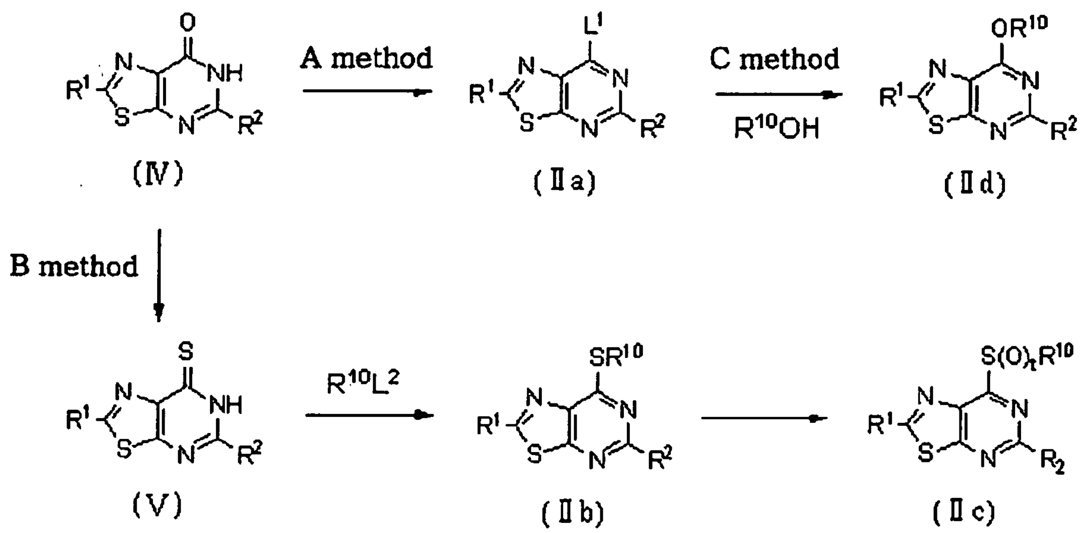


wherein, $L^1$ and $L^2$ are halogen atoms, $R^{10}$ represents the same meaning as defined above, and t is 1 or 2.

That is, as the A method, the compound (IV) is reacted with a halogenating agent to produce the compound (IIa). As the B method, the compound (IV) is reacted with a sulfurating agent to produce the compound (V) and subsequently reacted with a compound represented by $R^{10}L^2$ under the presence of a base to obtain the compound (IIb), and further, the obtained compound is subjected to an oxidation reaction to produce the compound (IIc). As the C method, the compound (IIa) is reacted with a compound represented by $R^{10}OH$ under the presence of a base to produce the

compound (IId).

As the halogenating agent which is used in the A method, for example, phosphorous oxychloride, phosphorous pentachloride, phosphorus trichloride, sulfuryl chloride, phosphorus tribromide, and the like, can be used in an amount of about 1 to 5 equivalent. The reaction may be carried out under the presence of a base such as diethylaniline, dimethylaniline, pyridine, and the like. While the reaction may be carried out without a solvent, the following reaction solvents, for example, halogenated hydrocarbons such as dichloromethane, chloroform, carbon tetrachloride and 1,2-dichloroethane, aromatic hydrocarbons such as benzene, toluene, and xylene, ethers such as diethyl ether, tetrahydrofuran, and dioxane, acetonitrile, ethyl acetate, and the like may be used. The reaction is carried out under a cooling condition, a room temperature, or a heating condition, and the reaction time is generally about 1 to 20 hours, preferably about 1 to 10 hours.

As the sulfurating agent which is used in the process for producing the compound (V) from the compound (IV) in the B method, for example, Lawesson's Reagent, diphosphorus pentasulfide, and the like can be used in an amount of about 1 to 5 equivalent. Examples of the reaction solvent include halogenated hydrocarbons such as dichloromethane, chloroform, carbon tetrachloride and 1,2-dichloroethane, aromatic hydrocarbons such as benzene, toluene, and xylene, ethers such as diethyl ether, tetrahydrofuran, and dioxane, and the like. The reaction is carried out under a room temperature, or a heating condition, and the reaction time is generally about 1 to 20 hours, preferably about 1 to 10 hours.

As $R^{10}L^2$ which is used in the process for producing the compound (IIb) from the compound (V) in the B method, for example, methyl iodide, benzyl chloride, benzyl bromide, and the like, can be used in an amount of about 1 to 5 equivalent. Examples of the base include sodium hydroxide, potassium hydroxide, sodium carbonate, potassium carbonate, sodium hydrogencarbonate, potassium hydrogencarbonate, triethylamine, pyridine, N,N-dimethylaminopyridine, sodium methoxide, sodium ethoxide, potassium t-butoxide, sodium hydride, sodium amide, diazabicycloundecene (DBU), and the like. Examples of the reaction solvent include halogenated hydrocarbons such as dichloromethane, chloroform, carbon tetrachloride and 1,2-dichloroethane, aromatic hydrocarbons such as benzene, toluene, and xylene, alcohols such as methanol, ethanol, isopropanol, and t-butanol, ethers such as diethyl ether, tetrahydrofuran, and dioxane, acetone, acetonitrile, ethyl acetate, N,N-dimethylformamide, N,N-dimethylacetamide, 1-methyl-2-pyrrolidone, dimethylsulfoxide, hexamethylphosphoramide, and water, a mixed solvent thereof and the like. The reaction is carried out under a cooling condition, a room temperature, or a heating condition, and the reaction time is generally about 1 to 20 hours, preferably about 1 to 10 hours.

As the oxidizing agent which is used in the process for producing the compound (IIc) from the compound (IIb) in the B method, for example, m-chloroperbenzoic acid, hydrogen peroxide, peracetic acid, t-butylhydroperoxide, potassium peroxysulfate, potassium permanganate, sodium perborate, sodium periodate, sodium hypochlorite, halogen, and the like, can be used. The oxidizing agent is used in an amount of about 1 to 1.5 equivalent to the compound (IIb) to produce the compound (IIc) having t = 1, and is used in an amount of about 2 to 3 equivalent to the compound (IIb) to produce the compound (IIc) having t = 2. The reaction solvent is not particularly limited if it does not react with the oxidizing agent, and examples include halogenated hydrocarbons such as dichloromethane, chloroform, carbon tetrachloride and 1,2-dichloroethane, aromatic hydrocarbons such as benzene, toluene, and xylene, alcohols such as methanol, ethanol, isopropanol, and t-butanol, ethers such as diethyl ether, tetrahydrofuran, and dioxane, carboxylic acids such as acetic acid and trifluoroacetic acid, acetonitrile, ethyl acetate, N,N-dimethylformamide, N,N-dimethylacetamide, 1-methyl-2-pyrrolidone, dimethylsulfoxide and water, a mixed solvent thereof, and the like. The reaction can be carried out under a cooling condition, a room temperature, or a heating condition, and the reaction time is generally about 1 to 20 hours, preferably about 1 to 10 hours.

As $R^{10}OH$ which is used in the process for producing the compound (IId) from the compound (IIa) in the C method, for example, methanol, ethanol, phenol, and the like, can be used in an amount of 1 to 10 equivalent. Examples of the base include sodium hydroxide, potassium hydroxide, sodium carbonate, potassium carbonate, sodium hydrogencarbonate, potassium hydrogencarbonate, triethylamine, pyridine, N,N-dimethylaminopyridine, sodium methoxide, sodium ethoxide, potassium t-butoxide, sodium hydride, sodium amide, diazabicycloundecene (DBU), and the like. Examples of the reaction solvent include halogenated hydrocarbons such as dichloromethane, chloroform, carbon tetrachloride and 1,2-dichloroethane, aromatic hydrocarbons such as benzene, toluene, and xylene, ethers such as diethyl ether, tetrahydrofuran, and dioxane, acetone, acetonitrile, ethyl acetate, N,N-dimethylformamide, N,N-dimethylacetamide, 1-methyl-2-pyrrolidone, dimethylsulfoxide, hexamethylphosphoramide and water, a mixed solvent thereof, and the like. The reaction can be carried out under a cooling condition, a room temperature, or a heating condition, and the reaction time is generally about 1 to 20 hours, preferably about 1 to 10 hours.

In addition, the compound (IIb) can be produced, for example, according to a method represented by the following scheme.

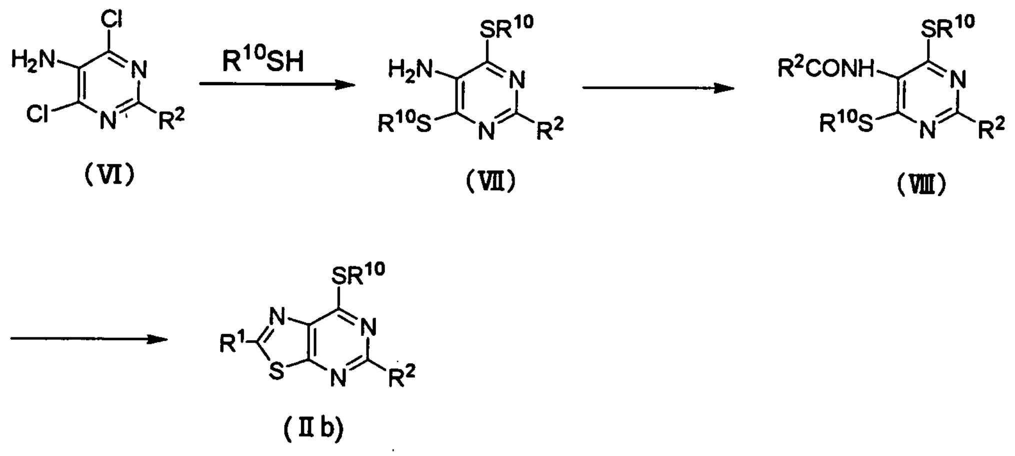


(VI) (VII) (VIII)

(IIb)

wherein, each symbol has the same meaning as defined above.

That is, the compound (VI) is reacted with about 2 to 3 equivalent amount of a compound represented by the formula $R^{10}SH$ under the presence of about 2 to 4 equivalent amount of a base to produce the compound (VII), the compound (VII) is subsequently reacted with about 1 to 5 equivalent amount of an acylating agent to produce the compound (VIII), and then the compound (VIII) is reacted with about 1 to 5 equivalent amount of a sulfurating agent to produce the compound (IIb).

As a compound represented by the formula $R^{10}SH$ which is used in the process for producing the compound (VII) from the compound (VI) in the present method, for example, methanethiol, ethanethiol, thiophenol, benzyl mercaptan, and the like can be used. Examples of the base include sodium hydroxide, potassium hydroxide, sodium carbonate, potassium carbonate, sodium hydrogencarbonate, potassium hydrogencarbonate, triethylamine, pyridine, N,N-dimethylaminopyridine, sodium methoxide, sodium ethoxide, potassium t-butoxide, sodium hydride, sodium amide, diazabicycloundecene (DBU), and the like. In addition, sodium thiomethoxide may be used when $R^{10}$ is a methyl group. Examples of the reaction solvent include halogenated hydrocarbons such as dichloromethane, chloroform, carbon tetrachloride and 1,2-dichloroethane, aromatic hydrocarbons such as benzene, toluene, and xylene, alcohols such as methanol, ethanol, isopropanol, and t-butanol, ethers such as diethyl ether, tetrahydrofuran, and dioxane, acetone, acetonitrile, ethyl acetate, N,N-dimethylformamide, N,N-dimethylacetamide, 1-methyl-2-pyrrolidone, dimethylsulfoxide, hexamethylphosphoramide and water, a mixed solvent thereof, and the like. The reaction is carried out under a cooling condition, a room temperature, or a heating condition, and the reaction time is generally about 1 to 20 hours, preferably about 1 to 10 hours.

As the acylating agent which is used in the process for producing the compound (VIII) from the compound (VII) in the present method, for example, an acid chloride represented by formula $R^2COCl$, an acid anhydride represented by formula $(R^2CO)_2O$, and the like may be used, and particularly the acid chloride is preferred. The reaction may be carried out under the presence of a base, and examples of the base include sodium carbonate, potassium carbonate, sodium hydrogencarbonate, potassium hydrogencarbonate, triethylamine, pyridine, N,N-dimethylaminopyridine, diazabicycloundecene (DBU), and the like. Examples of the reaction solvent include halogenated hydrocarbons such as dichloromethane, chloroform, carbon tetrachloride and 1,2-dichloroethane, aromatic hydrocarbons such as benzene, toluene, and xylene, ethers such as diethyl ether, tetrahydrofuran, and dioxane, acetone, acetonitrile, ethyl acetate, N, N-dimethylformamide, N,N-dimethylacetamide, 1-methyl-2-pyrrolidone, dimethylsulfoxide, hexamethylphosphoramide, and the like. The reaction can be carried out under a room temperature, or a heating condition, and the reaction time is generally about 1 to 20 hours, preferably about 1 to 10 hours.

As the sulfurating agent which is used in the process for producing the compound (IIb) from the compound (VIII), for example, Lawesson's Reagent, diphosphorus pentasulfide, and the like can be used. Examples of the reaction solvent include halogenated hydrocarbons such as dichloromethane, chloroform, carbon tetrachloride and 1,2-dichloroethane, aromatic hydrocarbons such as benzene, toluene, and xylene, ethers such as diethyl ether, tetrahydrofuran, and dioxane, and the like. The reaction is carried out under a room temperature, or a heating condition, and the reaction time is generally about 1 to 20 hours, preferably about 1 to 10 hours.

In accordance with a variety of substituents of the raw material compound (II), the raw material compounds (II) having different substituents can be produced by means of the substituent conversion with use of the compound produced by the above-mentioned production method, as a raw material. For the conversion of substituent, generally known methods can be used, and examples include a conversion to a carbamoyl group by hydrolysis/amidation of ester, a conversion to a hydroxymethyl group by reduction of a carboxy group, a conversion to alcohol by reduction or alkylation of a carbonyl group, oximation of a carbonyl group, acylation of an amino group, alkylation of an amino group, substitution/amination of active halogen by amine, alkylation of a hydroxy group, substitution/amination of a hydroxy group, and the like. In the case where a reactive substituent which generates a reaction other than the aimed reaction is present when processing the introduction of substituent or conversion of functional group, the raw material compound (II) can also be produced by initially introducing a protective group to the reactive substituent, if necessary, by means known *per se,* and then removing the protective group by means known *per se* after the aimed reaction.

Thus-obtained Compound (Ia) can be isolated and purified by separation means known *per se,* for example, concentration, vacuum concentration, solvent extraction, crystallization, recrystallization, partition, chromatography and the like.

When Compound (Ia) is obtained in a free form, the free form can be converted to a target salt form by methods known *per se* or methods analogous thereto. Conversely, when Compound (Ia) is obtained in a salt form, the salt form can be converted to the free form or other target salts by methods known *per se* or methods analogous thereto.

When Compound (Ia) has the form of isomer such as optical isomer, stereoisomer, regioisomer, rotational isomer and the like, Compound (Ia) encompasses such isomers and mixtures thereof. For example, when Compound (Ia) has optical isomers, optical isomers obtained by resolution of racemates are also included in Compound (Ia). Such the isomers can be obtained as single product by synthetic techniques and separation techniques known *per se* (concentration, solvent extraction, column chromatography, recrystallization, etc.).

Compound (Ia) may be in a crystal form, and Compound (Ia) encompasses both single crystal forms and mixed crystal forms. Crystals can be prepared by crystallization according to crystallization methods known *per se.*

Compound (Ia) may be either a solvate (e.g., hydrate, etc.) or a non-solvate, and encompasses both forms.

Compounds labeled with isotopes (e.g., $^{3}H$, $^{14}C$, $^{35}S$, $^{125}I$, etc.) and the like are also included in Compound (Ia).

A prodrug of Compound (Ia) or salts thereof (hereinafter, simply referred to as Compound (Ia)) refers to a compound that is converted to Compound (Ia) by a reaction induced by an enzyme, gastric acid or the like under the physiological conditions in vivo, that is, a compound converted to Compound (Ia) by enzymatic oxidation, reduction, hydrolysis or the like, or a compound that is converted to Compound (Ia) by gastric acid-induced hydrolysis. Examples of the prodrug of Compound (Ia) include a compound in which an amino group of Compound (Ia) is acylated, alkylated or phosphorylated (e.g., a compound in which an amino group of Compound (Ia) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated or the like); a compound in which a hydroxyl group of Compound (Ia) is acylated, alkylated, phosphorylated or borated (e.g., a compound in which a hydroxyl group of Compound (Ia) is acetylated, palmitoylated, propanoylated, pivaloylated, succinylated, fumarylated, alanylated, dimethylaminomethylcarbonylated or the like); a compound in which a carboxyl group of Compound (Ia) is esterified or amidated (e.g., a compound in which a carboxyl group of Compound (Ia) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methylamidated or the like); and the like. Such the compound can be prepared from Compound (Ia) by a method known *per se.*

In addition, the prodrug of Compound (Ia) may be a compound which is converted to Compound (Ia) under the physiological conditions, as described in "Development of Medicine", Vol. 7, Molecular Design, Hirokawa Shoten, pages 163 to 198 (1990).

Compound (Ia) of the present invention, a salt thereof, or a prodrug thereof (hereinafter, may be simply referred to as the compound of the present invention) has a tyrosine kinase inhibitory action, and it can be used for preventing or treating of tyrosine kinase-dependent diseases in a mammal. The tyrosine kinase-dependent disease includes accelerative cell proliferation diseases caused by abnormal tyrosine kinase enzyme activity.

Examples of tyrosine kinase include HER2 kinase, EGFR kinase, c-erbB-2 kinase, c-erbB-4 kinase, c-met kinase, tie-2 kinase, PDGFR kinase, VEGFR kinase, FGFR kinase, c-Kit kinase, ALK kinase, RET kinase, c-src kinase, lck kinase, Zap 70 kinase, fyn kinase, Syk kinase, p561ck kinase, Abl kinase, JAK kinase, FAK kinase, and the like.

In specific, since the compound of the present invention inhibits HER2 kinase and/or EGFR kinase, it is useful as a therapeutic agent for suppressing proliferation of cancer expressed HER2 kinase and/or EGFR kinase, and also as a prophylactic agent for preventing migration of hormone-dependent cancer to non-hormone dependent cancer. In addition, they have low toxicity (e.g., acute toxicity, chronic toxicity, genetic toxicity, reproductive toxicity, cardiac toxicity, drug interaction, carcinogenicity, etc.) and high water-solubility, and are excellent in the aspects of stability, pharmacokinetics (absorbability, distribution, metabolism, excretion, etc.) and efficacy, thus being useful as medicine.

That is, the compound of the present invention can be used as a safe prophylactic or therapeutic agent for diseases due to an abnormal cellular proliferation such as various cancer (particularly, breast cancer, prostatic cancer, pancreatic cancer, gastric cancer, lung cancer, colon cancer, rectal cancer, esophageal cancer, duodenal cancer, tongue cancer, pharyngeal cancer, cerebral tumor, neurilemoma, non-small cell lung cancer, small cell lung cancer, liver cancer, kidney cancer, bile duct cancer, corpus uteri cancer, cancer of uterine cervix, ovarian cancer, bladder cancer, cutaneous cancer, hemangioma, malignant lymphoma, malignant melanoma, thyroid cancer, bone tumor, angiofibroma, retina sarcoma, penile cancer, infant solid cancer, Kaposi's sarcoma, Kaposi's sarcoma due to AIDS, maxillary antrum tumour,

fibrous histiocytoma, leiomyosarcoma, rhabdomyosarcoma, leucaemia, or the like), atherosclerosis, neoangiogenesis (e.g., neoangiogenesis with growth of solid cancer or sarcoma, neoangiogenesis with metastasis of neoplasm, and neoangiogenesis with diabetic retinopathy), viral illness (HIV infection etc.), or the like.

The tyrosine kinase-dependent disease further includes cardiovascular diseases caused by abnormal tyrosine kinase enzyme activity. Therefore, the compound of the present invention can also be used as a prophylactic or therapeutic agent for cardiovascular diseases such as restenosis.

In addition, the compound of the present invention can also be used as a prophylactic or therapeutic agent for diseases such as osteoporosis, Paget's disease, hypercalcemia, osteoarthritis, Parkinson's disease, epilepsy, brain edema, acute cerebral infarction, diseases caused by allergic reaction including allergic diseases such as asthma, allergic rhinitis, atopic dermatitis, food allergy, contact dermatitis, urticarial rash, conjunctivitis and spring catarrh or by inflammatory reaction, autoimmune diseases such as rheumatoid arthritis, systemic lupus erythematosus and psoriasis, ulcer diseases including chronic ulcerative colitis, fibrosis disease, tumor, diseases caused by an immune reaction including rejection reaction of transplanted organ and graft versus host reaction, diseases caused by antibody-dependent cytotoxicity such as autoimmune hemolytic anemia and myasthenia gravis, thrombosis caused by aggregation of blood platelets, corpulence, Alzheimer's disease, diabetic retinopathy, atherosclerosis, COPD, or the like.

The compound of the present invention is effective as an antitumor agent for prevention or treatment of cancer, in particular, breast cancer, prostatic cancer, pancreatic cancer, gastric cancer, lung cancer, colon cancer, large bowel cancer or the like.

The compound of the present invention has low toxicity, and can be used as it is or as a pharmaceutical composition by admixing with a pharmacologically acceptable carrier, to mammals (e.g., human, horse, cow, dog, cat, rat, mouse, rabbit, pig, monkey, etc.).

Other active component, for example, following agents for hormone therapy, antitumor agents (e.g., chemotreating agents, immunotherapy agents, or agents for inhibiting actions of cell growth factors and receptors thereof, etc.), or the like may be included in a pharmaceutical composition together with the compound of the present invention.

For administering the compound of the present invention as a medicine to mammals such as human, there are ways of administering, for example, oral preparations such as a tablet, a capsule (including a soft capsule and a microcapsule), a powder, a granule and the like; or parenteral preparations such as an injectable preparation, a suppository, a pellet and the like. The "parenteral administration" include administrations by intravenous, intramuscular, subcutaneous, intraorgan route, intranasal, intracutaneous, ocular instillation, intracerebral, intrarectal, intravaginal, interperitoneal, intratumor, tumor-nearby and the like, and administration directly to the lesion.

The amount of administration of the compound of the present invention may vary depending on the administration route, subject disease or the like, however, in the case of administering orally to a patient (from 40 to 80 kg weight) suffering from breast cancer or prostatic cancer as an antitumor agent, for example, the amount of administration is 0. 5 to 100 mg/kg of body weight per a day, preferably 1 to 50 mg/kg of body weight per a day, and more preferably 1 to 25 mg/kg of body weight per a day. It can be administered once or two to three times a day.

The compound of the present invention can be safely orally or parenterally (e.g., topical, rectal, intravenous administration, etc.) administered as it is, or as pharmaceutical compositions mixed with pharmacologically acceptable carriers according to ordinary methods (for example, methods described in the Japanese Pharmacopeia, etc.), such as a tablet (including a sugar-coated tablet and a film-coated tablet), a powder, a granule, a capsule, a liquid, an emulsion, a suspension, an injectable preparation, a suppository, a sustained release preparation, a patch and the like.

Cancer can be further effectively prevented or treated by (1) administration of effective amount of the compound of the invention, and (2) combination of 1 to 3 kinds selected from the group consisting of (i) administration of effective amount of other antitumor agents, (ii) administration of effective amount of agents for hormone therapy, and (iii) non-drug therapy. The non-drug therapy includes surgery, radiotherapy, gene therapy, hyperthermia therapy, freeze therapy, optical laser burning therapy and the like, and two or more of these can be used in combination.

For example, the compound of the present invention can be used in combination with other agents for hormone therapies, antitumor agents (e.g., chemotreating agents, immunotherapy agents, or agents for inhibiting actions of cell growth factors and receptors thereof) etc., (hereinafter, abbreviated as concomitant drug).

The compound of the present invention exhibits an excellent antitumor action when used as a single agent, and the effect can be increased further by concomitantly using (multiple drug combination) one or some of above-mentioned concomitant drugs.

Examples of the "agents for hormone therapy" include fosfestrol, diethylstilbestrol, chlorotrianisene, medroxyprogesterone acetate, megestrol acetate, chlormadinone acetate, cyproterone acetate, danazol, allylestrenol, gestrinone, mepartricine, raloxifene, ormeloxifene, levormeloxifene, antiestrogen (e.g., tamoxifen citrate, toremifene citrate, etc.), pill, mepitiostane, testololactone, aminoglutethimide, LH-RH agonists (e.g., goserelin acetate, buserelin, leuprorelin, etc.), droloxifene, epitiostanol, ethinylestradiol sulfonate, aromatase inhibitors (e.g., fadrozole hydrochloride, anastrozole, letrozole, exemestane, vorozole, formestane, etc.), antiandrogen (e.g., flutamide, bicalutamide, nilutamide, etc.), 5α-reductase inhibitors (e.g., finasteride, epristeride, etc.), adrenocorticotropic hormone agents (e.g., dexamethasone, prednisolone, betamethasone, triamcinolone, etc.), androgen synthesis inhibitor (e.g., abiraterone, etc.), retinoid and drugs to delay metabolism of retinoid (e.g., Liarozole, etc.), and the like, and among them, LH-RH agonists (e.g., goserelin acetate, buserelin, leuprorelin, etc.) are preferable.

Examples of the "chemotreating agents" include alkylating agents, antimetabolites, antitumor antibiotics, plant-derived antitumor agents, and the like.

Examples of the "alkylating agents" include nitrogen mustard, nitrogen mustard-N-oxide hydrochloride, chlorambucil, cychlophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, threosulfan, trophosphamide, zinostatin stimalamer, carboquone, adozelesin, cystemustine, bizelesin, and the like.

Examples of the "antimetabolites" include mercaptopurine, 6-mercaptopurine riboside, thioinosine, methotrexate, enocitabine, cytarabine, cytarabine ocphosphate, ancitabine hydrochloride, 5-Fluouracil agents (e.g., fluorouracil, tegafur, UFT, doxifluridine, carmofur, Galocitabine, emitefur), aminopterin, leucovorin calcium, tabloid, butosine, calcium folinate, calcium levofolinate, cladribine, emitefur, fludarabine, gemcitabine, hydroxycarbamide, pentostatin, piritrexim, idoxuridine, mitoguazone, tiazofurin, ambamustine, and the like.

Examples of the "antitumor antibiotics" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, xorubicin hydrochloride, mitoxantrone hydrochloride, idarubicin hydrochloride, and the like.

Examples of the "plant-derived antitumor agents" include etoposide, etoposide phosphate, vinblastine sulfate, vincristine sulfate, vindesine sulfate, teniposide, paclitaxel, docetaxel, vinorelbine, and the like.

Examples of the "immunotherapy agents (BRM)" include picibanil, krestin, schizophyllan, lentinan, ubenimex, interferon, interleukin, macrophage colony-stimulating factor, granulocyte colony-stimulating factor, erythropoietin, lymphotoxin, BCG vaccine, corynebacterium parvum, levamisole, polysaccharide K, procodazol, and the like.

Examples of "cell growth factors" in the "drugs inhibiting the actions of cell growth factors and receptors thereof" may be any substance promoting cellular propagation, and in general, factors that of the peptide's molecular weight is 20,000 or less, and that exhibit action when bound to a receptor at a low concentration can be used. General examples include (1) EGF (epidermal growth factor) or a substance having substantially same activity [e.g., EGF, heregulin (HER2 ligand) etc.], (2) insulin or a substance having substantially same activity [e.g., insulin, IGF (insulin-like growth factor)-1, IGF-2, etc.], (3) FGF (fibroblast growth factor) or a substance having substantially same activity [e.g., acidic FGF, basic FGF, KGF (keratinocyte growth factor), FGF-10, etc.], (4) other cell growth factors [e.g., CSF (colony stimulating factor), EPO (erythropoietin), IL-2 (interleukin-2), NGF (nerve growth factor), PDGF (platelet-derived growth factor), TGF $\beta$ (transforming growth factor $\beta$), HGF (hepatocyte growth factor), VEGF (vascular endothelial growth factor), etc.], and the like.

The "receptor of cell growth factor" may be any receptors having binding capacity with the above-mentioned cell growth factors. General examples include EGF receptor, heregulin receptor (HER2), insulin receptor, IGF receptor, FGF-1 receptor, FGF-2 receptor, and the like.

The "drug for inhibiting the action of cell growth factor" include trastuzumab (herceptin (trade name), HER2 antibody), imatinib mesylate, ZD1839, cetuximab, and the like.

Other than the above-mentioned drugs, L-asparaginase, aceglatone, procarbazine hydrochloride, cobalt-protoporphyrin complex, Hg-Hematoporphyrin-sodium, topoisomerase I inhibitor (e.g., irinotecan, topotecan, etc.), topoisomerase II inhibitor (e.g., sobuzoxane, etc.), inducer of differentiation (e.g., retinoid, vitamin D, etc.), arterialization inhibitor, $\alpha$-blocker (e.g., tamsulosin hydrochloride, etc.) or the like can also be used.

Among the above-mentioned drugs, LH-RH agonist (e.g., goserelin acetate, buserelin, leuprorelin, etc.), trastuzumab (HER2 antibody) and the like are preferable as a concomitant drug.

With regard to the concomitant use of the compound of the present invention and a concomitant drug, the compound of the present invention and the concomitant drug are free of any limitation on the timing of the administration, and the compound of the present invention and the concomitant drug may be simultaneously administered to the administration object, or may be administered with time difference. The dose of the concomitant drug follows a clinical dose and can be appropriately determined depending on the administration object, administration route, disease, combination and the like.

The mode of administration of the compound of the present invention and the concomitant drug is not particularly limited, as long as the compound of the present invention and the concomitant drug are combined for administration. The administration mode is exemplified by (1) administration of a single preparation obtained by simultaneously formulating the compound of the present invention and the concomitant drug, (2) simultaneous administration through the same administration route of two preparations obtained by separately formulating the compound of the invention and the concomitant drug, (3) administration with a time interval through the same administration route of two preparations

obtained by separately formulating the compound of the present invention and the concomitant drug, (4) simultaneous administration through different administration routes of two preparations obtained by separately formulating the compound of the present invention and the concomitant drug, (5) administration with a time interval through different administration routes of two preparations obtained by separately formulating the compound of the present invention and the concomitant drug (for example, administration in order of the compound of the invention and then the concomitant drug, or administration in the reverse order), or the like.

**Examples**

The present invention will be further explained in detail below by way of Reference Examples, Examples, Preparation Examples and Test Examples. However, the present invention is not limited thereto.

Elution for column chromatography of Reference Examples and Examples was performed under an observation by TLC (Thin Layer Chromatography). For TLC observation, Kieselgel 60 $F_{254}$ plate, manufactured by Merck Co., Ltd., or NH TLC plate, manufactured by Fuji Silysia Chemical Ltd., was used as a TLC plate. For the developing solvent, the same used as an elution solvent in column chromatography was used, and for the detection method, UV detector was used. For silica gel for the column, Kieselgel 60 $F_{254}$ (70 to 230 mesh), manufactured by Merch Co., Ltd., or Chromatorex NH DM1020 (basic silica gel, 100 to 200 mesh), manufactured by Fuji silysia Chemical Ltd. was used. In silica gel column chromatography, the ratio of solvents was represented in capacity ratio of solvents which are mixed, and % was represented in weight percent within the limit of no specially mentioned.

NMR spectra represents proton NMR, which was measured with a VARIAN Gemini-200 (200MHz spectrometer), or a JEOL JNM-AL400 (400 MHz spectrometer) using tetramethylsilane as an internal standard, and the δ values were represented in ppm.

Symbols used in the Reference Examples and the Examples indicate the following meanings.

| | |
|---|---|
| S: | singlet |
| br: | broad (wide) |
| d: | doublet |
| t: | triplet |
| q: | quartet |
| dd: | double doublet |
| m: | multiplet |
| J: | coupling constant |
| Hz: | Hertz |
| DMSO: | dimethylsulfoxide |

Also, for the method of gene modification described below Test Examples, a method described in Maniatis et al., Molecular Cloning, Cold Spring Harbor Laboratory, 1989 or a method described in Appendixes Protocol of reagents was used.

**Reference Example 1**

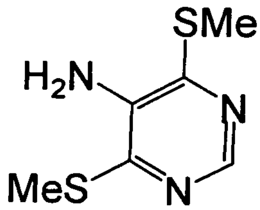

4,6-bis(methylthio)pyrimidin-5-amine

5-amino-4,6-dichloropyrimidine (50.0g) was dissolved in methanol (350ml), a 15% aqueous sodium thiomethoxide solution (430g) was added thereto, and the mixture was stirred at room temperature for 6 hours. Water (700ml) was added thereto, and the mixture was stirred again for 30 minutes. The precipitated crystal was collected by filtration, washed with water, and then dried to obtain the title compound (49.0g).

$^1$H-NMR($CDCl_3$) δ:2.64(6H,s),3.68-3.84(2H,m),8.41(1H,s).

**Reference Example 2**

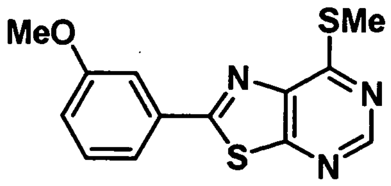

2-(3-methoxyphenyl)-7-(methylthio)[1,3]thiazolo[5,4-d]pyrimidine

The compound produced in Reference Example 1 (2.00g) was dissolved in ethyl acetate (100ml), 3-methoxybenzoyl chloride (2.81g) was added thereto, and the mixture was heated in reflux for 14 hours. The reaction solution was concentrated and dried. To the residue was added diethyl ether to obtain a pale yellow crystal (2.59g). The crystal (2.8g) was suspended with toluene (250ml), Lawesson's reagent (4.23g) was added thereto, and the mixture was heated at 120°C for 2.5 hours. To the reaction solution was added ethyl acetate, and the mixture was washed with a 5% aqueous sodium hydrogencarbonate, dried, and concentrated. The residue was purified with column chromatography (silica gel, developing solvent : ethyl acetate/n-hexane/chloroform = 1/5/5), and crystallized with ethyl acetate/n-hexane=1/2 to obtain the title compound (2.05g).

$^1$H-NMR($CDCl_3$) δ:2.73(3H,s),3.92(3H,s),7.04-7.12(1H,m), 7.42(1H,t,J=8.0Hz),7.62-7.70(2H,m),8.82(1H,s).

**Reference Example 3**

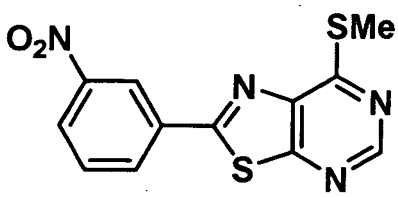

7-(methyl)-2-(3-nitrophenyl)[1,3]thiazolo[5,4-d]pyrimidine

The compound produced in Reference Example 1 (2.00g) was dissolved in N,N-dimethylacetamide (10ml), 3-nitrobenzoyl chloride (2.45g) was added thereto, and the mixture was stirred at room temperature for 14 hours. To the reaction solution was added water (150ml), the precipitated crystal was collected by filtration, washed with water, and dried, and 3.62g of the crystal was obtained. The crystal (3.55g) was suspended with toluene (150ml), Lawesson's reagent (5.12g) was added thereto, and the mixture was heated at 120°C for 3.5 hours. The reaction solution was cooled. The precipitated crystal was collected by filtration, and washed with toluene to obtain the title compound (3.00g).

$^1$H-NMR(DMSO-$d_6$) δ:2.72(3H,s),7.92(1H,t,J=8.0Hz),8.42-8.60(2H,m),8.80-8.90(1H,m),8.97(1H,s).

**Reference Example 4**

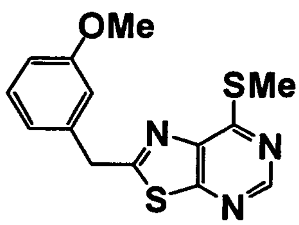

2-(3-methoxybenzyl)-7-(methylthio)[1,3]thiazolo[5,4-d]pyrimidine

The compound produced in Reference Example 1 (5.00g) was dissolved in N,N-dimethylacetamide (15ml), (3-methoxyphenyl)acetyl chloride (5.50g) was added thereto, and the mixture was stirred at room temperature for 8 hours. The reaction solution was poured into water, the precipitated crystal was collected by filtration, dried, and then dissolved in toluene (20ml). Lawesson's reagent (16.2g) was added thereto, and the mixture was heated at 110°C for 5 hours. The reaction solution was washed with a 5% aqueous sodium hydrogencarbonate, dried, and concentrated. The residue was purified with column chromatography (silica gel, developing solvent : ethyl acetate/n-hexane = 1/3), and then recrystallized from ethyl acetate/n-hexane to obtain the title compound (4.94g).

$^1$H-NMR($CDCl_3$) δ:2.71(3H,s),3.81(3H,s),4.43(2H,s),6.85-6.95(3H,m),7.26-7.31(1H,m),8.78(1H,s).

**Reference Example 5**

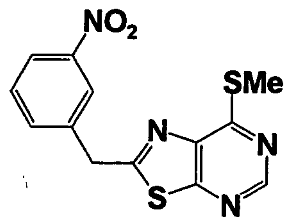

7-(methylthio)-2-(3-nitrobenzyl)[1,3]thiazolo[5,4-d]pyrimidine

In the same manner as shown in Reference Example 2, the compound obtained by a reaction of the compound produced in Reference Example 1 (3.00g) with (3-nitrophenyl)acetyl chloride (3.50g) was reacted with Lawesson's reagent (8.0g) to obtain the title compound (3.60g).

$^1$H-NMR($CDCl_3$) δ:2.73(3H,s),4.59(2H,s),7.57(1H,m),7.72 (1H,d,J=8.3Hz),8.20(1H,d,J=8.3),8.27(1H,s),8.82(1H,s).

**Reference Example 6**

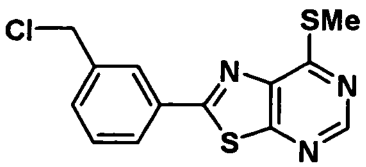

2-[3-(chloromethyl)phenyl]-7-(methylthio)[1,3]thiazolo[5,4-d]pyrimidine

The compound produced in Reference Example 1 (2.82g) was dissolved in N,N-dimethylacetamide (15ml), 3-chloromethylbenzoyl chloride (3.00g) was added thereto, and the mixture was stirred at room temperature for 16 hours. To the reaction solution was added ice water, the precipitated crystal was collected by filtration, washed with water, and dried, and 5.06g of the crystal was obtained. The crystal (5.00g) was suspended with toluene (300ml), Lawesson's reagent (7.14g) was thereto, and the mixture was heated at 120°C for 2.5 hours. To the reaction solution was added ethyl acetate, and the mixture was washed with a 5% aqueous sodium hydrogencarbonate, dried, and concentrated. The residue was purified with column chromatography (silica gel, developing solvent : ethyl acetate/n-hexane/chloroform = 1/5/5), and crystallized with diethyl ether to obtain the title compound (4.22g).

$^1$H-NMR($CDCl_3$) δ:2.74 (3H,s), 4.68 (2H,s), 7.46-7.62 (2H,m), 8.00-8.18 (2H,m), 8.83 (1H,s).

**Reference Example 7**

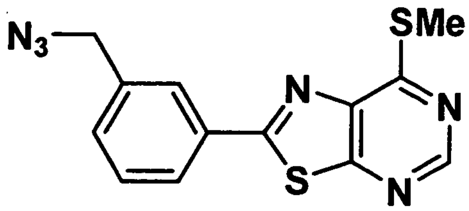

2-[3-(azidomethyl)phenyl]-7-(methylthio)[1,3]thiazolo[5,4-d]pyrimidine

The compound produced in Reference Example 6 (1.50g) and sodium azide (0.50g) were suspended with dimethylsulfoxide (8ml), and the mixture was stirred at 70°C for 17 hours. After cooling, cold water was added thereto, and the precipitated crystal was collected by filtration, and dried to obtain the title compound (1.42g). $^1$H-NMR ($CDCl_3$) δ:2.74(3H,s),4.49(2H,s),7.45-7.60(2H, m),8.00-8.15(2H,m),8.83(1H,s).

**Reference Example 8**

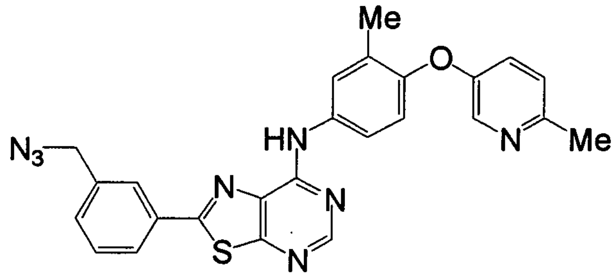

2-[3-(azidomethyl)phenyl]-N-{3-methyl-4-[(6-methylpyridin-3-yl)oxy]phenyl}[1,3]thiazolo[5,4-d]pyrimidin-7-amine

In the same manner as shown in Example 1, the title compound (0.30g) was obtained from the compound produced in Reference Example 7 (0.26g) and 3-methyl-4-[(6-methylpyridin-3-yl)oxy] aniline (0.28g). $^1$H-NMR($CDCl_3$) δ:2.31(3H,s),2.54(3H,s),4.50(2H,s),6.95 (1H,d,J=8.8Hz),7.08-7.14(2H,m),7.49-7.58(2H,m),7.66-7.69 (1H,m),7.74(1H,d,J=2.7Hz),7.98(1H,s),8.01-8.03(1H,m), 8.08(1H,s),8.27(1H,d, J=2.7Hz),8.62(1H,s).

**Reference Example 9**

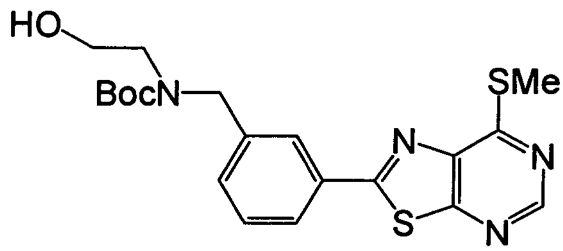

tert-butyl 2-hydroxyethyl{3-[7-(methylthio)[1,3]thiazolo[5,4-d]pyrimidin-2-yl]benzyl}carbamate

The compound produced in Reference Example 6 (2.00g) was dissolved in 1-methyl-2-pyrrolidone (12ml), 2-aminoethanol (0.79g) and sodium iodide (0.92g) were added thereto, and the mixture was stirred for 1 hour. Di-tert-butyl dicarbonate (2.90g) was added thereto, and the mixture was stirred again for 2 hours. A saturated aqueous sodium hydrogencarbonate was added thereto, and the mixture was extracted with ethyl acetate. The extract was dried, and concentrated, and the residue was purified with column chromatography (basic silica gel, developing solvent : ethyl acetate/n-hexane = 1/3) to obtain the title compound (1.50g).

$^1$H-NMR($CDCl_3$) δ:1.52 (9H,brs), 2.73 (3H,s), 2.89 (1H, brs), 3.52 (2H,brs), 3.70-3.85 (2H,brm), 4.58 (2H,brs), 7.35-7.55 (2H,m), 7.95-8.10 (2H, m), 8.82(1H,s).

**Reference Example 10**

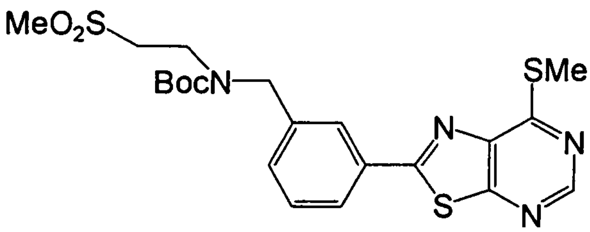

tert-butyl 2-(methylsulfonyl)ethyl{3-[7-(methylthio)[1,3]thiazolo[5,4-d]pyrimidin-2-yl]benzyl}carbamate

In the same manner as shown in Reference Example 9, the compound obtained from the compound produced in Reference Example 6 (1.00g) and 2-(methylsulfonyl)ethylamine (0.80g) was reacted with di-tert-butyl dicarbonate (1.4g) to obtain the title compound (1.21g).

$^1$H-NMR ($CDCl_3$) δ:1.55(9H,brs), 2.72(3H,s), 2.94(3H,brs), 3.20-3.40 (2H,m), 3.70-3.80 (2H,brm), 4.60 (2H,brs), 7.30-7.52 (2H, m), 7.94-8.08 (2H, m), 8.80 (1H,s).

**Reference Example 11**

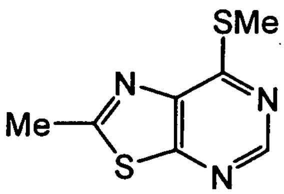

2-methyl-7-(methylthio)[1,3]thiazolo[5,4-d]pyrimidine

In the same manner as shown in Reference Example 2, the compound obtained by a reaction of the compound produced in Reference Example 1 (2.52g) with acetyl chloride (2ml) was reacted with Lawesson's reagent (5.50g) to obtain the title compound (1.54g).

$^1$H-NMR ($CDCl_3$) δ:2.72 (3H,s), 2.90 (3H,s), 8.82 (1H,s).

**Reference Example 12**

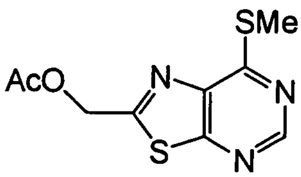

[7-(methylthio)[1,3]thiazolo[5,4-d]pyrimidin-2-yl]methyl acetate

In the same manner as shown in Reference Example 2, the compound obtained by a reaction of the compound produced in Reference Example 1 (8.00g) with 2-chloro-2-oxoethyl acetate (8.00g) was reacted with Lawesson's reagent (20.0g) to obtain the title compound (4.70g).

$^1$H-NMR ($CDCl_3$) δ:2.22 (3H,s), 2.71 (3H,s), 5.50 (2H,s), 8.84 (1H,s).

**Reference Example 13**

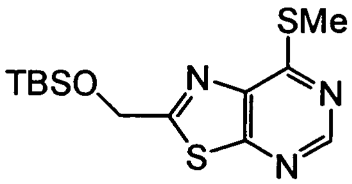

2-({[tert-butyl(dimethyl)silyl]oxy}methyl)-7-(methylthio)[1,3]thiazolo[5,4-d]pyrimidine

The compound produced in Reference Example 12 (5.20g) was dissolved in methanol (60ml), under an ice cooling condition, a 10% aqueous potassium hydroxide solution (12ml) was added thereto, and the mixture was stirred for 10 minutes. The reaction solution was concentrated, a saturated saline solution was added thereto, and then the mixture was extracted with ethyl acetate three times. The extract was dried, and concentrated, the residue was purified with column chromatography (silica gel, developing solvent : ethyl acetate/n-hexane/chloroform = 5/1/5), and a brown powder (1.21g) was obtained. The brown powder was dissolved in N,N-dimethylformamide (30ml), imidazole (0.65g) and tert-butyldimethylchlorosilane (1.50g) were added thereto, and the mixture was stirred at room temperature for 6 hours. Water (200ml) was added thereto, and the mixture was stirred again for 30 minutes. The precipitated crystal was collected by filtration, washed with water, and dried to obtain the title compound (1.60g).

$^1$H-NMR($CDCl_3$) δ:0.17 (6H,s), 0.98 (9H,s), 2.71 (3H,s), 5.08 (2H,s), 8.82 (1H,s).

**Reference Example 14**

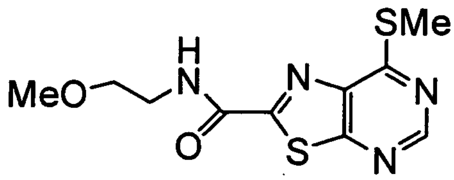

N-(2-methoxyethyl)-7-(methylthio)[1,3]thiazolo[5,4-d]prymidine-2-carboxamide

Ethyl 7-(methylthio)[1,3]thiazolo[5,4-d]pyrimidine-2-carboxylate (1.20g) was dissolved in methanol (10ml), and 2-methoxyethylamine (3ml) was added thereto. The reaction solution was stirred at 60°C for 1 hour, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate/n-hexane = 1/1) to obtain the title compound (0.79g).

$^1$H-NMR($CDCl_3$) δ:2.73(3H,s),3.43(3H,s),3.61(2H,t,J= 4.8Hz), 3.71(2H,dd,J=11.0,4.8Hz),7.68(1H,brs),8.89(1H,s).

**Reference Example 15**

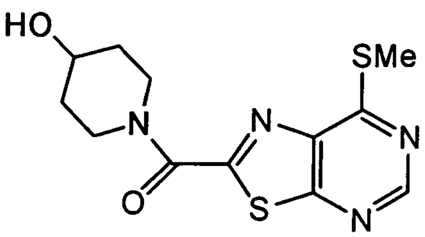

1-{[7-(methylthio)[1,3]thiazolo[5,4-d]pyrimidin-2-yl]carbonyl}piperidin-4-ol

Ethyl 7-(methylthio)[1,3]thiazolo[5,4-d]pyrimidine-2-carboxylate (1.50g) was dissolved in methanol (10ml), and piperidin-4-ol (3.5ml) was added thereto. The reaction solution was stirred at 50°C for 1 hour, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate/n-hexane = 1/1) to obtain the title compound (1.24g).

$^1$H-NMR($CDCl_3$) δ:1.69-1.76(2H,m),2.02-2.08(2H,m), 2.71(3H,s), 3.53-3.59 (1H,m), 4.01-4.11 (2H,m), 4.15-4.21 (1H, m), 4.70-4.76(1H,m), 8.89(1H,s).

**Reference Example 16**

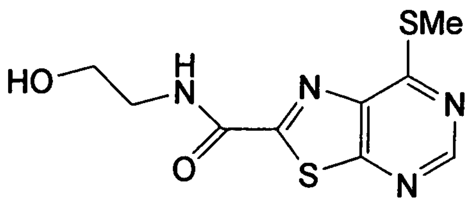

N-(2-hydroxyethyl)-7-(methylthio)[1,3]thiazolo[5,4-d]pyrimidine-2-carboxamide

In the same manner as shown in Reference Example 15, the title compound (0.95g) was obtained from ethyl 7-(methylthio)[1,3]thiazolo[5,4-d]pyrimidine-2-carboxylate (1.50g) and 2-aminoethanol (2ml).

$^1$H-NMR ($CDCl_3$) δ:2.6(1H,brs),2.72(3H,s),3.69-3.73(2H,m),3.89-3.91(2H,brs),7.77(1H,brs),8.89(1H,s).

**Reference Example 17**

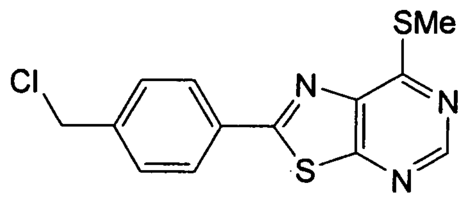

2-[4-(chloromethyl)phenyl]-7-(methylthio)[1,3]thiazolo[5,4-d]pyrimidine

The compound produced in Reference Example 1 (3.00g) was dissolved in N,N-dimethylacetamide (15ml), 4-chloromethylbenzoyl chloride (3.78g) was added thereto, and the mixture was stirred at room temperature for 21 hours. To the reaction solution was added water (200ml). The precipitated crystal was collected by filtration, washed with water, and dried to obtain a crystal (4.83g). The crystal (4.80g) was suspended with toluene (150ml), Lawesson's reagent (6.85g) was added thereto, and the mixture was heated at 120°C for 1.5 hours. To the reaction solution was added ethyl acetate, and the mixture was washed with a 5% aqueous sodium hydrogencarbonate, dried, and concentrated. The residue was purified with column chromatography (silica gel, developing solvent : ethyl acetate/n-hexane/chloroform = 1/5/5), and crystallized with diethyl ether to obtain the title compound (3.42g).

$^1$H-NMR($CDCl_3$) δ:2.73(3H,s),4.65(2H,s),7.53(2H,d,J= 8.0Hz), 7.11(2H,d,J=8.0Hz),8.82(1H,s).

**Reference Example 18**

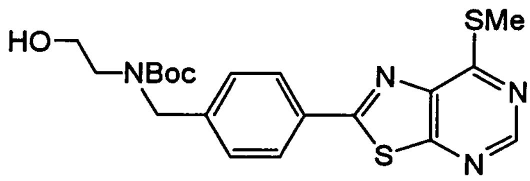

tert-butyl 2-hydroxyethyl{4-[7-(methylthio)[1,3]thiazolo[5,4-d]pyrimidin-2-yl]benzyl}carbamate

The compound produced in Reference Example 17 (0.67g) was dissolved in 1-methyl-2-pyrrolidone (3ml), and sodium iodide (0.33g) and 2-aminoethanol (0.7ml) were added thereto. The reaction solution was stirred at room temperature for 1 hour, a saturated aqueous sodium hydrogencarbonate was added thereto, and the mixture was extracted with ethyl acetate. The extract was dried and concentrated, and the residue was dissolved in tetrahydrofuran (10ml). Di-tert-butyl dicarbonate (0.48g) was added thereto, the mixture was stirred for 3 hours, and the organic layer was washed by adding a saturated aqueous sodium hydrogencarbonate. The organic layer was dried and concentrated. The residue was purified with column chromatography (silica gel, developing solvent : ethyl acetate/n-hexane = 1/1) to obtain the title compound (0.59g).

$^1$H-NMR($CDCl_3$) δ:1.55(9H,brs),2.73(3H,s),3.67(2H,brs), 3.75(2H,brs),4.56(2H,brs),7.38(2H,d,J=8.3Hz),8.08(2H,d, J=8.3 Hz), 8.82(1H,s).

**Reference Example 19**

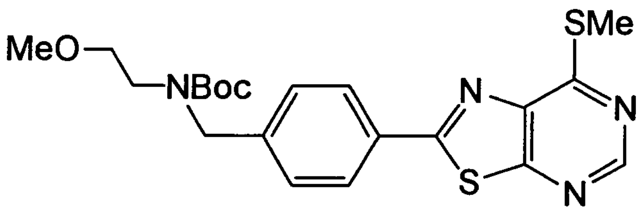

tert-butyl 2-methoxyethyl{4-[7-(methylthio)[1,3]thiazolo[5,4-d]pyrimidin-2-yl]benzyl}carbamate

In the same manner as shown in Reference Example 18, the compound obtained from the compound produced in Reference Example 17 (2.00g) and 2-methoxyethylamine (2ml) was reacted with di-tert-butyl dicarbonate (1.5g) to obtain the title compound (1.31g).

$^1$H-NMR($CDCl_3$) δ:1.57 (9H,s), 2.73 (3H,s), 3.32 (3H,s), 3.34-3.60 (4H,brm), 4.58 (2H,brs), 7.37 (2H,brs), 8.05-8.08 (2H, brm). 8.81 (1H,s).

**Reference Example 20**

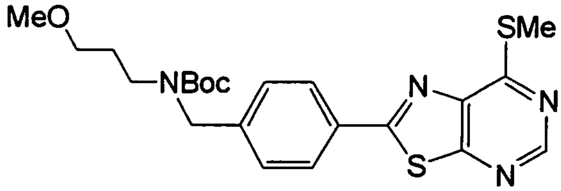

tert-butyl 3-methoxypropyl{4-[7-(methylthio)[1,3]thiazolo[5,4-d]pyrimidin-2-yl]benzyl}carbamate

In the same manner as shown in Reference Example 18, the compound obtained from the compound produced in Reference Example 17 (2.04g) and 3-methoxypropylamine (2ml) was reacted with di-tert-butyl dicarbonate (1.43g) to obtain the title compound (2.21g).

$^1$H-NMR($CDCl_3$) δ:1.55(9H,brs),1.80(2H,brs),2.73 (3H,s), 3.31(3H,s),3.16-3.43(4H,m),4.50(2H,brs),7.37(2H,brs), 8.05-8.08(2H,m),8.81(1H,s).

**Reference Example 21**

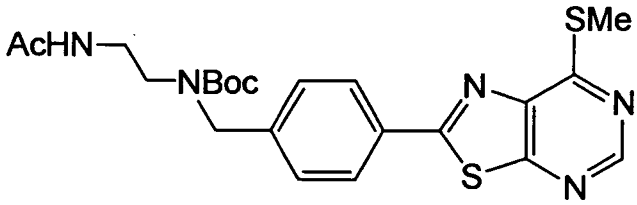

tert-butyl 2-(acetylamino)ethyl{4-[7-(methylthio)[1,3]thiazolo[5,4-d]pyrimidin-2-yl]benzyl}carbamate

In the same manner as shown in Reference Example 18, the compound obtained from the compound produced in Reference Example 17 (2.03g) and N-(2-aminoethyl)acetamide (2ml) was reacted with di-tert-butyl dicarbonate (1.41g) to obtain the title compound (1.34g).

$^1$H-NMR (CDCl$_3$) δ:1.57(9H,brs),1.95(3H,brs),2.73(3H,s), 3.31-3.48(4H,m),4.50(2H,brs),6.31(1H,brs),7.31-7.36(2H, brs), 8.08(2H,d,J=8.4Hz),8.82(1H,s).

**Reference Example 22**

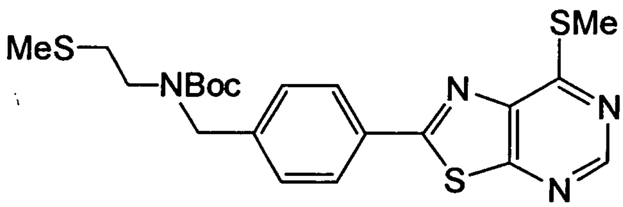

tert-butyl 2-(methylthio)ethyl{4-[7-(methylthio)[1,3]thiazolo[5,4-d]pyrimidin-2-yl]benzyl}carbamate

In the same manner as shown in Reference Example 18, the compound obtained from the compound produced in Reference Example 17 (1.26g) and 2-(methylthio)ethylamine (1.4ml) was reacted with di-tert-butyl dicarbonate (1.00g) to obtain the title compound (1.13g).

$^1$H-NMR (CDCl$_3$) δ:1.56 (9H,brs), 2.11 (3H,s), 2.73 (3H,s), 3.34-3.60 (4H,brm), 4.58 (2H,brs), 7.37 (2H,brs), 8.05-8.08 (2H,brm), 8.81(1H,s).

**Reference Example 23**

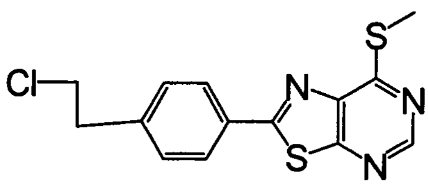

2-[4-(2-chloroethyl)phenyl]-7-(methylthio)[1,3]thiazolo[5,4-d]pyrimidine

In the same manner as shown in Reference Example 2, the compound obtained by a reaction of the compound produced in Reference Example 1 (5.00g) with 4-(2-chloroethyl)benzoyl chloride (6.05g) was further reacted with Lawesson's reagent (15.2g) to obtain the title compound (6.80g).

$^1$H-NMR(CDCl$_3$) δ:2.73(3H,s),3.13-3.17(2H,m),3.75-3.79(2H,m), 7.36-7.39(2H,m),8.05-8.08(2H,m),8.82(1H,s).

**Reference Example 24**

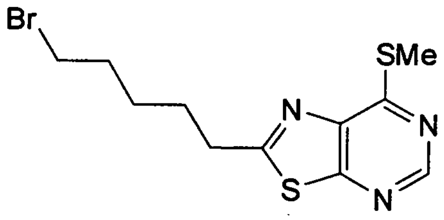

2-(5-bromopentyl)-7-(methylthio)[1,3]thiazolo[5,4-d]pyrimidine

The compound produced in Reference Example 1 (2.00g) was dissolved in N,N-dimethylacetamide (6ml), 5-bromohexanoyl chloride (3.00g) was added thereto, and the mixture was stirred at room temperature for 16 hours. To the reaction solution was added water (100ml), the precipitated crystal was collected by filtration, washed with water, and dried to obtain a crystal (3.83g). The crystal (3.83g) was suspended with toluene (100ml), Lawesson's reagent (4.45g) was added thereto, and the mixture was heated at 120°C for 1.5 hours. The reaction solution was purified with column chromatography (basic silica gel, developing solvent : ethyl acetate/n-hexane = 1/1), and crystallized with n-hexane to obtain the title compound (2.50g).

$^1$H-NMR($CDCl_3$) $\delta$:1.50-1.70(2H,m),1.75-2.08(4H,m),2.71 (3H,s), 3.17(2H,t,J=7.8Hz),3.43(1H,t,J=6.6Hz),3.56(1H,t, J=6.6Hz), 8.80 (1H,s).

**Reference Example 25**

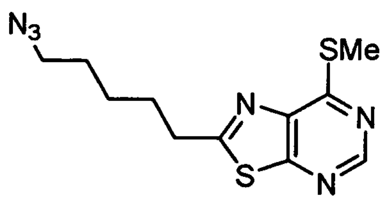

2-(5-azidopentyl)-7-(methylthio)[1,3]thiazolo[5,4-d]pyrimidine

The compound produced in Reference Example 24 (1.00g) and sodium azide (0.30g) were suspended with dimethylsulfoxide (4ml), and the mixture was stirred at 70°C for 16 hours. After cooling, the reaction solution was poured into water, and the mixture was extracted with ethyl acetate. The extract was dried and concentrated, and the residue was purified with column chromatography (silica gel, developing solvent : ethyl acetate/n-hexane = 1/2) to obtain the title compound as a colorless oily matter (0.82g).

$^1$H-NMR($CDCl_3$) $\delta$:1.44-1.78(4H,m),1.82-2.08(2H,m),2.70(3H,s), 3.16(2H,t,J=7.6Hz),3.31(2H,t,J=6.6Hz),8.80(1H,s).

**Reference Example 26**

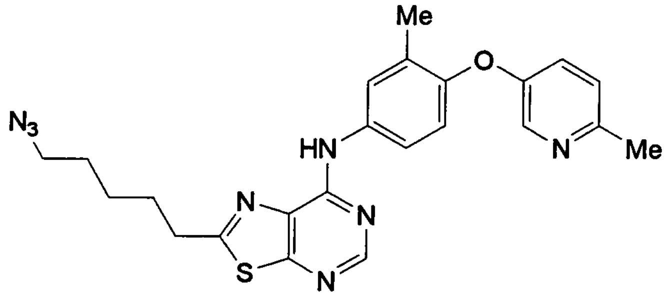

2-(5-azidopentyl)-N-{3-methyl-4-[(6-methylpyridin-3-yl)oxy]phenyl}[1,3]thiazolo[5,4-d]pyrimidin-7-amine

The compound produced in Reference Example 25 (0.81g) was dissolved in ethyl acetate (20ml), m-chloroperbenzoic acid (1.0g) was added thereto, and the mixture was stirred under an ice cooling condition for 30 minutes, and then stirred at room temperature for 30 minutes. The reaction solution was respectively washed with a 5% aqueous sodium thiosulfate solution (5ml), a 5% aqueous sodium hydrogencarbonate (10ml) and a saturated saline solution (10ml), dried and concentrated. The crystal (0.35g) was dissolved in 1-methyl-2-pyrrolidone (3ml), 3-methyl-4-[(6-methylpyridin-3-yl)oxy]aniline (0.30g) was added thereto, and the mixture was stirred at 100°C for 2 hours. The reaction solution was poured into water, and the mixture was extracted with ethyl acetate. The extract was dried and concentrated, and the residue was purified with column chromatography (silica gel, developing solvent : ethyl acetate/n-hexane = 2/1) to obtain the title compound as a pale yellow oily matter (0.15g).

$^1$H-NMR($CDCl_3$) δ:1.45-1.80(4H,m),1.82-2.02(2H,m),2.29(3H,s), 2.53(3H,s),3.13(2H,t,J=7.4Hz),3.32(2H,t,J=6.6Hz), 6.93(1H,d,J =8.8Hz),7.02-7.14(2H,m),7.56-7.72(2H,m), 7.83(1H,s),8.24-8.29(1H,m),8.59(1H,s).

**Reference Example 27**

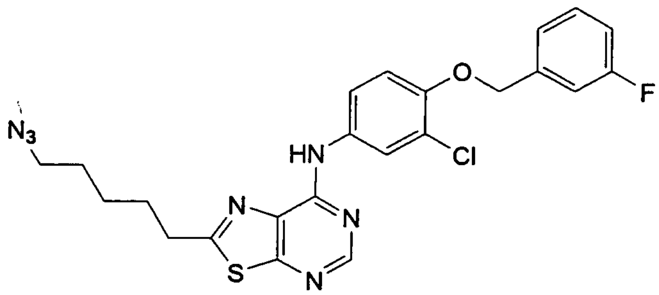

2-(5-azidopentyl)-N-{3-chloro-4-[(3-fluorobenzyl)oxy]phenyl}[1,3]thiazolo[5,4-d]pyrimidin-7-amine

The compound produced in Reference Example 25 (0.81g) was dissolved in ethyl acetate (20ml), m-chloroperbenzoic acid (1.00g) was added thereto, and the mixture was stirred under an ice cooling condition for 30 minutes, and then stirred at room temperature for 30 minutes. The reaction solution was respectively washed with a 5% aqueous sodium thiosulfate solution (5ml), a 5% aqueous sodium hydrogencarbonate (10ml) and a saturated saline solution (10ml), dried and concentrated. The crystal (0.35g) was dissolved in 1-methyl-2-pyrrolidone (3ml), 3-chloro-4-[(3-fluorobenzoyl)oxy]aniline (0.35g) was added thereto, and the mixture was stirred at 120°C for 1.5 hours. The reaction solution was poured into water, and the mixture was extracted with ethyl acetate. The extract was dried and concentrated, and the residue was purified with column chromatography (silica gel, developing solvent : ethyl acetate/n-hexane = 1/1), and then recrystallized from ethyl acetate/n-hexane = 1/4 to obtain the title compound (0.33g).

$^1$H-NMR ($CDCl_3$) δ:1.4-1.80(4H,m),1.82-2.05(2H,m),3.12 (2H,t,J=7.6Hz),3.32(2H,t,J=6.6Hz),5.16(2H,s),6.95-7.12(1H, m), 6.97(1H,d,J=8.8Hz),7.15-7.50(3H,m),7.59(1H,dd,J=2.6,8.8Hz), 7.75(1H,d,brs),7.95(1H,d,J=2.6Hz),8.58(1H,s).

**Reference Example 28**

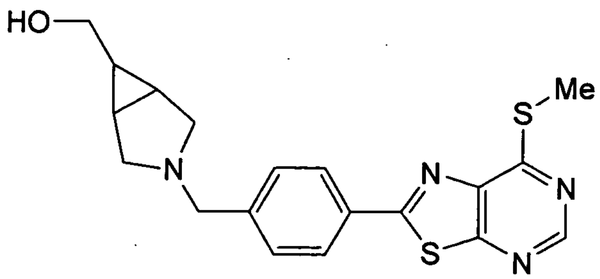

(3-{4-[7-(methylthio)[1,3]thiazolo[5,4-d]pyrimidin-2-yl]benzyl}-3-azabicyclo[3.1.0]hex-6-yl)methanol

The compound produced in Reference Example 17 (0.40g) was dissolved in 1-methyl-2-pyrrolidone (5ml), potassium iodide (0.26g) and 3-azabicyclo[3.1.0]hex-6-ylmethanol (0.16g) were added thereto, and the mixture was stirred at room temperature for 1 night. The reaction solution was poured into water, and basified by adding a 1N sodium hydroxide, and the mixture was extracted with ethyl acetate. The extract was washed with water, dried and concentrated, and the residue was purified with column chromatography (basic silica gel, developing solvent : ethyl acetate). The obtained crystal was washed with diethyl ether to obtain the title compound (0.28g).

$^1$H-NMR ($CDCl_3$) δ:1.20-1.38 (3H,m), 1.60 (1H,m), 2.39 (2H,d,J=8.8Hz),2.73(3H,s),3.02(2H,d,J=8.8Hz),3.40-3.50 (2H, m),3.66(2H,s),7.42(2H,d,J=8.4Hz),8.03(2H,d,J=8.4Hz),8.81 (1H,s).

**Reference Example 29**

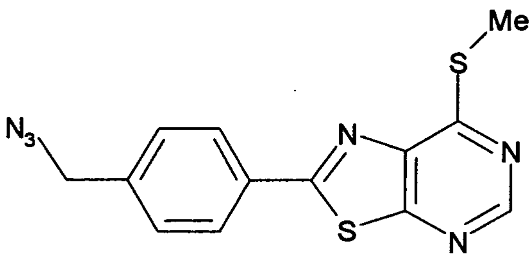

2-[4-(azidomethyl)phenyl]-7-(methylthio)[1,3]thiazolo[5,4-d]pyrimidine

In the same manner as shown in Reference Example 7, the compound produced in Reference Example 17 (5.43g) was reacted with sodium azide (1.80g) to obtain the title compound (4.80g).

$^1$H-NMR(DMSO-$d_6$) δ:2.69(3H,s),4.61(2H,s),7.59-7.62(2H,m), 8.12-8.15(2H,m),8.92(1H,s).

**Reference Example 30**

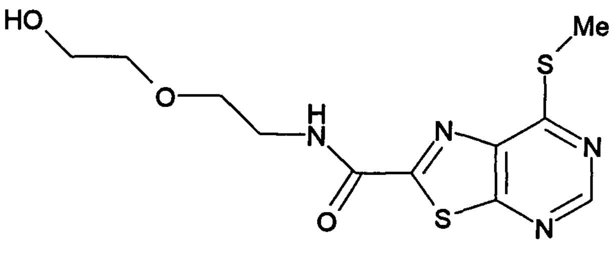

N-[2-(2-hydroxyethoxy)ethyl]-7-(methylthio)[1,3]thiazolo[5,4-d]pyrimidine-2-carboxamide

In the same manner as shown in Reference Example 14, the title compound (0.96g) was obtained from ethyl 7-(methylthio)[1,3]thiazolo[5,4-d]pyrimidine-2-carboxylate (1.00g), and 2-(2-aminoethoxy)ethanol (1.80g). $^1$H-NMR

($CDCl_3$) δ:2.73 (3H,s), 3.66-3.68 (2H,m), 3.71-3.75 (4H,m), 3.81-3.83 (2H,m), 7.84 (1H,brs), 8.89 (1H,s).

**Reference Example 31**

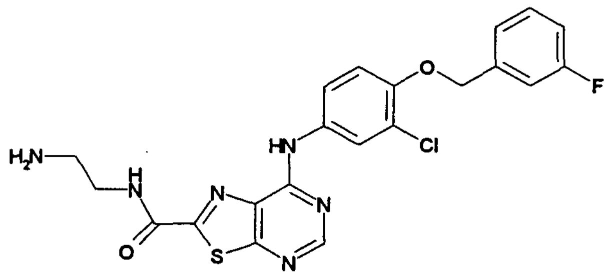

N-(2-aminoethyl)-7-({3-chloro-4-[(3-fluorobenzyl)oxy]phenyl}amino)[1,3]thiazolo[5,4-d]pyrimidine-2-carboxamide

In the same manner as shown in Reference Example 14 and Example 27, the title compound (0.58g) was obtained from ethyl 7-(methylthio)[1,3]thiazolo[5,4-d]pyrimidine-2-carboxylate (0.70g), tert-butyl (2-aminoethyl)carbamate (0.70g) and 3-chloro-4-[(3-fluorobenzyl)oxy]aniline (1.45g). $^1$H-NMR (DMSO-$d_6$) δ:2.75-2.79 (2H,m), 3.37-3.41 (2H,m), 5.25 (2H,s), 7.16-7.49 (6H,m), 7.73-7.76 (1H,m), 8.08(1H,s), 8.31-8.65 (1H,m), 8.63 (1H,s).

**Reference Example 32**

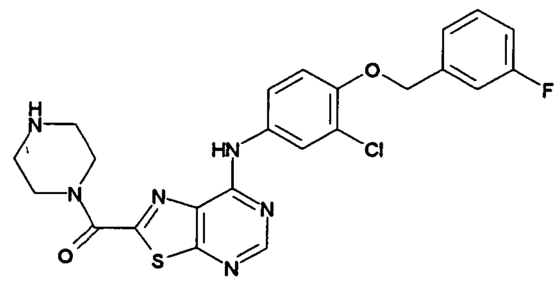

N-{3-chloro-4-[(3-fluorobenzyl)oxy]phenyl}-2-(piperazin-1-ylcarbonyl)[1,3]thiazolo[5,4-d]pyrimidin-7-amine

In the same manner as shown in Reference Example 31, the title compound (3.62g) was obtained from ethyl 7-(methylthio)[1,3]thiazolo[5,4-d]pyrimidine-2-carboxylate (3.00g), tert-butyl piperazine-1-carboxylate (2.98g) and 3-chloro-4-[(3-fluorobenzyl)oxy]aniline (5.60g).

$^1$H-NMR(DMSO-$d_6$) δ:2.82(4H,brs),3.64(2H,brs),4.25(2H,brs), 5.27(2H,s),7.15-7.49(5H,m),7.67-7.72(1H,m),7.97-7.98 (1H,m), 8.57(1H,s),10.11(1H,s).

**Reference Example 33**

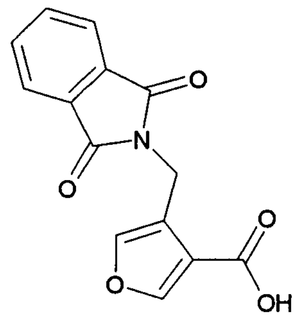

4-[(1,3-dioxo-1,3-dihydro-2H-isoindol-2-yl)methyl]-3-furoic acid

Methyl 4-(hydroxymethyl)-3-furoate (10.0g) was dissolved in dichloromethane (200ml), triethylamine (20ml) and methanesulfonyl chloride (8.5ml) were added under an ice cooling condition thereto, and the mixture was stirred for 1 hour. The reaction solution was poured into water, and the organic layer was dried and concentrated. The residue was dissolved in N,N-dimethylformamide (100ml), potassium 1,3-dioxo-1,3-dihydroisoindol-2-id (12.5g) was added thereto, and the mixture was stirred for 1 hour. A saturated aqueous sodium hydrogencarbonate was added thereto, and the mixture was extracted with ethyl acetate. The extract was dried and concentrated, and the residue was purified with column chromatography (basic silica gel, developing solvent : ethyl acetate/n-hexane = 3/1). The obtained compound was dissolved in N,N-dimethylformamide (45ml), lithium chloride (25g) was added thereto, and the mixture was stirred at 150°C for 8 hours. A saturated aqueous sodium hydrogencarbonate was added under an ice cooling condition thereto, and the mixture was extracted with ethyl acetate. The extract was dried and concentrated, and then the residue was purified with column chromatography (silica gel, developing solvent : methanol/ethyl acetate = 1/9) to obtain the title compound (5.83g).

$^1$H-NMR (DMSO-$d_6$) δ:4.80 (2H,s), 7.74 (1H,s), 7.84-7.92 (4H,m), 8.37 (1H,s), 13.11 (1H,brs).

**Reference Example 34**

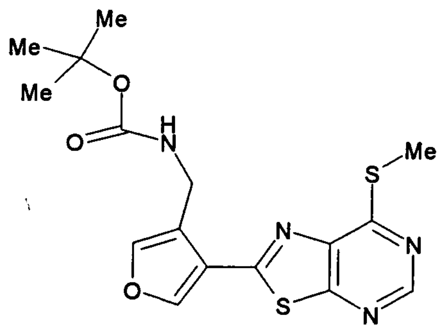

tert-butyl ({4-[7-(methylthio)[1,3]thiazolo[5,4-d]pyrimidin-2-yl]-3-furyl}methyl)carbamate

The compound produced in Reference Example 33 (2.30g) was dissolved in dichloromethane (50ml), oxalyl chloride (1.80ml) was added thereto, and the mixture was stirred for 8 hours. The reaction solution was concentrated, the obtained residue was dissolved in N,N-dimethylacetamide (30ml), the compound produced in Reference Example 1 (1.50g) was added thereto, and then the mixture was stirred at room temperature for 14 hours. A saturated aqueous sodium hydrogencarbonate was added under an ice cooling condition thereto, and the mixture was extracted with dichloromethane. The extract was dried and concentrated, and then the residue was purified with column chromatography (silica gel, developing solvent : ethyl acetate). The obtained compound was dissolved in ethanol (40ml), hydrazine monohydrate (5ml) was added thereto, the mixture was stirred for 5 hours, and then hydrazine monohydrate (5ml) was further added thereto, and the mixture was stirred for 14 hours. A saturated aqueous sodium hydrogencarbonate was added thereto, and the mixture was extracted with ethyl acetate. To the extract was added di-tert-butyl dicarbonate (2ml), and the mixture was dried and concentrated. The residue was purified with column chromatography (silica gel, developing solvent : ethyl acetate/n-hexane = 1/2). The obtained compound was dissolved in toluene (10ml), Lawesson's reagent (0.41g) was added thereto, and the mixture was heated under stirring at 90°C for 2.5 hours. To the reaction

solution was added ethyl acetate, and the mixture was washed with a 5% aqueous sodium hydrogencarbonate, dried and concentrated. The residue was purified with column chromatography (basic silica gel, developing solvent : ethyl acetate/n-hexane = 1/5) to obtain the title compound (0.35g).

$^1$H-NMR($CDCl_3$) δ:1.45(9H,s),2.73(3H,s),4.43(2H,d, J=6.2Hz), 6.22(1H,brs),7.58(1H,brs),8.03(1H,s), 8.82(1H,s).

**Reference Example 35**

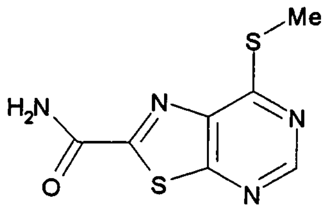

7-(methylthio)[1,3]thiazolo[5,4-d]pyrimidine-2-carboxamide

Ethyl 7-(methylthio)[1,3]thiazolo[5,4-d]pyrimidine-2-carboxylate (0.50g) was dissolved in a saturated ammonia-methanol solution (10ml), and the mixture was stirred for 50 hours. The solvent was distilled off, and tetrahydrofuran (4ml) was added thereto. Hexane (2ml) was further added thereto, and the precipitated crystal was collected by filtration to obtain the title compound (0.47g).

$^1$H-NMR(DMSO-$d_6$) δ:2.70 (3H,s), 8.29 (1H,brs), 8.50 (1H,brs), 9.00 (1H,s).

**Reference Example 36**

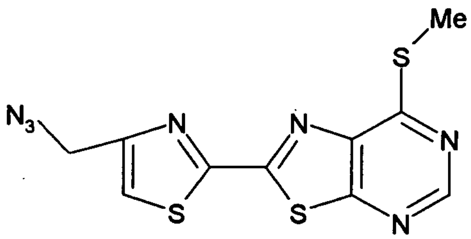

2-[4-(azidomethyl)-1,3-thiazol-2-yl]-7-(methylthio)[1,3]thiazolo[5,4-d]pyrimidine

The compound produced in Reference Example 35 (0.10g) was suspended with toluene (15ml), Lawesson's reagent (0.22g) was added thereto, and the mixture was stirred at 110°C for 1 hour. The reaction solution was purified partially with column chromatography (basic silica gel, developing solvent : ethyl acetate), the obtained compound was dissolved in a mixed solvent of toluene (5ml) and ethanol (5ml), 1,3-dichloroacetone (1.00g) was added thereto, and then the mixture was stirred at 120°C for 2 hours. After cooling to 0°C, the precipitated crystal was collected by filtration, washed with diethyl ether, dried, and dissolved in acetonitrile (7ml). Sodium iodide (0.005g) and sodium azide (0.075g) were added thereto, and the mixture was stirred at 50°C for 3 hours. Under an ice cooling condition, to the reaction solution was added ethyl acetate. The mixture was washed with a 5% aqueous sodium hydrogencarbonate, dried and concentrated. The residue was purified with column chromatography (silica gel, developing solvent : methanol/ethyl acetate = 5/95) to obtain the title compound (0.031g).

$^1$H-NMR ($CDCl_3$) δ:2.71 (3H,s), 4.69 (2H,s), 8.14 (1H,s), 8.98 (1H,s).

**Reference Example 37**

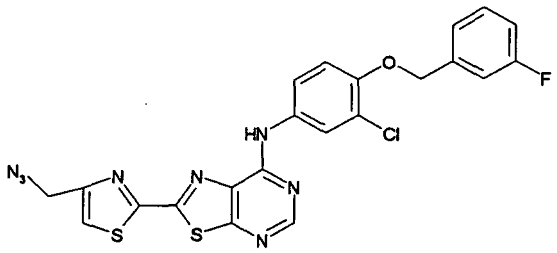

2-[4-(azidomethyl)-1,3-thiazol-2-yl]-N-{3-chloro-4-[(3-fluorobenzyl)oxy]phenyl}[1,3]thiazolo[5,4-d]pyrimidin-7-amine

In the same manner as shown in Example 1, the title compound (0.17g) was obtained from the compound produced in Reference Example 36 (0.31g) and 3-chloro-4-[(3-fluorobenzyl)oxy]aniline (0.28g).

$^1$H-NMR(DMSO-$d_6$) δ:4.68(2H,s),5.26(2H,s),7.17-7.80 (6H,m), 8.06(2H,s),8.57(1H,s),10.21(1H,s).

**Reference Example 38**

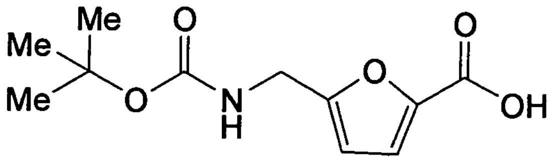

5-{[(tert-butoxycarbonyl)amino]methyl}-2-furoic acid

A mixture of ethyl 5-[(1,3-dioxo-1,3-dihydro-2H-isoindol-2-yl)methyl]-2-furoate (6.93g), hydrazine monohydrate (1.68ml) and ethanol (150ml) was heated under reflux for 3 hours. The reaction solution was concentrated under reduced pressure, ethyl acetate (150ml) was added thereto, and the mixture was stirred. The insoluble matter was filtered off, and the filtrate was concentrated under reduced pressure. The residue was dissolved in tetrahydrofuran (80ml), triethylamine (3.88ml) and di-tert-butyl dicarbonate (5.86ml) were added thereto, and the mixture was stirred at room temperature for 15 hours. After concentrating under reduced pressure, the residue was dissolved in ethanol (100ml), and a 1N aqueous sodium hydroxide solution (40ml) was added thereto. After stirring at room temperature for 5.5 hours, the reaction solution was concentrated under reduced pressure. The residue was suspended with water, and the mixture was adjusted to pH 1 with 1N hydrochloric acid. After extracting with ethyl acetate, the organic layers were combined and dried over anhydrous magnesium sulfate. After concentrating under reduced pressure, the residue was washed with diethyl ether, and dried at 80°C under reduced pressure to obtain the title compound (3.80g).

$^1$H-NMR ($CDCl_3$) δ:1.45 (9H,s), 4.38 (2H,d,J=5.4Hz), 5.04 (1H,br), 6.40 (1H,d,J=3.3Hz), 7.25 (1H,d,J=3.3Hz).

**Reference Example 39**

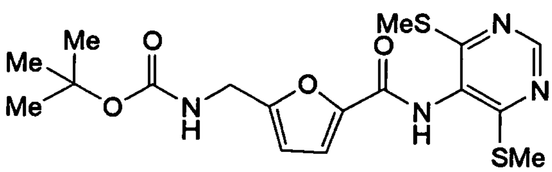

tert-butyl {[5-({[4,6-bis(methylthio)pyrimidin-5-yl]amino}carbonyl)-2-furyl]methyl}carbamate

To a mixture of the compound produced in Reference Example 38 (1.90g), oxalyl chloride (1.0ml) and dichloromethane (40ml) was added dropwise N,N-dimethylformamide (0.15ml) under an ice cooling condition. After stirring for 1 hour under an ice cooling condition, the reaction solution was concentrated under reduced pressure. The residue was dissolved in N,N-dimethylacetamide (3ml), and a solution (12ml) of 4,6-bis(methylthio)pyrimidin-5-amine (1.30g) in

N,N-dimethylacetamide was added at room temperature thereto. After stirring at room temperature for 22 hours, ethyl acetate was added thereto, and the mixture was washed with an aqueous sodium hydrogencarbonate, water, and a saturated saline solution by turns, dried over anhydrous magnesium sulfate, and then concentrated under reduced pressure. The residue was purified with column chromatography (silica gel, developing solvent : n-hexane/ethyl acetate = 4/1→3/2) to obtain the title compound (1.48g).
$^1$H-NMR ($CDCl_3$) δ:1.47 (9H,s), 2.55 (6H,s), 4.40 (2H,d, J=5.7Hz), 4.96 (1H,brs), 6.41 (1H,d,H=3.3Hz), 7.22 (1H,d, J=3.3Hz), 7.49 (1H,brs), 8.71 (1H,s).

**Reference Example 40**

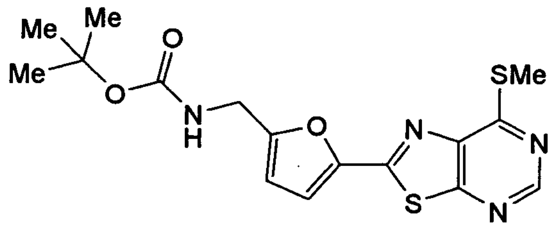

tert-butyl ({5-[7-(methylthio)[1,3]thiazolo[5,4-d]pyrimidin-2-yl]-2-furyl}methyl)carbamate

A mixture of the compound produced in Reference Example 39 (1.48g), Lawesson's reagent (1.98g) and toluene (100ml) was heated under reflux for 8 hours. Lawesson's reagent (1.98g) was further added thereto, and the mixture was heated under reflux for 15.5 hours, cooled to room temperature, and then purified with column chromatography (basic silica gel, developing solvent : n-hexane/ethyl acetate = 3/1→1/1). The obtained solid was washed with diisopropyl ether, and dried at 60°C under reduced pressure to obtain the title compound (0.413g).
$^1$H-NMR ($CDCl_3$) δ:1.48 (9H,s), 2.72 (3H,s), 4.42 (2H,d, J=6.0Hz), 4.79(1H,brd),6.46(1H,d,J=3.3Hz),7.25(1H,d,J=3.3Hz), 8.79(1H,s).

**Reference Example 41**

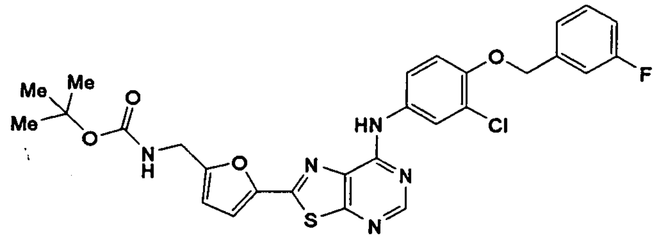

tert-butyl ({5-[7-({3-chloro-4-[(3-fluorobenzyl)oxy]phenyl}amino)[1,3]thiazolo[5,4-d]pyrimidin-2-yl]-2-furyl}methyl)carbamate

To a mixture of the compound produced in Reference Example 40 (0.413g), dichloromethane (30ml) and N, N-dimethylacetamide (20ml) was added m-chloroperbenzoic acid (0.309g) under an ice cooling condition, and the mixture was stirred under an ice cooling condition for 45 minutes, and at room temperature for 30 minutes respectively. m-Chloroperbenzoic acid (0.34g) was further added thereto, and the mixture was stirred under at room temperature for 45 minutes. An aqueous sodium hydrogencarbonate was added thereto under an ice cooling condition, and the mixture was extracted with dichloromethane. The organic layers were combined, washed with an aqueous sodium hydrogencarbonate, and dried over anhydrous magnesium sulfate. After concentrating under reduced pressure, the residue was dissolved in 1-methyl-2-pyrrolidinone (2.5ml), 3-chloro-4-[(3-fluorobenzyl)oxy]aniline (0.305g) was added thereto. The mixture was stirred at 120°C for 75 minutes, an aqueous sodium hydrogencarbonate was added under an ice cooling condition thereto, and the mixture was extracted with ethyl acetate. The extract was washed with a saturated saline solution, dried over anhydrous magnesium sulfate, and concentrated under reduced pressure. The residue was purified

with column chromatography (silica gel, developing solvent : n-hexane/ethyl acetate = 4/1→1/1) to obtain the title compound (0.429g).
$^1$H-NMR($CDCl_3$) δ:1.41(9H,s),4.24(2H,d,J=5.7Hz), 5.24 (2H, s), 6.52(2H,d,J=3.3Hz),7.17(1H,m),7.23(1H,d,J=9.0Hz), 7.25-7.34(2H,m),7.42-7.53(2H,m),7.76(1H,dd,J=9.0,2.7Hz), 8.07(1H,d,J=2.7Hz),8.50(1H,s).

**Reference Example 42**

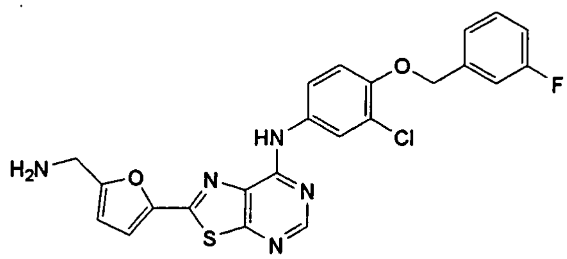

2-[5-(aminomethyl)-2-furyl]-N-{3-chloro-4-[(3-fluorobenzyl)oxy]phenyl}[1,3]thiazolo[5,4-d]pyrimdin-7-amine
To the compound produced in Reference Example 41 (0.39g) was added trifluoroacetic acid (5ml) under an ice cooling condition, and the mixture was stirred at room temperature for 20 minutes. Toluene (50ml) was added thereto, and the mixture was concentrated under reduced pressure. The residue was purified with column chromatography (basic silica gel, developing solvent : n-hexane/ethyl acetate = 1/1→ethyl acetate) to obtain the title compound (0.235g).
$^1$H-NMR (DMSO-$d_6$) δ: 3.80(2H,s),5.24(2H,s),6.56 (1H,d,J=3.3Hz),7.14-7.33(5H,m),7.44(1H,m),7.77(1H,dd, J=9.0,2.4Hz),8.08(1H,d,J=2.4Hz),8.49(1H,s),10.06(2H,brs).

**Example 1**

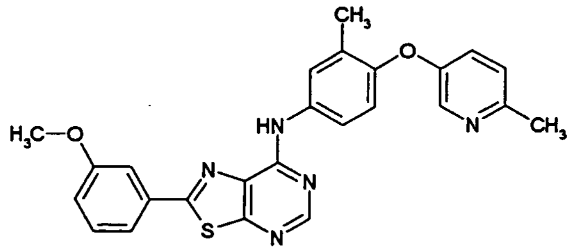

2-(3-methoxyphenyl)-N-{3-methyl-4-[(6-methylpyridin-3-yl)oxy]phenyl}[1,3]thiazolo[5,4-d]pyrimidin-7-amine
The compound produced in Reference Example 2 (0.50g) was dissolved in dichloromethane (5ml), m-chloroperbenzoic acid (0.43g) was added thereto under an ice cooling condition, and the mixture was stirred at room temperature for 10 minutes. The organic layer was washed by adding a saturated aqueous sodium hydrogencarbonate under an ice cooling condition, dried, and concentrated. The residue was dissolved in 1-methyl-2-pyrrolidone (5ml), 3-methyl-4-[(6-methylpyridin-3-yl)oxy]aniline (0.71g) was added thereto, and the mixture was stirred at 120°C for 3 hours. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/4) to obtain the title compound (0.62g).
$^1$H-NMR ($CDCl_3$) δ:2.31(3H,s),2.54(3H,s),3.94(3H,s), 6.95(1H,d,J=8.4Hz),7.08-7.14(3H,m),7.44(1H,t,J=8.4Hz), 7.62-7.74(4H,m),7.99(1H,s),8.27(1H,brs),8.61(1H,s).

Example 2

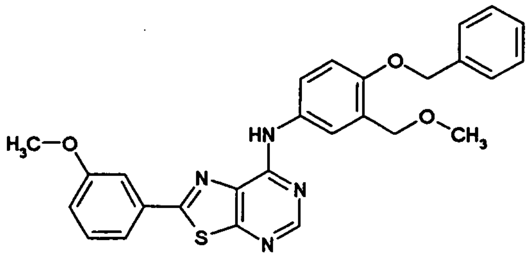

N-[4-(benzyloxy)-3-(methoxymethyl)phenyl]-2-(3-methoxyphenyl)[1,3]thiazolo[5,4-d]pyrimidin-7-amine

The compound produced in Reference Example 2 (0.098g) was dissolved in dichloromethane (5ml), m-chloroperbenzoic acid (0.084g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and 4-(benzyloxy)-3-(methoxymethyl)aniline (0.16g) was added thereto. The reaction solution was stirred at 120°C for 3 hours. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/3) to obtain the title compound (0.11g).

$^1$H-NMR(DMSO-$d_6$) δ:3.37(3H,s),3.89(3H,s),4.50 (2H,s), 5.16(2H,s),7.10-7.20(2H,m),7.32-7.54(6H,m),7.69-7.77 (4H, m),8.48(1H,brs),9.89(1H,s).

## Example 3

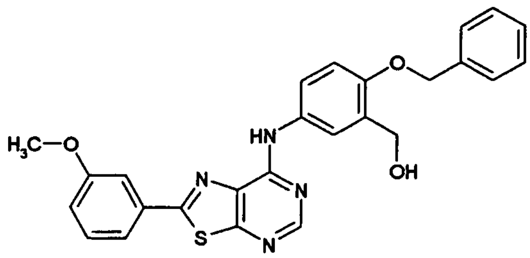

(2-(benzyloxy)-5-{[2-(3-methoxyphenyl)[1,3]thiazolo[5,4-d]pyrimidin-7-yl]amino}phenyl)methanol

The compound produced in Reference Example 2 (0.19g) was dissolved in dichloromethane (5ml), m-chloroperbenzoic acid (0.16g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and [5-amino-2-(benzyloxy)phenyl]methanol (0.28g) was added thereto. The reaction solution was stirred at 120°C for 3 hours. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/3) to obtain the title compound (0.20g).

$^1$H-NMR($CDCl_3$) δ:2.30-2.70 (1H,brs), 3.94 (3H,s), 4.80 (2H,d,J = 6.3Hz), 5.16 (2H,s), 7.00 - 7.03 (2H,m), 7.35 - 7.45 (6H,m), 7.61 - 7.63 (2H,m), 7.69 - 7.70 (1H,m), 7.76 - 7.79 (1H.,m), 7.92 (1H,brs), 8.57 (1H,s).

## Example 4

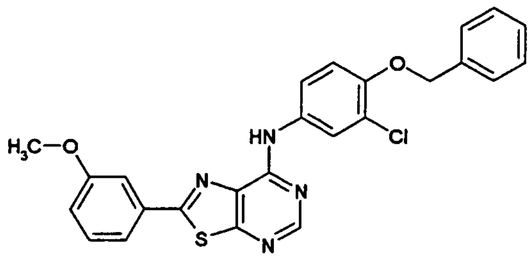

N-[4-(benzyloxy)-3-chlorophenyl]-2-(3-methoxyphenyl)[1,3]thiazolo[5,4-d]pyrimidin-7-amine

The compound produced in Reference Example 2 (0.10g) was dissolved in dichloromethane (5ml), m-chloroperbenzoic acid (0.085g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and 4-(benzyloxy)-3-chloroaniline (0.15g) was added thereto. The reaction solution was stirred at 120°C for 3 hours. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/6) to obtain the title compound (0.11g).

$^1$H-NMR(DMSO-$d_6$) δ:3.89(3H,s),5.23(2H,s),7.19-7.21 (1H,m), 7.28(1H,m),7.35-7.55(6H,m),7.69-7.81(3H,m),8.08(1H,s), 8.54(1H,s),9.99(1H,s) .

**Example 5**

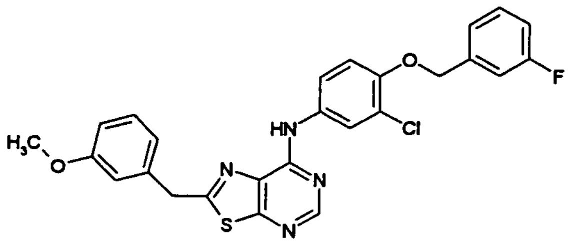

N-{3-chloro-4-[(3-fluorobenzyl)oxy]phenyl}-2-(3-methoxybenzyl)[1,3]thiazolo[5,4-d]pyrimidin-7-amine

The compound produced in Reference Example 4 (0.070g) was dissolved in dichloromethane (5ml), m-chloroperbenzoic acid (0.060g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (3ml), and 3-chloro-4-[(3-fluorobenzyl)oxy]aniline (0.12g) was added thereto. The reaction solution was stirred at 120°C for 3 hours. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/5) to obtain the title compound (0.040g) .

$^1$H-NMR($CDCl_3$) δ:3.82(3H,s),4.37(2H,s),5.16(2H,s),6.78-7.02(5H,m),7.20-7.38(4H,m),7.58-7.61(1H,m),7.82(1H,s), 7.95 (1H,d,J=1.4Hz),8.56(1H,s).

**Example 6**

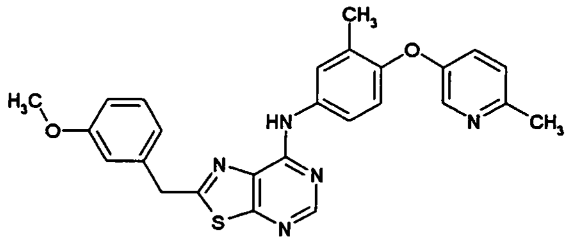

2-(3-methoxybenzyl)-N-{3-methyl-4-[(6-methylpyridin-3-yl)oxy]phenyl}[1,3]thiazolo[5,4-d]pyrimidin-7-amine

In the same manner as shown in Example 5, the title compound (0.16g) was obtained from the compound produced in Reference Example 4 (0.30g) and 3-methyl-4-[(6-methylpyridin-3-yl)oxy]aniline (0.29g).

$^1$H-NMR($CDCl_3$) δ:2.28(3H,s),2.53(3H,s),3.82(3H,s), 4.38(2H,s),6.85-6.95(4H,m),7.09-7.10(2H,m),7.30(1H,t, J=7.6Hz),7.61-7.63(1H,m),7.69(1H,s),7.90(1H,s),8.25(1H,d, J=2.7Hz), 8.56 (1H,s).

**Example 7**

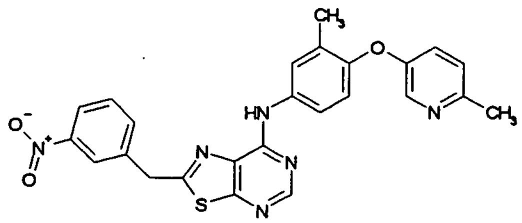

N-{3-methyl-4-[(6-methylpyridin-3-yl)oxy]phenyl}-2-(3-nitrobenzyl)[1,3]thiazolo[5,4-d]pyrimidin-7-amine

In the same manner as shown in Example 5, the title compound (0.21g) was obtained from the compound produced in Reference Example 5 (0.50g) and 3-methyl-4-[(6-methylpyridin-3-yl)oxy]aniline (0.64g).

$^1$H-NMR($CDCl_3$) δ:2.28(3H,s),2.52(3H,s),4.52(2H,s),6.92 (1H,d,J=8.8Hz),7.08-7.12(2H,m),7.55-7.63(2H,m),7.67-7.80 (2H,m),7.85(1H,s),8.17-8.21(1H,m),8.23-8.27(2H,m),8.58(1H,s).

**Example 8**

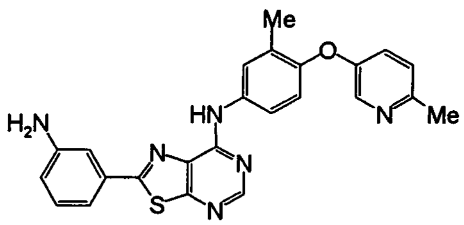

2-(3-aminophenyl)-N-{3-methyl-4-[(6-methylpyridin-3-yl)oxy]phenyl}[1,3]thiazolo[5,4-d]pyrimidin-7-amine

The compound produced in Reference Example 3 (0.21g) was dissolved in dichloromethane (10ml), m-chloroperbenzoic acid (0.31g) was added thereto under an ice cooling condition, and the mixture was stirred at room temperature for 10 minutes. The organic layer was washed by adding a saturated aqueous sodium hydrogencarbonate and a saturated saline solution respectively under an ice cooling condition, dried and concentrated. The residue was dissolved in 1-methyl-2-pyrrolidone (5ml), 3-methyl-4-[(6-methylpyridin-3-yl)oxy]aniline (0.43g) was added thereto, and the mixture was stirred at 120°C for 3 hours. A saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 1/4), and a brown powder (0.26g) was obtained. The brown powder was dissolved in a mixed solvent (20ml) of tetrahydrofuran/methanol=1/1, sodium borohydride (0.097g) and nickel bromide (0.010g) were added under an ice cooling condition thereto, and the mixture was stirred for 1 hour. A saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/3) to obtain the title compound (0.13g).
$^1$H-NMR($CDCl_3$) δ:2.31(3H,s),2.54(3H,s),3.90(2H,brs), 6.84-6.86(1H,m),6.95(1H,d,J=8.6Hz),7.08-7.12(2H,m), 7.29-7.32(1H,m),7.41-7.43(2H,m),7.65-7.67(1H,m),7.74(1H,d, J=2.7Hz),7.97(1H,s),8.27(1H,d,J=2.7Hz),8.61(1H,s).

**Example 9**

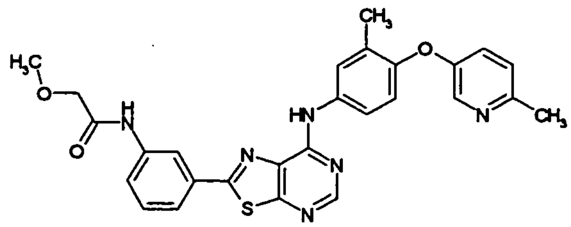

2-methoxy-N-{3-[7-({3-methyl-4-[(6-methylpyridin-3-yl)oxy]phenyl}amino)[1,3]thiazolo[5,4-d]pyrimidin-2-yl]phenyl} acetamide

The compound produced in Example 8 (0.25g) was dissolved in tetrahydrofuran (10ml), and triethylamine (0.5ml) was added thereto. Under an ice cooling condition, methoxyacetyl chloride (0.3ml) was added thereto, and the mixture was stirred for 10 minutes. To the reaction solution was added a 5% aqueous sodium hydrogencarbonate, and the mixture was extracted with ethyl acetate. The extract was dried and concentrated, and then the residue was purified with column chromatography (silica gel, developing solvent : ethyl acetate/n-hexane = 1/5) to obtain the title compound (0.21g).
$^1$H-NMR($CDCl_3$) δ:2.30(3H,s),2.53(3H,s),3.56(3H,s),4.08 (2H,s),6.94(1H,d,J=8.8Hz),7.10-7.12(2H,m),7.47-7.51(1H, m), 7.67-7.74(3H,m),7.79(1H,d,J=7.7Hz),8.05(1H,s),8.27(1H,s), 8.40-8.44(2H,m),8.61(1H,s).

**Example 10**

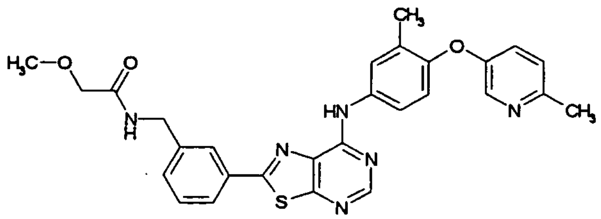

2-methoxy-N-{3-[7-({3-methyl-4-[(6-methylpyridin-3-yl)oxy]phenyl}amino)[1,3]thiazolo[5,4-d]pyrimidin-2-yl]benzyl} acetamide

The compound produced in Reference Example 8 (0.30g) was dissolved in a mixed solvent of ethyl acetate/ methanol=15/1, 10% Palladium/activated carbon (1.60g) was added thereto, and the mixture was stirred under a hydrogen atmosphere for 4 hours. The reaction solution was filtered and the filtrate was concentrated. The residue was dissolved in tetrahydrofuran (10ml), and triethylamine (0.1ml) was added thereto. Under an ice cooling condition, methoxyacetyl chloride (0.7ml) was added thereto, and the mixture was stirred for 10 minutes. To the reaction mixture was added a 5% aqueous sodium hydrogencarbonate, and the mixture was extracted with ethyl acetate. The extract was dried and concentrated, and then the residue was purified with column chromatography (silica gel, developing solvent : ethyl acetate/n-hexane = 1/2~methanol/ethyl acetate=2/98) to obtain the title compound (0.17g).
$^1$H-NMR ($CDCl_3$) δ:2.27(3H,s), 2.49(3H,s), 3.40(3H,s), 3.96 (2H,s), 4.57(2H,d,J=4.1Hz), 6.91(2H,d,J=5.9Hz), 7.04-7.10 (2H,m), 7.43-7.49(2H,m), 7.63-7.71(2H,m), 7.91-7.93(1H,m), 7.98 (2H, d, J=5.9Hz), 8.23 (1H,brs), 8.61 (1H, s).

**Example 11**

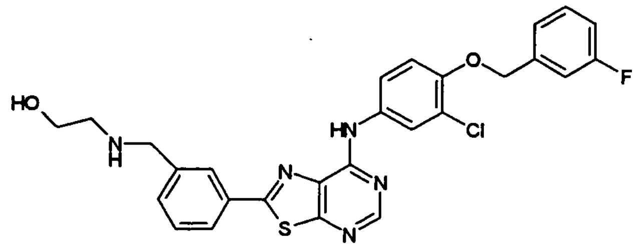

2-((3-[7-({3-chloro-4-[(3-fluorobenzyl)oxy]phenyl}amino)[1,3]thiazolo[5,4-d]pyrimidin-2-yl]benzyl}amino)ethanol

The compound produced in Reference Example 9 (0.30g) was dissolved in dichloromethane (5ml), m-chloroperbenzoic acid (0.18g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy]aniline (0.37g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 1/5), and a pale yellow powder (0.16g) was obtained. The compound (0.12g) was dissolved in trifluoroacetic acid (5ml), and the mixture was stirred at room temperature for 10 minutes. The solvent was distilled off, and the residue was dissolved in triethylamine (lml) and then purified with column chromatography (basic silica gel, developing solvent : ethyl acetate) to obtain the title compound (0.50g).

$^1$H-NMR($CDCl_3$) δ:2.89(2H,t,J=5.2Hz),3.74(2H,t,J=5.2Hz), 3.94(2H,s),5.16(2H,s),6.98(1H,d,J=8.8Hz),7.04(1H,dd, J=8.8,2.
4Hz),7.22(2H,d,J=10.8Hz),7.34-7.39(1H,m),7.45-7.51(2H,m), 7.63-7.66(1H,m),7.90-7.92(1H,m),7.97-7.99(2H,m),8.07 (1H,s), 8.55(1H,s).

**Example 12**

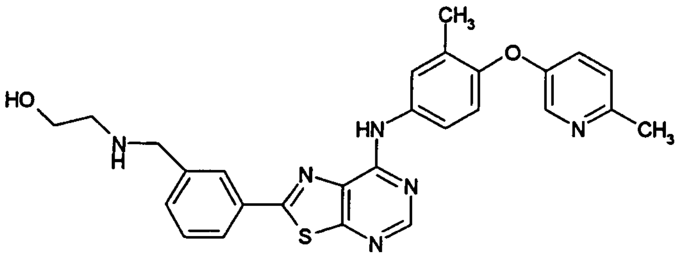

2-({3-[7-({3-methyl-4-[(6-methylpyridin-3-yl)oxy]phenyl}amino)[1,3]thiazolo[5,4-d]pyrimidin-2-yl]benzyl}amino)ethanol

The compound produced in Reference Example 9 (0.30g) was dissolved in dichloromethane (5ml), m-chloroperbenzoic acid (0.18g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.30g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 1/5), and a pale yellow powder (0.12g) was obtained. The compound (0.10g) was dissolved in trifluoroacetic acid (5ml), and the mixture was stirred at room temperature for 10 minutes. The solvent was distilled off, and the residue was dissolved in triethylamine (1ml) and then purified with

column chromatography (basic silica gel, developing solvent : ethyl acetate) to obtain the title compound (0.87g).
$^1$H-NMR($CDCl_3$) δ:2.29(3H,s),2.55(3H,s),3.07-3.10(2H,m),3.82-3.84(2H,m),3.77-3.90(1H,brs),4.12(2H,s),6.92(1H,d, J=8.8Hz),7.10(1H,d,J=8.4Hz),7.15(1H,dd,J=8.4,2.8Hz),7.41-7.47(2H,m),7.70(1H,dd,J=8.8,2.8Hz),7.78(1H,brs),7.83 (1H,d,J= 7.2Hz),8.18(1H,s),8.26(1H,brs),8.48(1H,s),8.56(1H,s).

**Example 13**

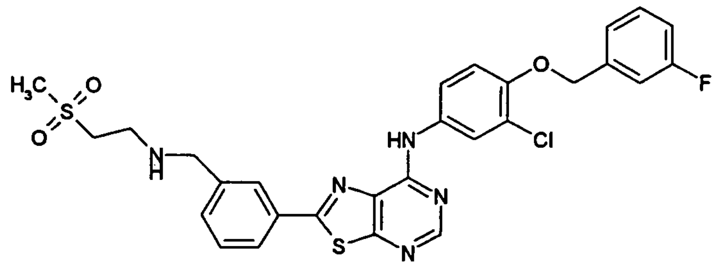

N-{3-chloro-4-[(3-fluorobenzyl)oxy]phenyl}-2-[3-({[2-(methylsulfonyl)ethyl]amino}methyl)phenyl][1,3]thiazolo[5,4-d]pyrimidin-7-amine

The compound produced in Reference Example 10 (0.35g) was dissolved in dichloromethane (5ml), m-chloroperbenzoic acid (0.23g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy]aniline (0.33g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 1/5), and a pale yellow powder (0.27g) was obtained. The compound was dissolved in trifluoroacetic acid (5ml), and the mixture was stirred at room temperature for 10 minutes. The solvent was distilled off, and the residue was dissolved in triethylamine (1ml) and then purified with column chromatography (basic silica gel, developing solvent : ethyl acetate) to obtain the title compound (0.24g).
$^1$H-NMR ($CDCl_3$) δ:3.03 (3H,s), 3.25 (3H,s), 3.96 (2H,s), 5.17 (2H,s),6.99-7.03(2H,m),7.22-7.24(2H,m),7.34-7.38(1H, m),7.48-7.49(2H,m),7.66-7.69(1H,m),7.91-7.94(1H,m),8.00-8.01(2H,m), 8.10(1H,s),8.60(1H,s).

**Example 14**

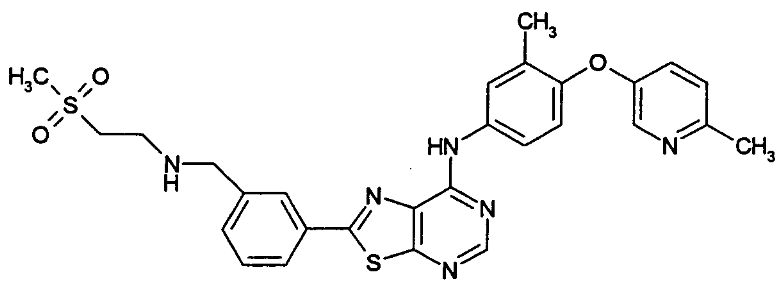

N-{3-methyl-4-[(6-methylpyridin-3-yl)oxy]phenyl}-2-[3-({[2-(methylsulfonyl)ethyl]amino}methyl)phenyl][1,3]thiazolo[5,4-d]pyrimidin-7-amine

The compound produced in Reference Example 10 (0.40g) was dissolved in dichloromethane (5ml), m-chloroperbenzoic acid (0.30g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.43g) was added thereto. The reaction solution was stirred at 120°C for

3 hours, a saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 1/5), and a pale yellow powder was obtained. The compound was dissolved in trifluoroacetic acid (5ml), and the mixture was stirred at room temperature for 10 minutes. The solvent was distilled off, and the residue was dissolved in triethylamine (1ml) and then purified with column chromatography (basic silica gel, developing solvent : ethyl acetate) to obtain the title compound (0.11g).
$^1$H-NMR($CDCl_3$) δ:2.33(3H,s),2.57(3H,s),3.06(3H,s),3.28-3.31(4H,m),4.01(2H,s),6.97(1H,d,J=8.5Hz),7.12-7.19(2H, m), 7.51-7.55(2H,m),7.71-7.74(1H,m),7.79(1H,d,J=2.7Hz),7.96-7.98(1H,m),8.13(2H,d,J=8.5Hz),8.30(1H,d,J=2.7Hz), 8.64(1H,s).

**Example 15**

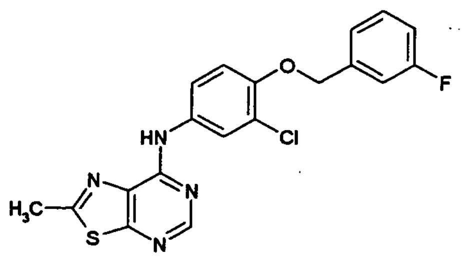

N-{3-chloro-4-[(3-fluorobenzyl)oxy]phenyl}-2-methyl[1,3]thiazolo[5,4-d]pyrimidin-7-amine

The compound produced in Reference Example 11 (0.30g) was dissolved in dichloromethane (5ml), m-chloroperbenzoic acid (0.28g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and 3-chloro-4-[(3-fluorobenzyl)oxy]aniline (0.66g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate/n-hexane = 1/3) to obtain the title compound (0.15g).
$^1$H-NMR ($CDCl_3$) δ:2.83 (3H,s), 5.16 (2H, s), 6.96-7.05 (2H,m), 7.20-7.25(2H,m),7.33-7.39(1H,m),7.58(1H,dd, J=7.4,2.7Hz), 7.75(1H,brs), 7.94(1H,d,J=2.7Hz), 8.54(1H,s).

**Example 16**

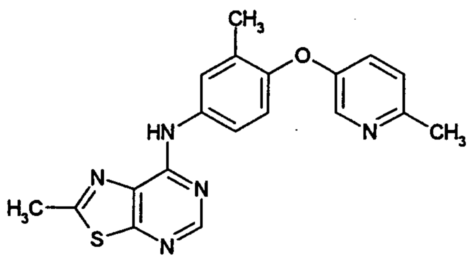

2-methyl-N-{3-methyl-4-[(6-methylpyridin-3-yl)oxy]phenyl}[1,3]thiazolo[5,4-d]pyrimidin-7-amine

The compound produced in Reference Example 11 (0.40g) was dissolved in dichloromethane (6ml), m-chloroperbenzoic acid (0.39g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and 3-chloro-4-[(6-methylpyridin-3-yl)oxy]aniline (0.75g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, and the residue was purified with

column chromatography (silica gel, developing solvent : ethyl acetate/n-hexane = 1/3) to obtain the title compound (0.061g).
$^1$H-NMR ($CDCl_3$) δ:2.28 (3H,s), 2.52 (3H,s), 2.83 (3H,s), 6.91-6.93 (1H,m), 7.07-7.09 (2H,brm), 7.59-7.62 (1H,m), 7.69 (1H,brs), 7.84 (1H,brs), 8.25 (1H,brs), 8.57 (1H,s).

**Example 17**

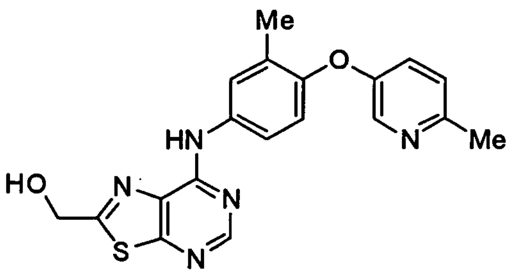

[7-({3-methyl-4-[(6-methylpyridin-3-yl)oxy]phenyl}amino)[1,3]thiazolo[5,4-d]pyrimidin-2-yl]methanol

In the same manner as shown in Example 1, the residue obtained by a reaction of the compound produced in Reference Example 13 (1.60g) with m-chloroperbenzoic acid (1.71g) was reacted with 3-methyl-4-[(6-methylpyridin-3-yl)oxy]aniline (2.42g) to obtain the title compound (1.73g).
$^1$H-NMR($CDCl_3$) δ:2.28(3H,s),2.53(3H,s),3.14(1H,brs), 5.07(2H,brs),7.02-7.14(2H,m),7.59-7.62(1H,m),7.68(1H,brs), 7.84 (1H,brs),8.24(1H,brs),8.61 (1H, s).

**Example 18**

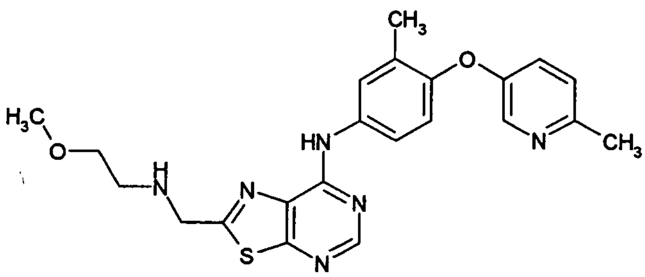

2-{[(2-methoxyethyl)amino]methyl}-N-{3-methyl-4-[(6-methylpyridin-3-yl)oxy]phenyl}[1,3]thiazolo[5,4-d]pyrimidin-7-amine

The compound produced in Example 17 (0.20g) was dissolved in dichloromethane (10ml), triethylamine (0.5ml) and p-toluenesulfonyl chloride (0.12g) were added thereto under an ice cooling condition, and the mixture was stirred for 4 hours. The organic layer was washed by adding a saturated aqueous sodium hydrogencarbonate under an ice cooling condition, dried and concentrated. The residue was purified with column chromatography (silica gel, developing solvent : ethyl acetate/n-hexane = 1/3), and a brown powder (0.26g) was obtained. The compound (0.10g) was dissolved in 2-methoxyethylamine (2ml), and the mixture was stirred for 1 hour. A saturated aqueous sodium hydrogencarbonate was added thereto, and the mixture was extracted with ethyl acetate. The extract was dried and concentrated, and the residue was purified with column chromatography (silica gel, developing solvent : ethyl acetate/n-hexane = 1/1) to obtain the title compound (0.12g).
$^1$H-NMR($CDCl_3$) δ:2.29 (3H, s), 2.53 (3H, s), 2.94 (2H, t, J=5.0Hz), 3.39(3H,s),3.56(2H,t,J=5.0Hz),4.23(2H,s),6.92(1H, d,J=8.8Hz), 7.06-7.12(2H,m),7.60-7.63(1H,m),7.70(1H,brd),7.83 (1H,brs),8.25(1H,brs),8.59(1H,s).

**Example 19**

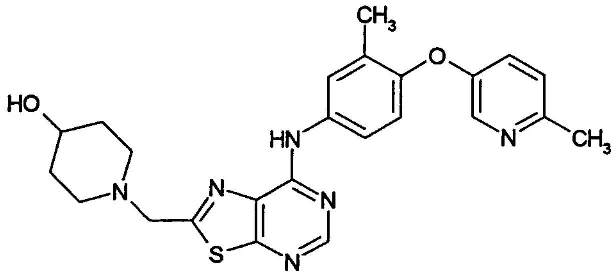

1-{[7-({3-methyl-4-[(6-methylpyridin-3-yl)oxy]phenyl}amino)[1,3]thiazolo[5,4-d]pyrimidin-2-yl]methyl}piperidin-4-ol

The compound produced in Example 17 (0.20g) was dissolved in dichloromethane (10ml), triethylamine (0.5ml) and p-toluenesulfonyl chloride (0.12g) were added thereto under an ice cooling condition, and the mixture was stirred for 4 hours. The organic layer was washed by adding a saturated aqueous sodium hydrogencarbonate under an ice cooling condition, dried and concentrated. The residue was purified with column chromatography (silica gel, developing solvent : ethyl acetate/n-hexane = 1/3), and a brown powder (0.26g) was obtained. The compound (0.10g) was dissolved in toluene (5ml), piperidin-4-ol (0.30g) was added thereto, and the mixture was stirred for 1 hour. The solvent was distilled off, and the residue was purified with column chromatography (basic silica gel, developing solvent : ethyl acetate/n-hexane = 1/1) to obtain the title compound (0.10g). $^1$H-NMR ($CDCl_3$) δ:1.86-1.99(4H,m),2.28(3H,s),2.54(3H,s), 2.87-3.15 (3H,m),3.67-3.85(2H,brm),3.91(2H,s),6.91-6.94(1H,m),7.06-7.13(2H,m),7.58-7.64(1H,m),7.70(1H,brs), 7.87(1H,brs), 8.25(1H,brs),8.61(1H,s).

**Example 20**

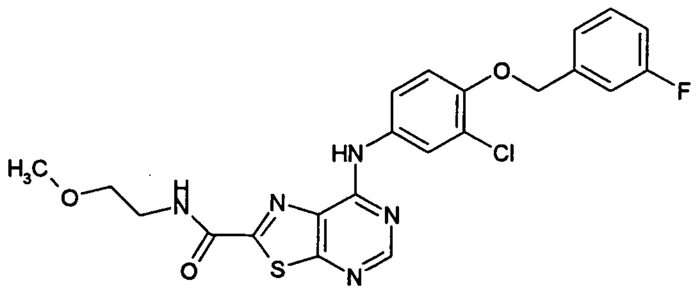

7-({3-chloro-4-[(3-fluorobenzyl)oxy]phenyl}amino)-N-(2-methoxyethyl)[1,3]thiazolo[5,4-d]pyrimidine-2-carboxamide

The compound produced in Reference Example 14 (0.50g) was dissolved in dichloromethane (9ml), m-chloroperbenzoic acid (0.52g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (10ml), and 3-chloro-4-[(3-fluorobenzyl) oxy]aniline (0.60g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate/n-hexane = 1/1) to obtain the title compound (0.68g).

$^1$H-NMR ($CDCl_3$) δ:3.45(3H,s),3.62-3.65(2H,m),3.71-3.75(2H,m),5.18(2H,s),7.00-7.06(2H,m),7.21-7.24(2H,m), 7.35-7.40(1H,m),7.58(1H,brs),7.63-7.66(1H,m),7.81(1H,s),7.96 (1H,s),8.66(1H,s).

**Example 21**

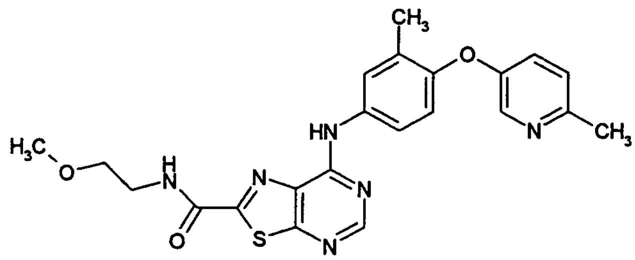

N-(2-methoxyethyl)-7-({3-methyl-4-[(6-methylpyridin-3-yl)oxy]phenyl}amino)[1,3]thiazolo[5,4-d]pyrimidine-2-carboxamide

The compound produced in Reference Example 14 (0.70g) was dissolved in dichloromethane (20ml), m-chloroperbenzoic acid (0.68g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (10ml), and 3-methyl-4-[(6-methylpyridin-3-yl)oxy]aniline (1.10g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate/n-hexane = 1/1) to obtain the title compound (0.46g).
$^1$H-NMR($CDCl_3$) δ:2.32(3H,s),2.54(3H,s),3.46(3H,s),3.64 (2H,t,J=5.1Hz),3.74(2H,dd,J=9.4,5.1Hz),6.94(1H,d, J=8.5Hz), 7.09-7.16(2H,m),7.61-7.66(2H,m),7.73(1H,brs),7.91(1H,brs), 8.26(1H,brs),8.67(1H,s).

**Example 22**

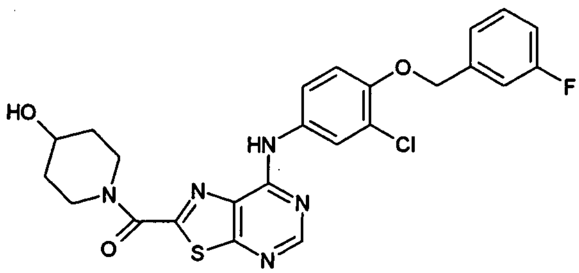

1-{[7-({3-chloro-4-[(3-fluorobenzyl)oxy]phenyl}amino)[1,3]thiazolo[5,4-d]pyrimidin-2-yl]carbonyl}piperidin-4-ol

The compound produced in Reference Example 15 (0.40g) was dissolved in dichloromethane (10ml), m-chloroperbenzoic acid (0.35g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (6ml), and 3-chloro-4-[(3-fluorobenzyl)oxy] aniline (0.48g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/1) to obtain the title compound (0.060g).
$^1$H-NMR($CDCl_3$) δ:1.67-1.75(2H,m),2.00-2.06(2H,brm), 3.51-3.62(1H,brm),3.88-4.00(1H,brm),4.05-4.23(2H,brm), 4.51-4.62(1H,brm),5.17(2H,s),6.98-7.05(2H,m),7.20-7.25(2H,m), 7.34-7.39(1H,m),7.57-7.60(1H,m),7.71(1H,s),7.88 (1H,brs), 8.64(1H,s).

**Example 23**

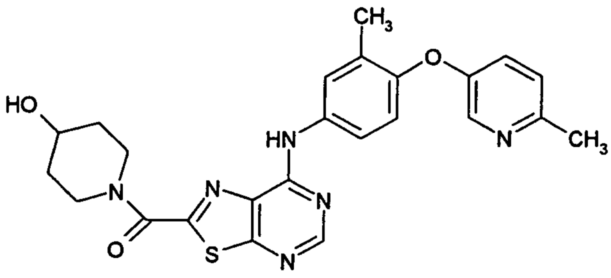

1-{[7-({3-methyl-4-[(6-methylpyridin-3-yl)oxy]phenyl}amino)[1,3]thiazolo[5,4-d]pyrimidin-2-yl] carbonyl}piperidin-4-ol

The compound produced in Reference Example 15 (0.45g) was dissolved in dichloromethane (22ml), m-chloroperbenzoic acid (0.73g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.41g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/1) to obtain the title compound (0.082g).

$^1$H-NMR($CDCl_3$) δ:1.64-1.75(2H,m),2.05-2.17(2H,m),2.31 (3H,s),2.54(3H,s),3.54-3.64(1H,brm),3.91-4.03(1H,brm), 4.07-4.24(2H,brm),4.53-4.65(1H,brm),6.93(1H,d,J=8.8Hz),7.11-7.16(2H,m),7.57-7.59(1H,m),7.65(1H,s),7.75(1H,s), 8.24 (1H,s),8.66(1H,s).

**Example 24**

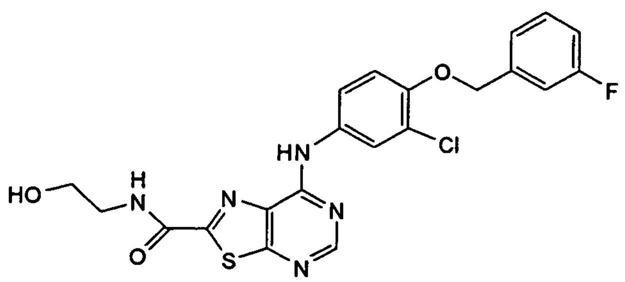

7-({3-chloro-4-[(3-fluorobenzyl)oxy]phenyl}amino)-N-(2-hydroxyethyl)[1,3]thiazolo[5,4-d]pyrimidine-2-carboxamide

The compound produced in Reference Example 16 (0.51g) was dissolved in dichloromethane (9ml), m-chloroperbenzoic acid (0.52g) was added thereto under an ice cooling condition, and the mixture was stirred for 10 minutes. The mixture was washed by adding a saturated aqueous sodium hydrogencarbonate under an ice cooling condition, dried and concentrated. The residue was dissolved in 1-methyl-2-pyrrolidone (10ml), and 3-chloro-4-[(3-fluorobenzyl) oxy]aniline (0.60g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and then the residue was purified with column chromatography (silica gel, developing solvent : ethyl acetate/n-hexane = 1/8) to obtain the title compound (0.12g).

$^1$H-NMR (DMSO-$d_6$) δ:3.44-3.49 (2H,m), 3.57-3.62 (2H,m), 4.97 (1H,brs), 5.26 (2H, s), 7.16-7.21 (1H,m), 7.26-7.50 (4H, m), 7.74-7.77 (1H,bm), 8.08 (1H,brs), 8.37-8.40 (1H,m), 8.62 (1H, s), 10.20 (1H,s).

**Example 25**

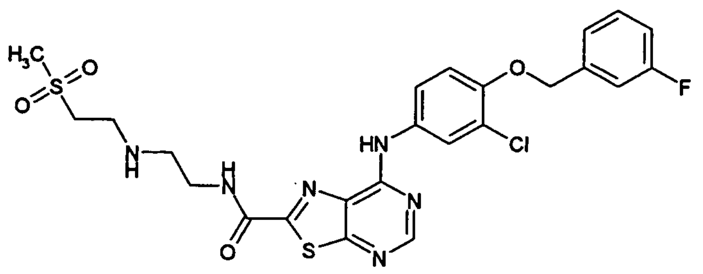

7-({3-chloro-4-[(3-fluorobenzyl)oxy]phenyl}amino)-N-(2-{[2-(methylsulfonyl)ethyl]amino}ethyl)[1,3]thiazolo[5,4-d]pyrimidine-2-carboxamide

The compound produced in Example 24 (0.050g) was dissolved in dichloromethane (10ml), triethylamine (0.1ml) and methanesulfonyl chloride (0.014g) were added under an ice cooling condition thereto, and the mixture was stirred for 2 hours. The organic layer was washed by adding a saturated aqueous sodium hydrogencarbonate under an ice cooling condition, dried and concentrated. The residue was dissolved in acetonitrile (5ml), and 2-(methylsulfonyl)ethylamine (1ml) and sodium iodide (0.007g) were added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated, and then the residue was purified with column chromatography (basic silica gel, developing solvent : ethyl acetate) to obtain the title compound (0.017g).

$^1$H-NMR ($CDCl_3$) δ:2.98-3.05 (2H,m), 3.05 (3H,s), 3.27-3.35 (4H,m), 3.62-3.66 (2H,m), 5.18 (2H, s), 7.00-7.22 (2H,m), 7.23-7.26 (2H,m), 7.35-7.39 (1H,m), 7.79-7.81 (1H,m), 7.99 (1H,brs), 8.10 (1H,brs), 8.65(1H,s).

## Example 26

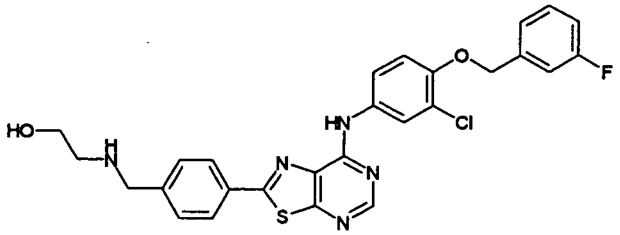

2-({4-[7-({3-chloro-4-[(3-fluorobenzyl)oxy]phenyl}amino)[1,3]thiazolo[5,4-d]pyrimidin-2-yl]benzyl}amino)ethanol

The compound produced in Reference Example 18 (0.50g) was dissolved in dichloromethane (10ml), m-chloroperbenzoic acid (0.20g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy]aniline (0.56g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4). The obtained compound was dissolved in trifluoroacetic acid (5ml), and then the mixture was stirred at room temperature for 10 minutes. The solvent was distilled off, and the residue was recrystallized from ethyl acetate/triethylamine to obtain the title compound (0.24g).

$^1$H-NMR($CDCl_3$) δ:2.30(1H,brs),2.60(2H,brm),3.49(2H,brm), 3.81(2H,s),4.50(1H,brs),5.26(2H,s),7.16-7.21(1H,m), 7.26-7.35(3H,m),7.45-7.50(1H,m),7.56(2H,d,J=8.3Hz),7.81 (1H,dd,J=9.0,2.7Hz),8.10-8.14(3H,m),8.54(1H,s),9.98(1H, s).

## Example 27

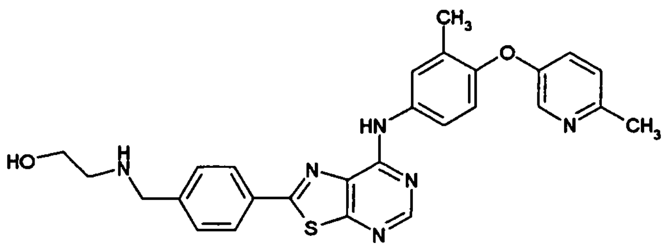

2-({4-[7-({3-methyl-4- [(6-methylpyridin-3-yl)oxy]phenyl}amino)[1,3]thiazolo[5,4-d]pyrimidin-2-yl]benzyl}amino)ethanol

The compound produced in Reference Example 18 (0.42g) was dissolved in dichloromethane (10ml), m-chloroperbenzoic acid (0.25g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.49g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was dissolved in triethylamine (1ml), and purified with column chromatography (basic silica gel, developing solvent:ethyl acetate) to obtain the title compound (0.27g).

$^1$H-NMR ($CDCl_3$) δ:2.31 (3H,s), 2.54 (3H,s), 2.87 (2H,t, J=5.2Hz), 3.72 (2H,t,J=5.2Hz), 3.93 (2H,brs), 6.94-6.96 (1H,m), 7.09-7.12 (2H,m), 7.50(2H,d,J=8.4Hz), 7.66-7.68 (1H,m), 7.74 (1H,brs), 7.96 (1H,brs), 8.04 (2H,d,J=8.4Hz), 8.27 (1H, brs), 8.61(1 H, s).

**Example 28**

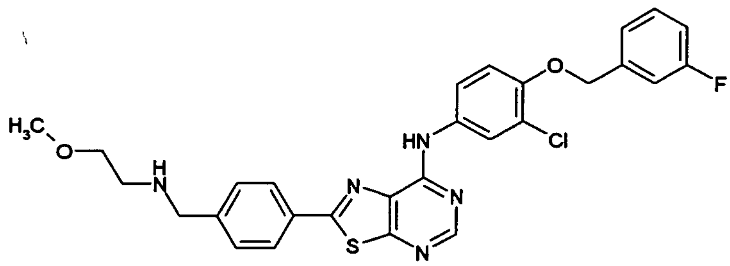

N-{3-chloro-4-[(3-fluorobenzyl)oxy]phenyl}-2-(4-{[(2-methoxyethyl)amino]methyl}phenyl)[1,3]thiazolo[5,4-d]pyrimidin-7-amine

The compound produced in Reference Example 19 (0.50g) was dissolved in dichloromethane (10ml), m-chloroperbenzoic acid (0.20g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy] aniline (0.56g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was recrystallized from ethyl acetate/triethylamine to obtain the title compound (0.40g).

$^1$H-NMR(DMSO-$d_6$) δ:3.07-3.10(2H,m),3.32(3H,s),3.62 (2H,t,J=3.2Hz),4.28(2H,s),5.26(2H,s),7.16-7.21(1H,m), 7.27-7.35(3H,m),7.45-7.51(1H,m),7.74(2H,d,J=5.7Hz),7.80-7.83(1H,m),8.11(1H,brs),8.23(2H,d,J=5.7Hz),8.56(1H,s), 10.03( 1H,s).

**Example 29**

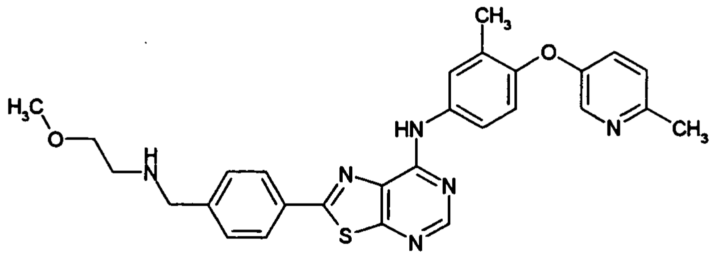

2-(4-{[(2-methoxyethyl)amino]methyl}phenyl)-N-{3-methyl-4-[(6-methylpyridin-3-yl)oxy]phenyl}[1,3]thiazolo[5,4-d]pyrimidin-7-amine

The compound produced in Reference Example 19 (0.50g) was dissolved in dichloromethane (10ml), m-chloroperbenzoic acid (0.20g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.48g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was dissolved in triethylamine (1ml), and purified with column chromatography (basic silica gel, developing solvent : ethyl acetate) to obtain the title compound (0.39g).

$^1$H-NMR(DMSO-$d_6$) δ:2.22(3H,s),2.45(3H,s),3.12-3.15(2H,m), 3.32 (3H,s), 3.59-3.62 (2H,m), 4.26 (2H,s), 6.98 (1H,d, J=8.8Hz), 7.20-7.27 (2H,m), 7.73 (2H,d,J=8.0Hz), 7.78 (1H, dd, J=8. 8,2.0Hz), 7.87 (1H,brs), 8.18 (1H,brs), 8.24 (2H, d,J=8.0Hz), 8.56 (1H,s), 8.75 -9.08 (1H,brs) 9.97 (1H, s).

**Example 30**

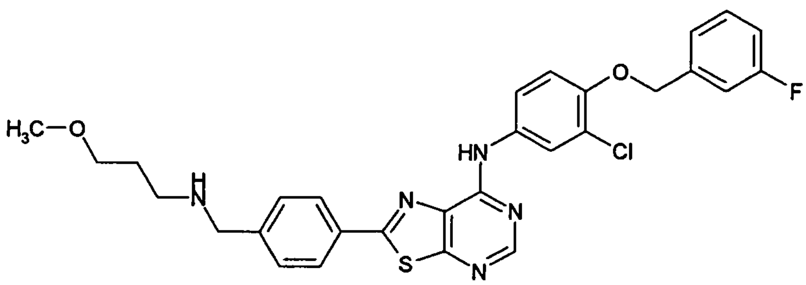

N-{3-chloro-4-[(3-fluorobenzyl)oxy]phenyl}-2-(4-{[(3-methoxypropyl)amino]methyl}phenyl)[1,3]thiazolo[5,4-d]pyrimidin-7-amine

The compound produced in Reference Example 20 (0.36g) was dissolved in dichloromethane (10ml), m-chloroperbenzoic acid (0.15g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy] aniline (0.39g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was recrystallized from ethyl acetate/triethylamine to obtain the title compound (0.25g).

$^1$H-NMR (DMSO-$d_6$) δ:1.72-1.79(2H,m),2.74(2H,d,J=4.9Hz), 3.23(3H,s),3.37-3.41(2H,m),3.98(2H,s),5.26(2H,s), 7.16-7.21(1H,m),7.26-7.35(3H,m),7.45-7.51(1H,m),7.62(2H,d, J=5.7Hz),7.80-7.83(1H,m),8.11-8.12(1H,m),8.16(2H,d, J=5.7Hz),8.55(1H,s),9.99(1H,s).

**Example 31**

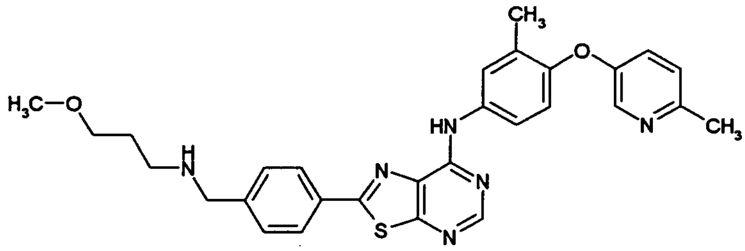

2-(4-{[(3-methoxypropyl)amino]methyl}phenyl)-N-{3-methyl-4-[(6-methylpyridin-3-yl)oxy]phenyl}[1,3]thiazolo[5,4-d]pyrimidin-7-amine

The compound produced in Reference Example 20 (0.35g) was dissolved in dichloromethane (10ml), m-chloroperbenzoic acid (0.15g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.33g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was dissolved in triethylamine (1ml), and purified with column chromatography (basic silica gel, developing solvent : ethyl acetate) to obtain the title compound (0.19g).

$^1$H-NMR (DMSO-$d_6$) δ:1.84-1.91 (2H,m), 2.22 (3H,s), 2.45 (3H,s), 2.99 (2H,t,J=5.2Hz), 3.25 (3H,s), 3.39 (2H,m), 4.25 (2H,s), 6.99 (1H, d,J=5.9Hz), 7.21-7.27 (2H,m), 7.72 (2H,d,J=5.4Hz), 7.78 (1H,dd,J=5.9, 1.8Hz), 7.88 (1H,d,J=1.6Hz), 8.19 (1H,d,J=1.6Hz), 8. 25 (2H,d,J=5.4Hz), 8.56 (1H,s), 9.97(1H,s).

**Example 32**

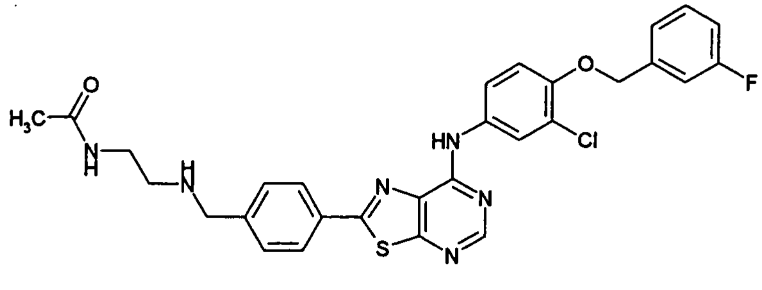

N-[2-({4-[7-({3-chloro-4-[(3-fluorobenzyl)oxy]phenyl}amino)[1,3]thiazolo[5,4-d]pyrimidin-2-yl]benzyl}amino)ethyl]acetamide

The compound produced in Reference Example 21 (0.50g) was dissolved in dichloromethane (10ml), m-chloroperbenzoic acid (0.21g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy]aniline (0.53g) was added thereto. The reaction solution was stirred at 120°C for 2 hours, a saturated aqueous sodium

hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was recrystallized from ethyl acetate/hexane/triethylamine to obtain the title compound (0.31g).
$^1$H-NMR(DMSO-$d_6$) δ:1.80(3H,s),2.56(2H,t,J=6.0Hz), 3.15(2H,q,J=6.0Hz),3.79(2H,s),5.26(2H,s),7.17-7.22(1H,m), 7.26-7.35(3H,m),7.45-7.51(1H,m),7.56(2H,d,J=8.4Hz),7.80-7.83(2H,m),8.11(2H,d,J=8.4Hz),8.12(1H,s),9.54(1H,s), 9.99(1H, s) .

**Example 33**

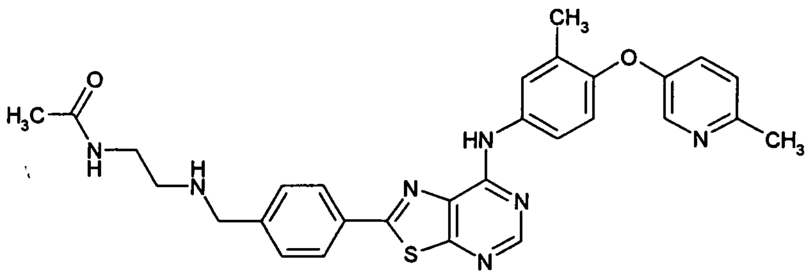

N-[2-({4-[7-({3-methyl-4-[(6-methylpyridin-3-yl)oxy] phenyl}amino)[1,3]thiazolo[5,4-d]pyrimidin-2-yl]benzyl}amino)phenyl]acetamide

The compound produced in Reference Example 21 (0.50g) was dissolved in dichloromethane (10ml), m-chloroperbenzoic acid (0.20g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.45g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (5ml), and then the mixture was stirred at room temperature for 10 minutes. The solvent was distilled off, and the residue was dissolved in triethylamine (lml), and purified with column chromatography (basic silica gel, developing solvent : ethyl acetate) to obtain the title compound (0.26g).
$^1$H-NMR(DMSO-$d_6$) δ:1.85(3H,s),2.22(3H,s),2.45(3H,s), 3.12-3.07(2H,m),3.41-3.92(1H,brs),4.30(2H,s),6.99(1H,d, J=8.8Hz),7.23-7.28(2H,m),7.73(2H,d,J=8.4Hz),7.79(1H,dd, J=8.8,2.4Hz),7.88(1H,brs),8.13-8.17(1H,m),8.20(1H,brs), 8.25 (2H,d,J=8.4Hz),8.56(1H,s),9.07(1H,brs),9.99(1H,s).

**Example 34**

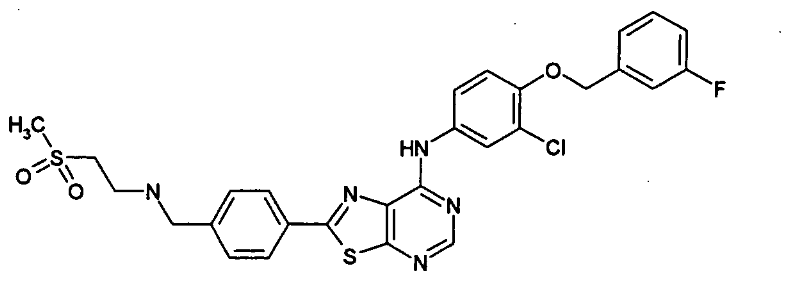

N-{3-chloro-4-[(3-fluorobenzyl)oxy]phenyl}-2-[4-({[2-(methylsulfonyl)ethyl]amino}methyl)phenyl][1,3]thiazolo[5,4-d]pyrimidin-7-amine

The compound produced in Reference Example 22 (0.20g) was dissolved in dichloromethane (2.5ml), m-chloroperbenzoic acid (0.23g) was added thereto under an ice cooling condition, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-chloro-4-[(3-fluorobenzyl)oxy]aniline (0.80g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (4ml), and then the mixture was stirred at room temperature for 10 minutes. The solvent was distilled off, and the residue was recrystallized from ethyl acetate/hexane/triethylamine to obtain the title compound (0.056g).

$^1$H-NMR ($CDCl_3$) δ:3.02 (3H,s), 3.18-3.21 (4H,brm), 3.92 (2H,s), 5.17 (2H,s), 6.98-7.04 (2H,m), 7.21-7.24 (2H,m), 7.34-7.38 (1H,m), 7.47 (2H,d,J=8.4Hz), 7.89 (1H,brs), 7.98 (1H,brs), 8.03 (2H,d,J=8.4Hz), 8.60 (1H,s).

## Example 35

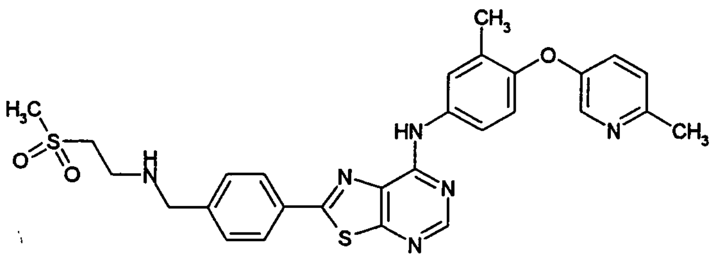

N-{3-methyl-4-[(6-methylpyridin-3-yl)oxy]phenyl}-2-[4-({[2-(methylsulfonyl)ethyl]amino}methyl)phenyl][1,3]thiazolo[5,4-d]pyrimidin-7-amine

The compound produced in Reference Example 22 (0.10g) was dissolved in dichloromethane (4ml), m-chloroperbenzoic acid (0.14g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (3ml), and 3-methyl-4-[(6-methylpyridin-3-yl)oxy]aniline (0.080g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (3ml), and then the mixture was stirred at room temperature for 10 minutes. The solvent was distilled off, and the residue was dissolved in triethylamine (0.5ml), and purified with column chromatography (basic silica gel, developing solvent : ethyl acetate) to obtain the title compound (0.037g).

$^1$H-NMR($CDCl_3$) δ:2.31(3H,s),2.54(3H,s),3.02(3H,s),3.19-3.22(4H,brm),3.92(2H,s),6.95(1H,d,J=8.8Hz),7.09(1H,d, J=8.4Hz ),7.12(1H,dd,J=8.4,2.8Hz),7.48(2H,d,J=8.0Hz),7.66(1H,dd,J=8. 8,2.4Hz),7.73(1H,d,J=2.4Hz),8.04(2H,d, J=8.OHz),8.26(1H,d,J=2 .4Hz),8.61(1H,s).

## Example 36

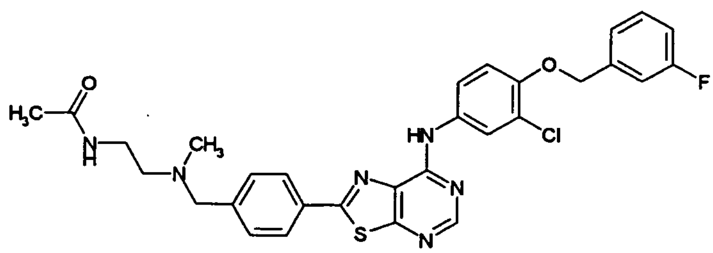

N-{2-[{4-[7-({3-chloro-4-[(3-fluorobenzyl)oxy]phenyl} amino)[1,3]thiazolo[5,4-d]pyrimidin-2-yl]benzyl}(methyl)amino] ethyl}acetamide

The compound produced in Example 32 (0.10g) was dissolved in N,N-dimethylformamide (3ml), potassium carbonate (0.023g) and iodomethane (0.025g) were added thereto, and the mixture was stirred for 1 hour. A saturated aqueous ammonium chloride solution was added thereto under an ice cooling condition, and the mixture was extracted with ethyl acetate. The extract was dried and concentrated, and the residue was purified with column chromatography (basic silica gel, developing solvent : ethyl acetate) to obtain the title compound (0.061g).

$^1$H-NMR($CDCl_3$) δ:2.00(3H,s),2.28(3H,s),2.56(2H,t, J=5.6Hz),3.39(2H,q,J=5.6Hz),3.61(2H,s),5.17(2H,s),5.96(1H,br s), 6.99-7.06(2H,m),7.10-7.24(2H,m),7.34-7.40(1H,m),7.45(2H,d, J=8.0Hz),7.63-7.66(1H,m),7.89(1H,brs),7.97(1H,brs), 8.03 (2H,d,J=8.0Hz),8.60(1H,s) .

**Example 37**

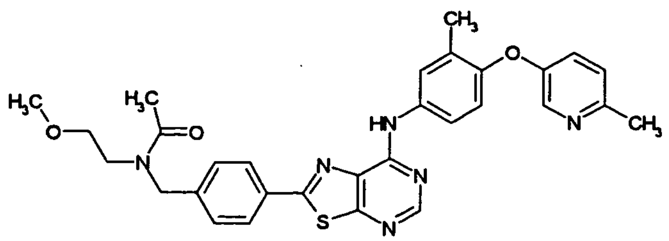

N-(2-methoxyethyl)-N-{4-[7-({3-methyl-4-[(6-methylpyridin-3-yl)oxy]phenyl}amino)[1,3]thiazolo[5,4-d]pyrimdin-2-yl] benzyl}acetamide

The compound produced in Example 29 (0.10g) was dissolved in tetrahydrofuran (3ml), acetic anhydride (0.4ml) and triethylamine (0.2ml) were added under an ice cooling condition thereto, and the mixture was stirred for 30 minutes. A saturated aqueous sodium hydrogencarbonate was added thereto, and the mixture was extracted with ethyl acetate. The extract was dried and concentrated, and the residue was purified with column chromatography (basic silica gel, developing solvent : ethyl acetate/n-hexane=2/1) to obtain the title compound (0.083g).

$^1$H-NMR($CDCl_3$) δ:2.25(3H,s),2.30(3H,s),2.53(3H,s),3.32 (3H,s),3.47(2H,s),3.57-3.62(2H,m),4.74(2H,brs),6.94(1H,d, J=8.5Hz),7.07-7.13(2H,m),7.32-7.34(1H,m),7.40(1H,d,J=8.1Hz), 7.65(1H,d,J=8.5Hz),7.72(1H,brs),7.99-8.01(2H,m), 8.06(1H,d, J=8.1Hz),8.26(1H,brs),8.60(1H,s).

**Example 38**

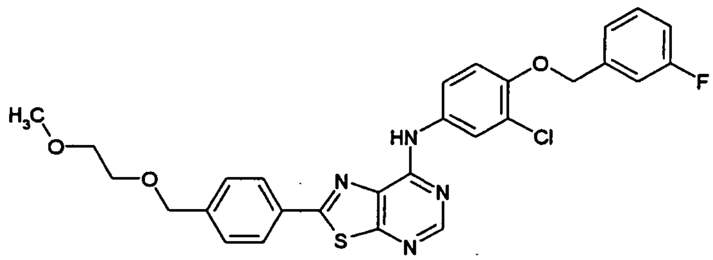

N-{3-chloro-4-[(3-fluorobenzyl)oxy]phenyl}-2-{4-[(2-methoxyethoxy)methyl]phenyl}[1,3]thiazolo[5,4-d]pyrimidin-7-amine

2-Methoxyethanol (0.8ml) was dissolved in N,N-dimethylformamide (3ml), sodium hydride (0.023g) was added thereto under an ice cooling condition, and the mixture was stirred for 30 minutes. The compound produced in Reference Example 17 (0.40g) was added thereto, and the mixture was further stirred for 1 hour. A saturated aqueous ammonium chloride solution was added thereto under an ice cooling condition, and the mixture was extracted with ethyl acetate. The extract was dried and concentrated. The residue was purified with column chromatography (basic silica gel, developing solvent : ethyl acetate), and an oily matter (0.21g) was obtained. The oily matter was dissolved in dichloromethane

(10ml), m-chloroperbenzoic acid (0.10g) was added thereto under an ice cooling condition, and the mixture was stirred for 10 minutes. The organic layer was washed by adding a aqueous saturated sodium hydrogencarbonate under an ice cooling condition, dried and concentrated. The residue was dissolved in 1-methyl-2-pyrrolidone (7ml), and 3-chloro-4-[(3-fluorobenzyl)oxy]aniline (0.061g) was added thereto. The reaction solution was stirred at 80°C for 2 hours, a saturated aqueous sodium hydrogencarbonate was added thereto under an ice cooling condition, and the mixture was extracted with ethyl acetate. The extract was dried and concentrated. The residue was purified with column chromatography (basic silica gel, developing solvent : ethyl acetate/n-hexane=1/4) to obtain the title compound (0.017g).
$^1$H-NMR($CDCl_3$) δ:3.43(3H,s),3.46-3.64(2H,m),3.67-3.70(2H,m),4.67(2H,s),5.17(2H,s),6.99-7.06(2H,m),7.21-7.25(2H,m),7.31-7.38(1H,m),7.51(2H,d,J=8.1Hz),7.63-7.66(1H,m),7.89(1H,brs),7.98(1H,brs),8.04(2H,d,J=8.1Hz),8.60 (1H,s).

**Example 39**

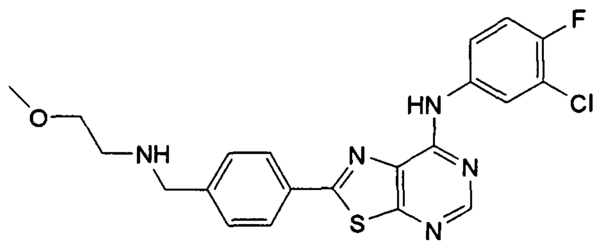

N-(3-chloro-4-fluorophenyl)-2-(4-{[(2-methoxyethyl)amino]methyl}phenyl)[1,3]thiazolo[5,4-d]pyrimidin-7-amine

The compound produced in Reference Example 19 (0.20g) was dissolved in dichloromethane (5ml), m-chloroperbenzoic acid (0.11g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (3ml), and 3-chloro-4-fluoroaniline (0.065g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/4), the obtained compound was dissolved in trifluoroacetic acid (3ml), and then the mixture was stirred at room temperature for 10 minutes. The solvent was distilled off, and the residue was dissolved in triethylamine (1ml), and purified with column chromatography (basic silica gel, developing solvent : ethyl acetate) to obtain the title compound (0.15g).
$^1$H-NMR($CDCl_3$) δ:2.83-2.86(2H,m),3.54(3H,s),3.54-3.56(2H,m), 3.91(2H,s),7.21-7.17(1H,m),7.49-7.68(3H,m),7.96-8.11(4H,m), 8.62(1H,s).

**Example 40**

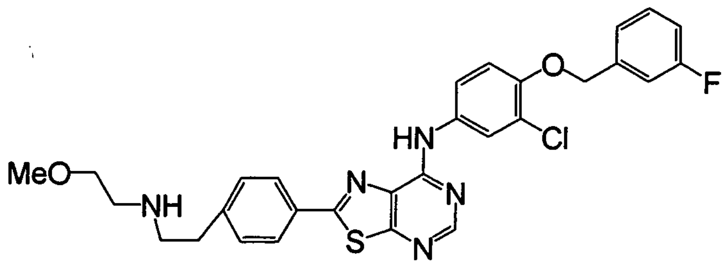

N-{3-chloro-4-[(3-fluorobenzyl)oxy]phenyl}-2-(4-{2-[(2-methoxyethyl)amino]ethyl}phenyl)[1,3]thiazolo[5,4-d]pyrimdin-7-amine

The compound produced in Reference Example 23 (0.25g) was dissolved in dichloromethane (8ml), m-chloroperbenzoic acid (0.20g) was added thereto under an ice cooling condition, and the mixture was stirred for 10 minutes.

The organic layer was washed by adding a saturated aqueous sodium hydrogencarbonate under an ice cooling condition, dried and concentrated. The residue was dissolved in 1-methyl-2-pyrrolidone (3ml), and 3-chloro-4-[(3-fluorobenzyl)oxy] aniline (0.22g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/1), and a yellow powder was obtained. The compound (0.040g) was dissolved in acetonitrile (2ml), and 2-methoxyethylamine (0.5ml) and sodium iodide (0.017g) were added thereto. The reaction solution was stirred at 70°C for 1 hour, a saturated aqueous sodium hydrogencarbonate was added thereto under an ice cooling condition, and then the mixture was extracted with ethyl acetate. The extract was dried and concentrated, and the residue was purified with column chromatography (basic silica gel, developing solvent : ethyl acetate) to obtain the title compound (0.026g).

$^1$H-NMR($CDCl_3$) $\delta$:2.81-2.84(2H,m),2.89-2.96(4H,m),3.35(3H,s), 3.43-3.51(2H,m),5.16(2H,s),5.87 (1H,brs),6.70-6.97 (2H,d,m), 7.20-7.26(2H,m),7.33-7.37 (3H,m),7.62-7.64(1H,m),7.97-7.99(4H,m),8.58(1H,s).

**Example 41**

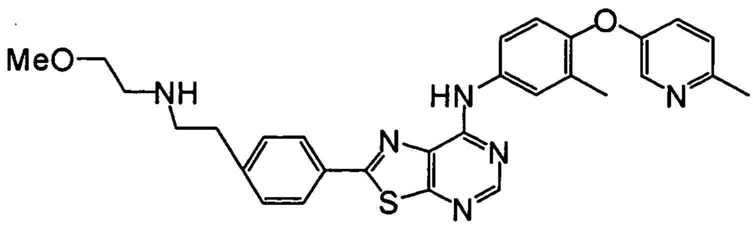

2-(4-{2-[(2-methoxyethyl)amino]ethyl}phenyl)-N-{3-methyl-4-[(6-methylpyridin-3-yl)oxy]phenyl}[1,3]thiazolo[5,4-d]pyrimidin-7-amine

The compound produced in Reference Example 23 (0.25g) was dissolved in dichloromethane (8ml), m-chloroperbenzoic acid (0.20g) was added thereto under an ice cooling condition, and the mixture was stirred for 10 minutes. The organic layer was washed by adding a saturated aqueous sodium hydrogencarbonate under an ice cooling condition, dried and concentrated. The residue was dissolved in 1-methyl-2-pyrrolidone (5ml), and 3-methyl-4-[(6-methylpyridin-3-yl)oxy]aniline (0.18g) was added thereto. The reaction solution was stirred at 120°C for 2 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate/n-hexane=1/1), and a yellow powder was obtained. The compound (0.030g) was dissolved in acetonitrile (1.5ml), and 2-methoxyethylamine (0.3ml) and sodium iodide (0.015g) were added thereto. The reaction solution was stirred at 70°C for 1 hour, a saturated aqueous sodium hydrogencarbonate was added thereto under an ice cooling condition, and then the mixture was extracted with ethyl acetate. The extract was dried and concentrated, and the residue was purified with column chromatography (basic silica gel, developing solvent : ethyl acetate) to obtain the title compound (0.017g).

$^1$H-NMR($CDCl_3$) $\delta$:2.31(3H,s),2.54(3H,s),2.83-2.98(6H,m), 3.36(3H,s),3.49-3.52(2H,s),6.94-6.96(1H,m),7.08-7.14(2H, m), 7.36-7.38(2H,m),7.64-7.74(2H,m),7.96-8.01(1H,m),7.92(3H,m), 8.27(1H,m),8.60(1H,s).

**Example 42**

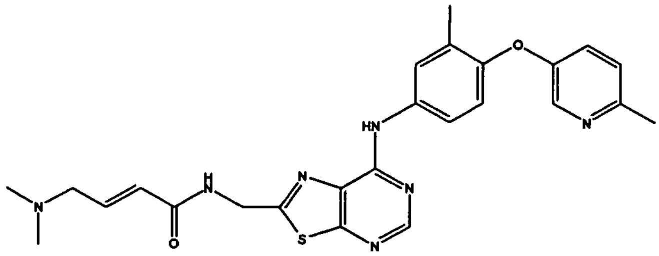

(2E)-4-(dimethylamino)-N-{[7-({3-methyl-4-[(6-methylpyridin-3-yl)oxy]phenyl}amino)[1,3]thiazolo[5,4-d]pyrimidin-2-yl]methyl}but-2-enamide

The compound produced in Example 17 (0.20g) was dissolved in dichloromethane (10ml), triethylamine (0.5ml) and p-toluenesulfonyl chloride (0.12g) were added under an ice cooling condition thereto, and the mixture was stirred for 4 hours. The organic layer was washed by adding a saturated aqueous sodium hydrogencarbonate under an ice cooling condition, dried and concentrated. The residue was purified with column chromatography (silica gel, developing solvent : ethyl acetate/n-hexane=1/3), and a brown powder (0.26g) was obtained. The compound (0.16g) was dissolved in acetonitrile (8ml), sodium azide (0.053g) was added thereto, and the mixture was stirred at 70°C for 6 hours. A saturated sodium aqueous hydrogencarbonate was added thereto under an ice cooling condition, and the mixture was extracted with ethyl acetate. The extract was dried and concentrated. The residue was dissolved in ethyl acetate (5ml), 10% Palladium/activated carbon (0.09g) was added thereto, and the mixture was stirred under a hydrogen atmosphere for 10 minutes. The reaction solution was filtered and the filtrate was concentrated. The residue was dissolved in tetrahydrofuran (10ml), 1-hydroxy-1H-benzotriazole monohydrate (0.066g), 1-ethyl-3-(3-dimethylaminopropyl)-carbodiimide (0.080g) and (2E)-4-(dimethylamino)but-2-enoic acid (0.042g) were added thereto, and the mixture was stirred for 8 hours. A saturated sodium aqueous hydrogencarbonate was added thereto under an ice cooling condition, and the mixture was extracted with ethyl acetate. The extract was dried and concentrated, and the residue was purified with column chromatography (basic silica gel, developing solvent : ethyl acetate) to obtain the title compound (0.012g).
$^1$H-NMR($CDCl_3$) δ:2.30(6H,s),2.31(3H,s),2.55(3H,s),3.11-3.13(2H,m),4.93-4.94(2H,m),6.09-6.13(1H,m),6.36(1H,brt), 6.93-7.01(2H,m),7.09-7.15(2H,m),7.62-7.65(1H,m),7.71(1H,s), 7.84(1H,s),8.27(1H,s),8.61(1H,s).

**Example 43**

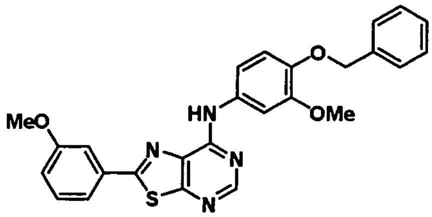

N-[4-(benzyloxy)-3-methoxyphenyl]-2-(3-methoxyphenyl)[1,3]thiazolo[5,4-d]pyrimidin-7-amine

The compound produced in Reference Example 2 (0.50g) was dissolved in chloroform (15ml), m-chloroperbenzoic acid (0.64g) was added thereto, and the mixture was stirred at room temperature for 30 minutes. The reaction solution was washed with a 5% aqueous sodium thiosulfate solution (5ml), a 5% aqueous sodium hydrogencarbonate (10ml) and a saturated saline solution (10ml) respectively, dried and then concentrated. The crystal (0.10g) was dissolved in 1-methyl-2-pyrrolidone (3ml), 4-benzyloxy-3-methoxyaniline (0.14g) was added thereto, and the mixture was stirred at 160°C for 1 hour. The reaction solution was poured into water, and the mixture was extracted with ethyl acetate. The extract was dried and concentrated. The residue was purified with column chromatography (silica gel, developing solvent : ethyl acetate/n-hexane=2/1), and crystallized from ethyl acetate/n-hexane=1/4 to obtain the title compound (0.088g).
$^1$H-NMR($CDCl_3$) δ:3.93(3H,s),3.96(3H,s),5.18(2H,s),6.92 (1H,d,J=8.8Hz),7.00-7.70(11H,m),7.91(1H,brs),8.58(1H,s).

**Example 44**

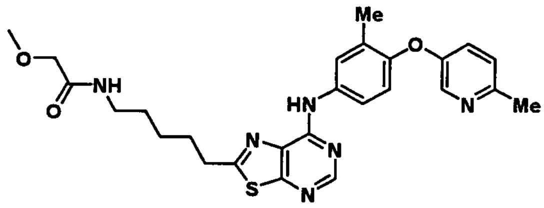

2-methoxy-N-{5-[7-({3-methyl-4-[(6-methylpyridin-3-yl)oxy]phenyl}amino)[1,3]thiazolo[5,4-d]pyrimidin-2-yl]pentyl}acetamide

The compound produced in Reference Example 26 (0.37g) was dissolved in methanol (30ml), 10% Palladium carbon (contains water, 0.15g) was added thereto, and the mixture was stirred under a hydrogen atmosphere at room temperature for 5 hours. The catalyst was filtered off, and the filtrate was concentrated. The residue was dissolved in tetrahydrofuran (20ml), methoxyacetyl chloride (0.1ml) and triethylamine (0.28ml) were added thereto, and the mixture was stirred under an ice cooling condition for 30 minutes. To the reaction solution was added water, and the mixture was stirred, concentrated, and extracted with ethyl acetate. The extract was dried and concentrated. The residue was purified with column chromatography (basic silica gel, developing solvent : ethyl acetate/n-hexane=2/1), and recrystallized from ethyl acetate/n-hexane=1/2 to obtain the title compound (0.21g).

$^1$H-NMR($CDCl_3$) δ:1.40-1.75(4H,m),1.82-2.00(2H,m),2.29(3H,s), 2.53(3H,s),3.11(2H,t,J=7.4Hz),3.34(2H,q,J=6.6Hz), 3.40(3H,s), 3.88(2H,s),6.55(1H,br),6.93(1H,d,J=8.8Hz),7.00-7.15(2H,m), 7.60-7.75(2H,m),7.93(1H,brs),8.22-8.30(1H, m),8.58 (1H,s).

**Example 45**

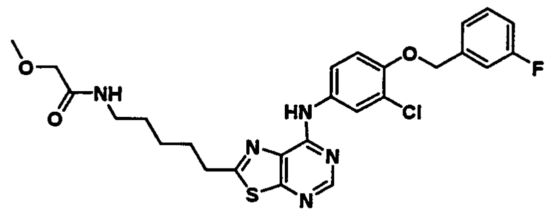

N-{5-[7-({3-chloro-4-[(3-fluorobenzyl)oxy]phenyl}amino)[1,3]thiazolo[5,4-d]pyrimidin-2-yl]pentyl}-2-methoxyacetamide

The compound produced in Reference Example 27 (0.34g) and nickel bromide (0.015g) were dissolved in tetrahydrofuran (6ml) and methanol (5ml), sodium borohydride (0.13g) was added by the manner as divided several times thereto, and the mixture was stirred under an ice cooling condition for 1 hour, and at room temperature for 2 hours respectively. The reaction solution was concentrated, water was added thereto, and then the mixture was extracted with ethyl acetate. The extract was dried and concentrated. The residue was dissolved in ethyl acetate (20ml), methoxyacetyl chloride (0.1ml) and triethylamine (0.28ml) were added thereto, and the mixture was stirred under an ice cooling condition for 30 minutes. To the reaction solution was added water, and the mixture was stirred, and extracted with ethyl acetate. The extract was dried and concentrated. The residue was purified with column chromatography (silica gel, developing solvent : ethyl acetate-ethyl acetate/methanol=10/1), and recrystallized from ethyl acetate to obtain the title compound (0.028g).

$^1$H-NMR($CDCl_3$) δ:1.40-1.75(4H,m),1.82-2.02(2H,m),3.10 (2H,t,J=7.2Hz),3.34(2H,q,J=6.6Hz),3.40(3H,s),3.88(2H,s), 5.16 (2H,s), 6.48-6.70 (1H,m), 6.95-7.10 (1H,m), 6.97 (1H,d,J=8.8Hz), 7.15-7.45 (3H,m), 7.62 (1H,dd,J=2.6,8.8Hz), 7.89 (1H,brs), 7.97 (1H,d,J=2.6Hz), 8.57 (1H,s).

**Example 46**

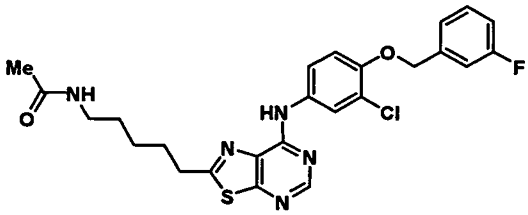

N-{5-[7-({3-chloro-4-[(3-fluorobenzyl)oxy]phenyl}amino)[1,3]thiazolo[5,4-d]pyrimidin-2-yl]pentyl}acetamide

The compound produced in Reference Example 27 (0.23g) was dissolved in tetrahydrofuran (6ml) and water (2ml), triphenylphosphine (2.4g) was added thereto, and the mixture was stirred at room temperature for 4 hours. To the reaction solution were added acetic anhydride (0.2ml), a 5% sodium aqueous hydrogencarbonate (10ml) and a saturated saline solution (2ml), and the mixture was stirred at room temperature for 1 hour. The reaction solution was poured into water, and the mixture was extracted with ethyl acetate. The extract was dried and concentrated, and then the residue was recrystallized from ethyl acetate to obtain the title compound (0.10g).

$^1$H-NMR($CDCl_3$) δ:1.35-1.70(4H,m),1.85-2.05(2H,m),1.97(3H,s), 3.10(2H,t,J=7.4Hz),3.28(2H,q,J=6.6Hz),5.16(2H,s), 5.50(1H,brs ),6.95-7.10(1H,m),6.97(1H,d,J=8.8Hz),7.12-7.45(3H,m), 7.62(1H,dd,J=2.6Hz,8.8Hz),7.91(1H,brs),7.97 (1H,d,J=2.6Hz),8. 57(1H,s).

**Example 47**

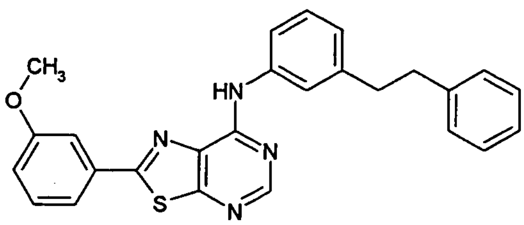

2-(3-methoxyphenyl)-N-[3-(2-phenylethyl)phenyl][1,3]thiazolo[5,4-d]pyrimidin-7-amine

In the same manner as shown in Example 1, the title compound (0.16g) was obtained from the compound produced in Reference Example 2 (0.20g) and 3-(2-phenylethyl)aniline (0.27g).

$^1$H-NMR($CDCl_3$) δ:2.99(4H,s),3.93(3H,s),6.99(1H,d,J=7.5Hz), 7.07(1H,m),7.18-7.36(6H,m),7.42(1H,t,J=8.4Hz), 7.59-7.65 (2H,m), 7.73 (1H,d,J=7.2Hz), 7.99 (1H,s), 8.61 (1H,s).

**Example 48**

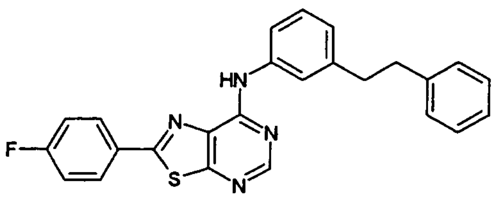

2-(4-fluorophenyl)-N-[3-(2-phenylethyl)phenyl][1,3]thiazolo[5,4-d]pyrimidin-7-amine

In the same manner as shown in Example 1, the title compound (0.17g) was obtained from 2-(4-fluorophenyl)-7-(methylthio)[1,3]thiazolo[5,4-d]pyrimidine (0.20g) and 3-(2-phenylethyl)aniline (0.28g).

$^1$H-NMR ($CDCl_3$) δ:2.99 (4H,s), 6.99 (1H,d,J=7.5Hz), 7.16-7.37 (7H,m), 7.64 (1H,s), 7.73 (1H,d,J=8.4Hz), 7.95 (1H,s), 8.04-8.10 (2H,m), 8.61 (1H,s).

**Example 49**

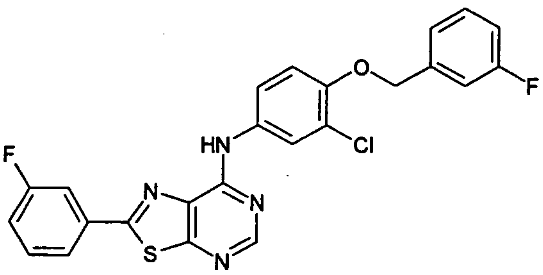

N-{3-chloro-4-[(3-fluorobenzyl)oxy]phenyl}-2-(3-fluorophenyl)[1,3]thiazolo[5,4-d]pyrimidin-7-amine

In the same manner as shown in Example 1, the title compound (0.07g) was obtained from 2-(3-fluorophenyl)-7-(methylthio)[1,3]thiazolo[5,4-d]pyrimidine (0.10g) and 3-chloro-4-[(3-fluorobenzyl)oxy]aniline (0.18g).
$^1$H-NMR ($CDCl_3$) δ:5.17 (2H,s), 6.95-7.08 (2H,m), 7.18-7.28 (2H,m), 7.35 (1H,m), 7.50 (1H,m), 7.63 (1H,m), 7.77-7.90 (3H,m), 7.98 (1H,d,J=2.7Hz), 8.60 (1H,s).

**Example 50**

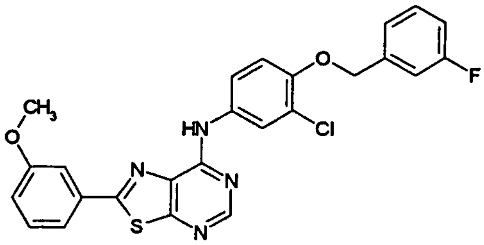

N-{3-chloro-4-[(3-fluorobenzyl)oxy]phenyl}-2-(3-methoxyphenyl)[1,3]thiazolo[5,4-d]pyrimidin-7-amine

In the same manner as shown in Example 1, the title compound (0.26g) was obtained from the compound produced in Reference Example 2 (0.22g) and 3-chloro-4-[(3-fluorobenzyl)oxy]aniline (0.38g).
$^1$H-NMR ($CDCl_3$) δ:3.93(3H,s),5.16(2H,s),6.97-7.10(3H,m),7.18-7.25 (1H,m), 7.32-7.45 (2H,m), 7.55-7.65 (3H,m), 7.88 (1H,s), 7.96 (1H,d,J=2.7Hz), 8.56(1H,s).

**Example 51**

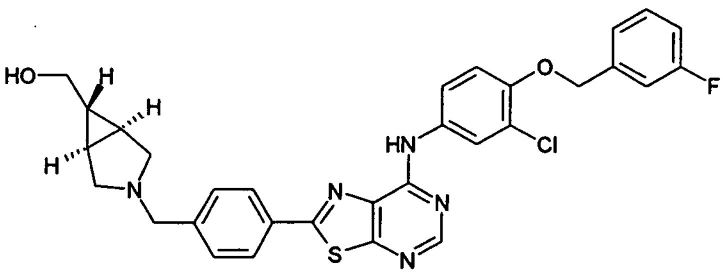

(3-{4-[7-({3-chloro-4-[(3-fluorobenzyl)oxy]phenyl}amino)[1,3]thiazolo[5,4-d]pyrimidin-2-yl]benzyl}-3-azabicyclo[3.1.0]hex-6-yl)methanol

The compound produced in Reference Example 28 (0.10g) was dissolved in dichloromethane (15ml), concentrated hydrochloric acid (50mg) was added thereto, and the mixture was stirred for 20 minutes. The solvent was distilled off. The obtained solid was dissolved in 1-methyl-2-pyrrolidone/water (3ml/3ml), m-chloroperbenzoic acid (0.096g) was added thereto, and then the mixture was stirred at room temperature for 20 minutes. To the reaction solution was added 3-chloro-4-[(3-fluorobenzyl)oxy]aniline (0.13g), and the mixture was stirred at 120°C for 3 hours. The reaction solution was poured into water, and basified by adding a 1N sodium hydroxide, and the mixture was extracted with ethyl acetate. The extract was washed with water, dried and concentrated. The residue was purified with column chromatography (basic silica gel, developing solvent : ethyl acetate), and crystallized from dichloromethane/diethyl ether=1/1 to obtain the title compound (0.11g).

$^1$H-NMR ($CDCl_3$) δ:1.30-1.45 (3H,m), 1.61 (1H,m), 2.40 (2H,d,J=8.8Hz), 3.02 (2H,d,J=8.8Hz), 3.47 (2H,d,J=7.2Hz), 3.67 (2H ,s), 5.17 (2H,s), 6.95-7.08 (2H,m), 7.22 (1H,m), 7.33-7.48 (3H,m), 7.63 (1H,dd,J=9.0,2.8Hz) 7.91 (1H,s), 7.95-8.03 (3H,m), 8.59 (1H, s).

**Example 52**

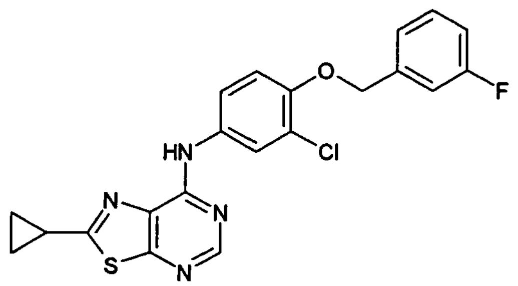

N-{3-chloro-4-[(3-fluorobenzyl)oxy]phenyl}-2-cyclopropyl[1,3]thiazolo[5,4-d]pyrimidin-7-amine

In the same manner as shown in Example 1, the title compound (0.15g) was obtained from 2-cyclopropyl-7-(methylthio)[1,3]thiazolo[5,4-d]pyrimidine (0.12g) and 3-chloro-4-[(3-fluorobenzyl)oxy]aniline (0.27g).

$^1$H-NMR($CDCl_3$) δ:1.20-1.32 (4H,m) ,2.38 (1H,m) ,5.16 (2H,s), 6.93-7.10 (2H,m), 7.16-7.43 (2H,m),7.59 (1H,dd, J=8.8,2.6 Hz), 7.66 (1H,s),7.93 (1H,d,J=2.4Hz) 8.54 (1H,s).

Example 53

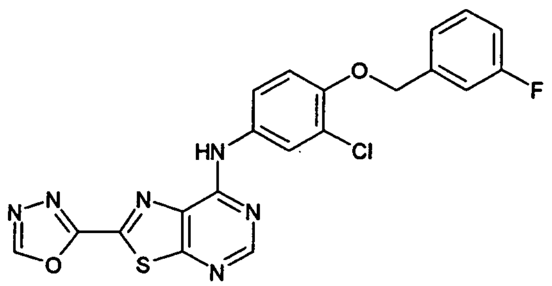

N-{3-chloro-4-[(3-fluorobenzyl)oxy]phenyl}-2-(1,3,4-oxadiazol-2-yl)[1,3]thiazolo[5,4-d]pyrimidin-7-amine

In the same manner as shown in Example 1, the title compound (0.015g) was obtained from 2-(1,3,4-oxadiazol-2-yl)-7-(methylthio)[1,3]thiazolo[5,4-d]pyrimidine (0.076g) and 3-chloro-4-[(3-fluorobenzyl)oxy]aniline (0.15g).

$^1$H-NMR ($CDCl_3$) δ:5.19 (2H, s), 6.97-7.10 (2H,m), 7.15-7.47 (2H,m), 7.59 (1H,m), 8.01 (2H,m), 8.66(1H,s) 8.70(1H,s).

**Example 54**

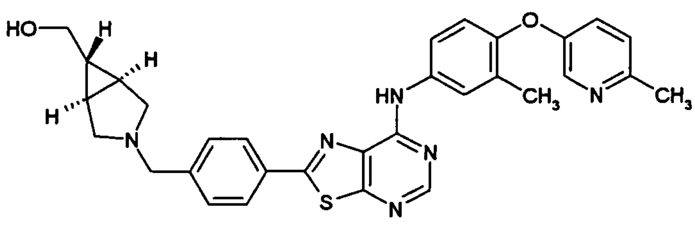

(3-{4-[7-({3-methyl-4-[(6-methylpyridin-3-yl)oxy]phenyl}amino)[1,3]thiazolo[5,4-d]pyrimidin-2-yl]benzyl}-3-azabicyclo [3.1.0]hex-6-yl)methanol

In the same manner as shown in Example 51, the title compound (0.13g) was obtained from the compound produced in Reference Example 28 (0.13g) and 3-methyl-4-[(6-methylpyridin-3-yl)oxy]aniline (0.14g).

$^1$H-NMR($CDCl_3$) δ:1.32(2H,brs),1.62(1H,m),1.91(1H,br), 2.29(3H,s),2.39(2H,d,J=8.6Hz),2.53(3H,s),3.01(2H,d, J=8.6Hz), 3.47(2H,d,J=7.4Hz),3.66(2H,s),6.94(1H,d,J=8.8Hz),7.05-7.16(2H,m),7.41(2H,d,J=8.0Hz),7.60-7.75(2H,m), 7.97(2H,d, J=8.0Hz),8.05(1H,s),8.27(1H,m),8.59(1H,s).

### Example 55

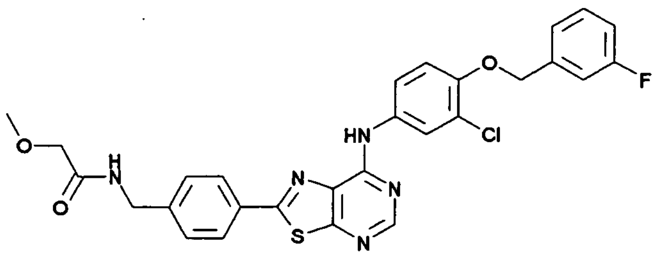

N-{4-[7-({3-chloro-4-[(3-fluorobenzyl)oxy]phenyl}amino)[1,3]thiazolo[5,4-d]pyrimidin-2-yl]benzyl}-2-methoxyacetamide

The compound produced in Reference Example 17 (1.00g) was dissolved in acetonitrile (50ml), sodium azide (850mg) was added thereto, and the mixture was stirred at 70°C for 5 hours. A saturated aqueous sodium hydrogencarbonate was added thereto under an ice cooling condition, and the mixture was extracted with ethyl acetate. The extract was dried and concentrated. The residue was dissolved in dichloromethane (30ml), and m-chloroperbenzoic acid (817mg) was added thereto, and then the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (16ml), and 3-chloro-4-[(3-fluorobenzyl)oxy]aniline (1.20g) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, the residue was purified with column chromatography (silica gel, developing solvent : ethyl acetate/n-hexane = 5/1), and then a pale yellow powder (1.32g) was obtained. The compound (0.10g) was dissolved in tetrahydrofuran (5ml), water (1ml) and triphenylphosphine (160mg) were added thereto, and the mixture was stirred for 24 hours. To the reaction solution was added a saturated aqueous sodium hydrogencarbonate, and the mixture was extracted with ethyl acetate. The extract was dried and concentrated. The residue was purified with column chromatography (basic silica gel, developing solvent : ethyl acetate), and a yellow powder (0.067g) was obtained. The compound (0.020g) was dissolved in tetrahydrofuran (2ml), triethylamine (0.054g) and methoxyacetyl chloride (0.011ml) were added thereto under an ice cooling condition, and the mixture was stirred for 10 minutes. To the reaction solution was added a saturated aqueous sodium hydrogencarbonate, and the mixture was extracted with ethyl acetate. The extract was dried and concentrated and the residue was purified with column chromatography (basic silica gel, developing solvent : ethyl acetate) to obtain the title compound (0.017g).

$^1$H-NMR(DMSO-$d_6$) δ:3.35(3H,s),3.90(2H,s),4.40(2H,d,J=6.OHz), 5.26(2H,s),7.16-7.21(1H,m),7.26-7.35(3H,m), 7.45-7.50(3H,m), 7.79-7.82(1H,m) 8.10-8.12 (2H,m), 8.49-8.54 (3H,m) , 9.99 (1H, s).

**Example 56**

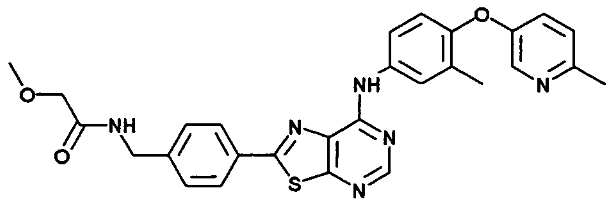

2-methoxy-N-{4-[7-({3-methyl-4-[(6-methylpyridin-3-yl)oxy]phenyl}amino)[1,3]thiazolo[5,4-d]pyrimidin-2-yl]benzyl}acetamide

The compound produced in Reference Example 17 (1.00g) was dissolved in acetonitrile (50ml), sodium azide (850mg) was added thereto, and the mixture was stirred at 70°C for 5 hours. A saturated aqueous sodium hydrogencarbonate was added thereto under an ice cooling condition, and the mixture was extracted with ethyl acetate. The extract was dried and concentrated. The residue was dissolved in dichloromethane (30ml), m-chloroperbenzoic acid (817mg) was added thereto, and the mixture was stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (16ml), and 3-methyl-4-[(6-methylpyridin-3-yl)oxy]aniline (970mg) was added thereto. The reaction solution was stirred at 120°C for 3 hours, a saturated aqueous sodium hydrogencarbonate was added thereto under an ice cooling condition, and the mixture was extracted with ethyl acetate. The extract was dried and concentrated, and the residue was purified with column chromatography (silica gel, developing solvent : ethyl acetate), and a pale yellow powder (1.02g) was obtained. The compound (0.10g) was dissolved in tetrahydrofuran (5ml), water (1ml) and triphenylphosphine (160mg) were added thereto, and the mixture was stirred for 24 hours. To the reaction solution was added a saturated aqueous sodium hydrogencarbonate, and the mixture was extracted with ethyl acetate. The extract was dried and concentrated. The residue was purified with column chromatography (basic silica gel, developing solvent : ethyl acetate), and a yellow powder (0.078g) was obtained. The compound (0.020g) was dissolved in tetrahydrofuran (2ml), triethylamine (0.060g) and methoxyacetyl chloride (0.014ml) were added under an ice cooling condition thereto, and the mixture was stirred for 10 minutes. To the reaction solution was added a saturated aqueous sodium hydrogencarbonate, and the mixture was extracted with ethyl acetate. The extract was dried and concentrated and then the residue was purified with column chromatography (basic silica gel, developing solvent : ethyl acetate) to obtain the title compound (0.020g).

$^1$H-NMR(DMSO-$d_6$) δ:2.22(3H,s),2.44(3H,s),3.35(3H,s), 3.90(2H,s),4.40(2H,d,J=6.4Hz),6.96-6.99(1H,m),7.20-7.26(2H,m),7.48-7.50(2H,m),7.76-7.79(1H,m),7.87(1H,s),8.11-8.19(3H,m),8.49-8.56(2H,m), 9.95(1H,s).

**Example 57**

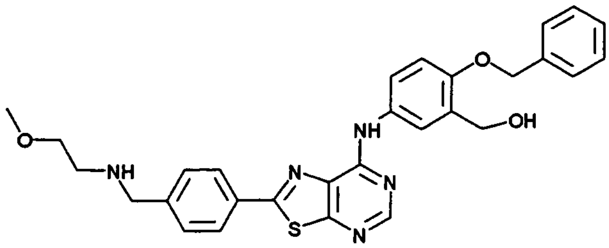

(2-(benzyloxy)-5-{[2-(4-{[(2-methoxyethyl)amino]methyl}phenyl)[1,3]thiazolo[5,4-d]pyrimidin-7-yl]amino}phenyl)methanol

In the same manner as shown in Example 26, the title compound (0.041g) was obtained from the compound produced in Reference Example 19 (0.10g) and [5-amino-2-(benzyloxy)phenyl]methanol (0.10g).

$^1$H-NMR($CDCl_3$) δ:2.84(2H,t,J=5.1Hz),3.38(3H,s),3.55 (2H,t,J=5.1Hz),3.91(2H,s),4.80(2H,s),5.16(2H,s),7.01(1H,d,J=8.5Hz),7.36-7.52(8H,m),7.69(1H,brs),7.75-7.78(1H,m),7.92 (1H,brs),8.02(2H,m),8.56(1H,s).

**Example 58**

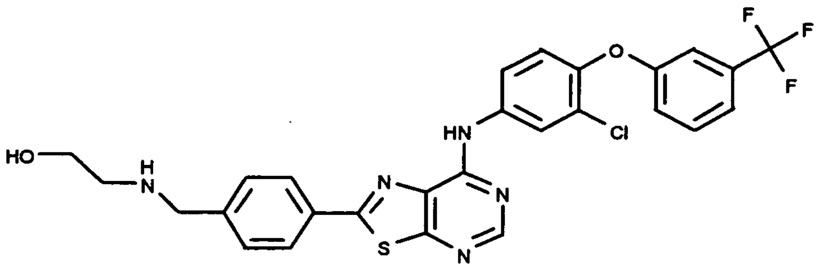

2-({4-[7-({3-chloro-4-[3-(trifluoromethyl)phenoxy]phenyl}amino)[1,3]thiazolo[5,4-d]pyrimidin-2-yl]benzyl}amino)ethanol

In the same manner as shown in Example 27, the title compound (0.14g) was obtained from the compound produced in Reference Example 18 (0.20g) and 3-chloro-4-[3-(trifluoromethyl)phenoxy]aniline (0.24g).
$^1$H-NMR ($CDCl_3$) δ:2.86 (2H,t,J=5.2Hz), 3.71 (2H,t,J=5.2Hz), 3.92 (2H,s), 7.10-7.21 (3H,m), 7.33-7.52 (4H,m), 7.73-7.77 (1H,m), 8.03-8.06 (3H,m), 8.20-8.21 (1H,m), 8.65 (1H,s).

**Example 59**

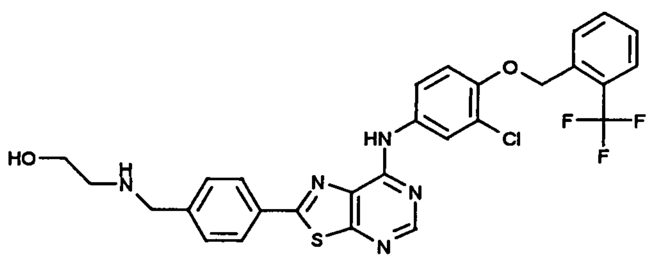

2-[(4-{7-[(3-chloro-4-{[2-(trifluoromethyl)benzyl]oxy}phenyl)amino][1,3]thiazolo[5,4-d]pyrimidin-2-yl}benzyl)amino]ethanol

In the same manner as shown in Example 27, the title compound (0.017g) was obtained from the compound produced in Reference Example 18 (0.27g) and 3-chloro-4-{[2-(trifluoromethyl)benzyl]oxy}aniline (0.32g).
$^1$H-NMR($CDCl_3$) δ:2.83-2.87(2H,m),3.67-3.72(2H,m),3.89-3.91(2H,m),4.25(1H,s),5.37(2H,s),6.98(2H,m),7.42-8.08 (11H,m), 8.58(1H,s).

**Example 60**

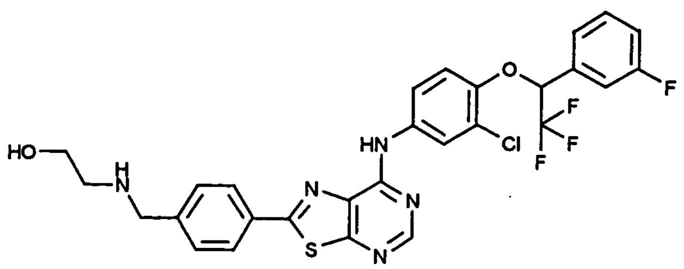

2-({4-[7-({3-chloro-4-[2,2,2-trifluoro-1-(3-fluorophenyl)ethoxy]phenyl}amino)[1,3]thiazolo[5,4-d]pyrimidin-2-yl]benzyl}amino)ethanol

In the same manner as shown in Example 27, the title compound (0.16g) was obtained from the compound produced in Reference Example 18 (0.29g) and 3-chloro-4-[2,2,2-trifluoro-1-(3-fluorophenyl)ethoxy]aniline (0.36g). $^1$H-NMR (DMSO-$d_6$) δ:2.58-2.62 (2H,m), 3.49 (2H,brs), 3.80 (2H,brs), 4.50 (1H,brs), 6.47 (1H,m), 7.19-8.14 (11H,m), 8.53 (1H,s), 10.02 (1H,s).

## Example 61

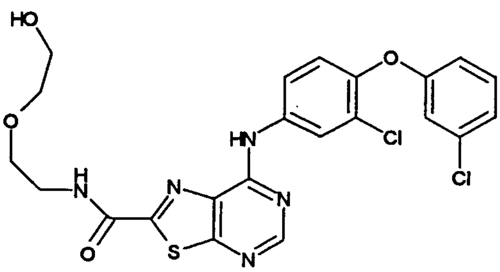

7-{[3-chloro-4-(3-chlorophenoxy)phenyl]amino}-N-[2-(2-hydroxyethoxy)ethyl][1,3]thiazolo[5,4-d]pyrimidine-2-carboxamide

In the same manner as shown in Example 1, the title compound (0.19g) was obtained from the compound produced in Reference Example 30 (0.25g) and 3-chloro-4-(3-chlorophenoxy)aniline (0.25g).

$^1$H-NMR($CDCl_3$) δ:2.25(1H,m), 3.69-3.76(6H,m),3.85-3.89(2H,m), 6.86-6.95(2H,m),7.06-7.13(2H,m), 7.23-7.29(1H,m), 7.73-7.77(2H,m), 8.17-8.22(2H,m), 8.71(1H,s).

## Example 62

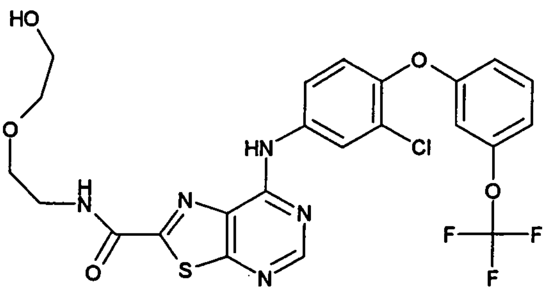

7-({3-chloro-4-[3-(trifluoromethoxy)phenoxy]phenyl}amino)-N-[2-(2-hydroxyethoxy)ethyl][1,3]thiazolo[5,4-d]pyrimidine-2-carboxamide

In the same manner as shown in Example 1, the title compound (0.024g) was obtained from the compound produced in Reference Example 30 (0.32g) and 3-chloro-4-[3-(trifluoromethoxy)phenoxy]aniline (0.36g).

$^1$H-NMR ($CDCl_3$) δ:2.46 (1H,brs), 3.69-3.76 (6H,m), 3.85 (2H,brs), 6.57-6.82 (3H,m), 7.14 (1H,d,J=8.8Hz), 7.20-7.32 (1H,m), 7.73-7.77(2H,m), 8.17-8.22 (2H,m), 8.71 (1H, s).

**Example 63**

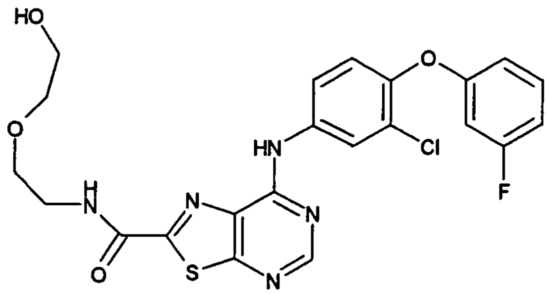

7-{[3-chloro-4-(3-fluorophenoxy)phenyl]amino}-N-[2-(2-hydroxyethoxy)ethyl][1,3]thiazolo[5,4-d]pyrimidine-2-carboxamide

In the same manner as shown in Example 1, the title compound (0.020g) was obtained from the compound produced in Reference Example 30 (0.32g) and 3-chloro-4-(3-fluorophenoxy)aniline (0.36g).

$^1$H-NMR ($CDCl_3$) δ:2.27(1H,brs), 3.69-3.76(6H,m),3.87(2H,brs), 6.57-6.90(3H,m),7.14(1H,d,J=8.8Hz), 7.31-7.36(1H, m), 7.73-7.78(2H,m), 8.19-8.24(2H,m), 8.71(1H,s).

**Example 64**

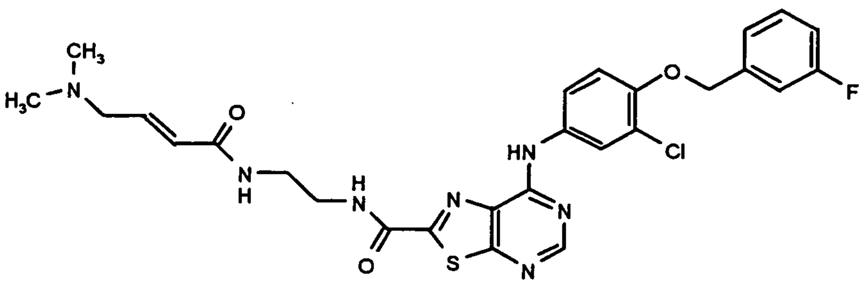

7-({3-chloro-4-[(3-fluorobenzyl)oxy]phenyl}amino)-N-(2-{(2E)-4-(dimethylamino)but-2-enoyl]amino}ethyl)[1,3]thiazolo[5,4-d]pyrimidine-2-carboxamide

The compound produced in Reference Example 31 (0.16g) was dissolved in dichloromethane (5ml), 1H-1,2,3-benzotriazol-1-ol (0.10g), N-[3-(dimethylamino)propyl]-N'-ethylcarbodiimide hydrochloride (0.35g), (2E)-4-(dimethylamino)but-2-enoic acid (0.22g) and triethylamine (0.25ml) were added thereto, and the mixture was stirred at room temperature for 12 hours. A saturated aqueous sodium hydrogencarbonate was added thereto under an ice cooling condition, and the mixture was extracted with dichloromethane. The extract was dried and concentrated and then the residue was purified with column chromatography (silica gel, developing solvent : ethyl acetate) to obtain the title compound (0.070g).

$^1$H-NMR(DMSO-$d_6$) δ:2.12(6H,s), 2.96-2.98(2H,m), 3.33-3.48(4H,m),5.26(2H,s),6.02(1H,d,J=5.5Hz), 6.52-6.61(1H,m), 7.16-7.51(5H,m),7.73-7.76(1H,m),8.09(1H,s), 8.22-8.24(1H,m), 8.63-8.68(2H,m), 10.03(1H,s).

**Example 65**

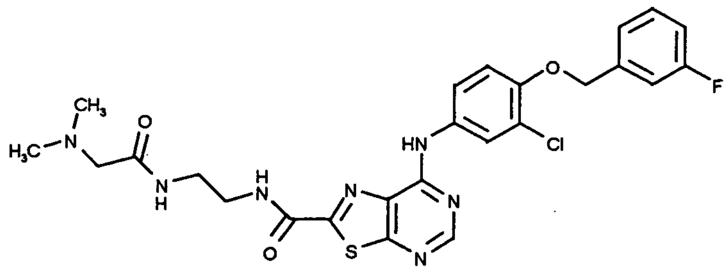

7-({3-chloro-4-[(3-fluorobenzyl)oxy]phenyl}amino)-N-{2-[(N,N-dimethylglycyl)amino]ethyl}[1,3]thiazolo[5,4-d]pyrimidine-2-carboxamide

In the same manner as shown in Example 64, the title compound (0.061g) was obtained from the compound produced in Reference Example 31 (0.069g) and N,N-dimethylglycine (0.12g).

$^1$H-NMR($CDCl_3$) δ:2.29(6H,s), 2.98(2H,s), 3.62-3.69(4H,m), 5.17 (2H,s), 6.98-7.26(4H,m),7.23-7.41(1H,m), 7.61-7.67 (2H, m), 7.98 (1H, d, J=2.6Hz), 8.12-8.24 (2H, m), 8.64 (1H, s).

**Example 66**

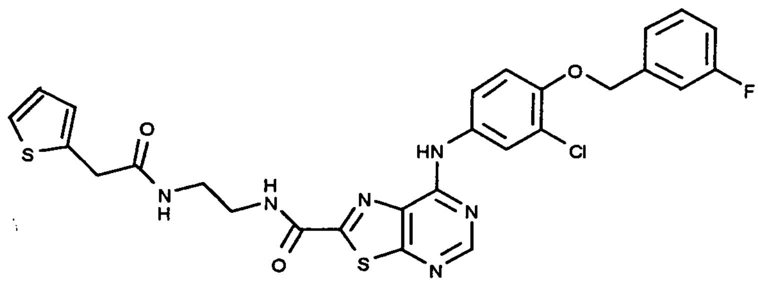

7-({3-chloro-4-[(3-fluorobenzyl)oxy]phenyl}amino)-N-{2-[(2-thienylacetyl)amino]ethyl}[1,3]thiazolo[5,4-d]pyrimidine-2-carboxamide

The compound produced in Reference Example 31 (0.060g) and thiophene-2-carbonyl chloride (0.02g) were dissolved in dichloromethane (4ml), triethylamine (0.10ml) was added thereto, and the mixture was stirred under an ice cooling condition for 1 hour. A saturated aqueous sodium hydrogencarbonate was added thereto under an ice cooling condition, and the mixture was extracted with dichloromethane. The extract was dried and concentrated and then the residue was purified with column chromatography (silica gel, developing solvent : ethyl acetate) to obtain the title compound (0.058g).

$^1$H-NMR(DMSO-$d_6$) δ:3.31-3.47(4H,m), 3.64(2H,s),5.26(2H,s), 6.89(2H,s),7.16-7.51(6H,m),7.72-7.76(1H,m), 8.09 (1H, s), 8.29(1H,brs), 8.63(2H,s), 10.00(1H,s).

**Example 67**

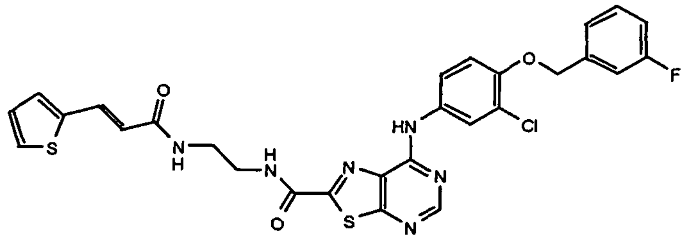

7-({3-chloro-4-[(3-fluorobenzyl)oxy]phenyl}amino)-N-(2-{[(2E)-3-(2-thienyl)prop-2-enoyl]amino}ethyl)[1,3]thiazolo[5,4-d]pyrimidine-2-carboxamide

In the same manner as shown in Example 64, the title compound (0.041g) was obtained from the compound produced in Reference Example 31 (0.12g) and (2E)-3-(2-thienyl)acrylic acid (0.15g).
$^1$H-NMR(DMSO-$d_6$) δ:3.34-3.49(4H,m),5.25(2H,s),6.36(1H, d,J=15.5Hz),7.09-7.76(10H,m),8.09(1H,s),8.33(1H,brs), 8.63 (2H,s), 10.02 (1H,brs) .

**Example 68**

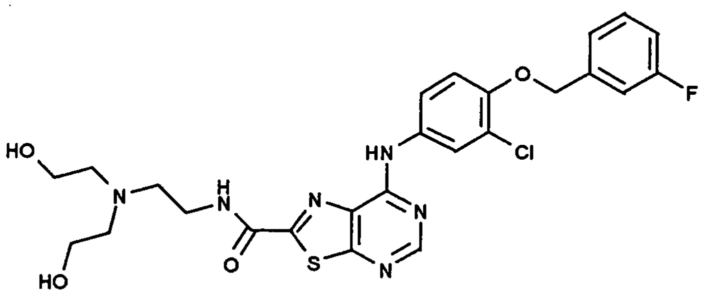

N-{2-[bis(2-hydroxyethyl)amino]ethyl}-7-({3-chloro-4-[(3-fluorobenzyl)oxy]phenyl}amino)[1,3]thiazolo[5,4-d]pyrimidine-2-carboxamide

In the same manner as shown in Example 36, the title compound (0.067g) was obtained from the compound produced in Reference Example 31 (0.090g) and 2-iodoethanol (1.5ml).
$^1$H-NMR($CDCl_3$) δ:2.83-2.91(6H,m),3.53-3.58(2H,m),3.75-3.79(4H,m),5.16(2H,s),6.91-7.40(5H,m),7.74-7.78(1H,m), 8.07 (1H,s),8.33(1H,brs),8.62(1H,s),9.07(1H,s).

**Example 69**

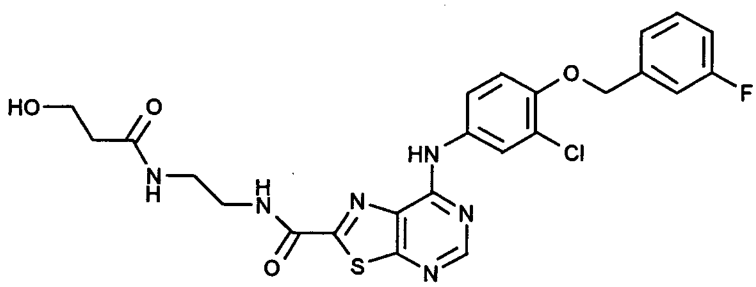

7-({3-chloro-4-[(3-fluorobenzyl)oxy]phenyl}amino)-N-{2-[(3-hydroxypropanoyl)amino]ethyl}[1,3]thiazolo[5,4-d]pyrimidine-2-carboxamide

In the same manner as shown in Example 64, the title compound (0.059g) was obtained from the compound produced in Reference Example 31 (0.12g) and 3-hydroxypropanoic acid (0.20g) .

$^1$H-NMR($CDCl_3$) δ:2.48-2.52(2H,m),3.62-3.69(4H,m),3.91-3.94(2H,m),5.16(2H,s),6.44(1H,brs),6.97-7.38(5H,m), 7.64-7.67(1H,m),7.94(2H,brs),8.37(1H,s),8.63(1H,s).

**Example 70**

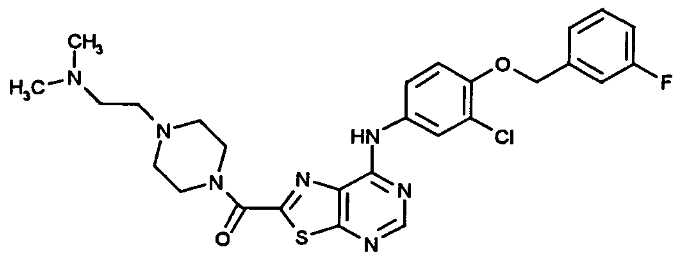

N-{3-chloro-4-[(3-fluorobenzyl)oxy]phenyl}-2-({4-[2-(dimethylamino)ethyl]piperazin-1-yl}carbonyl)[1,3]thiazolo[5,4-d] pyrimidin-7-amine

The compound produced in Reference Example 32 (0.12g) was dissolved in N,N-dimethylformamide (7ml), 2-chloro-N,N-dimethylethanamine hydrochloride (0.26g) and sodium iodide (0.01g) were added thereto, and the mixture was stirred for 1 hour. A saturated aqueous sodium hydrogencarbonate was added thereto, and the mixture was extracted with ethyl acetate. The extract was dried and concentrated, and then the residue was purified with column chromatography (basic silica gel, developing solvent : ethyl acetate/n-hexane=1/3) to obtain the title compound (0.033g).

$^1$H-NMR ($CDCl_3$) δ:2.27(6H,s), 2.47-2.62 (8H,m), 3.88 (2H,brs),4.32(2H,brs),5.18(2H,s),6.98-7.66(7H,m),7.78(1H,s), 8.65(1H,s).

**Example 71**

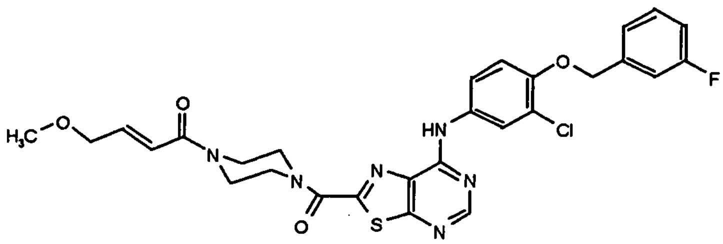

N-{3-chloro-4-[(3-fluorobenzyl)oxy]phenyl}-2-({4-[(2E)-4-methoxybut-2-enoyl]piperazin-1-yl}carbonyl)[1,3]thiazolo[5,4-d]pyrimidin-7-amine

In the same manner as shown in Example 64, the title compound (0.064g) was obtained from the compound produced in Reference Example 32 (0.22g) and (2E)-4-methoxybut-2-enoic acid (0.13g).

$^1$H-NMR($CDCl_3$) δ:3.43(3H,s),3.70-3.95(6H,m),4.12-4.14(2H,m), 4.38(2H,brs),5.18(2H,brs),6.53-6.59(1H,m),6.91-7.08 (4H,m), 7.23-7.41(2H,m),7.55-7.70(2H,m),7.90(1H,m),8.66 (1H, s).

**Example 72**

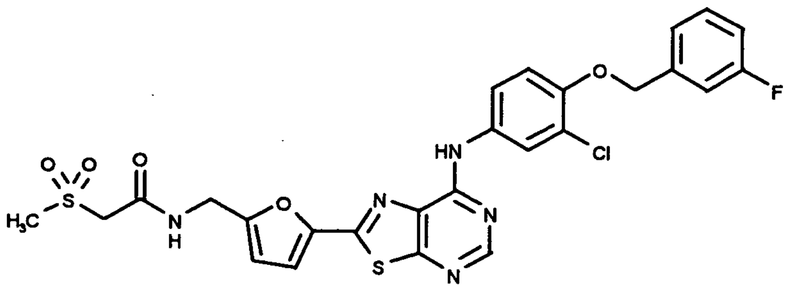

N-({5-[7-({3-chloro-4-[(3-fluorobenzyl)oxy]phenyl}amino)[1,3]thiazolo[5,4-d]pyrimidin-2-yl]-2-furyl}methyl)-2-(methylsulfonyl)acetamide

A mixture of the compound produced in Reference Example 42 (0.108g), 1-hydroxy-1H-benzotriazole monohydrate (0.0412g), 1-ethyl-3-(3-dimethylaminopropyl)-carboximide (0.108g), methanesulfonylacetic acid (0.0619g), N, N-dimethylacetamide (20ml) and tetrahydrofuran (2ml) was stirred at room temperature for 6 hours. A saturated aqueous sodium hydrogencarbonate was added thereto, and the mixture was extracted with ethyl acetate. The organic layer was dried over anhydrous magnesium sulfate, and concentrated under reduced pressure. The residue was purified with column chromatography (basic silica gel, developing solvent : n-hexane/ethyl acetate=1/1 →ethyl acetate), and further purified with HPLC, and the obtained solid was recrystallized from ethyl acetate to obtain the title compound (0.0281g).
$^1$H-NMR (DMSO-$d_6$) δ:3.14 (3H,s), 4.17 (2H,s), 4.48 (2H,d, J=5.4Hz), 6.63 (1H,d,J=3.6Hz), 7.18 (1H,m), 7.23 (1H,d, J=8.7Hz), 7. 28-7.34 (3H,m), 7.46 (1H,m), 7.76 (1H,dd,J=8.7,2.1Hz), 8.07 (1H,d,J=2.1Hz), 7.46 (1H,m), 7.76 (1H,dd, J=8.7,2.1Hz), 8.07 (1H,d, J=2.1Hz), 8.50(1H,s), 9.02 (1H,brt,J=5.4Hz), 10.11 (1H,brs).

**Example 73**

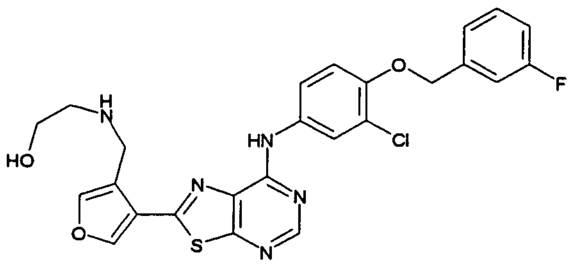

2-[({4-[7-({3-chloro-4-[(3-fluorobenzyl)oxy]phenyl}amino)[1,3]thiazolo[5,4-d]pyrimidin-2-yl]-3-furyl}methyl)amino]ethanol

The compound produced in Reference Example 34 (0.15g) was dissolved in dichloromethane (5ml), m-chloroperbenzoic acid (0.085g) was added thereto under an ice cooling condition, and stirred at room temperature for 10 minutes. The organic layer was washed by adding a saturated aqueous sodium hydrogencarbonate under an ice cooling condition, dried and concentrated. The residue was dissolved in 1-methyl-2-pyrrolidone (4ml), and 3-chloro-4-[(3-fluorobenzyl)oxy]aniline (0.15g) was added thereto. The reaction solution was stirred at 100°C for 3 hours, a saturated aqueous sodium hydrogencarbonate was added thereto under an ice cooling condition, and the mixture was extracted with ethyl acetate. The extract was dried and concentrated. The residue was purified with column chromatography (silica gel, developing solvent : ethyl acetate), the obtained compound was dissolved in trifluoroacetic acid (3ml), and the mixture was stirred at room temperature for 10 minutes. The solvent was distilled off, and the residue was dissolved in triethylamine (1ml), and purified partially with column chromatography (basic silica gel, developing solvent : ethyl acetate). The residue was dissolved in dichloromethane (2ml), 2-iodoethanol (0.80ml) was added thereto and the mixture was stirred for 25 hours. The solvent was distilled off, and the residue was purified with column chromatography (basic silica gel, developing solvent : methanol/ethyl acetate=1/10) to obtain the title compound (0.013g).
$^1$H-NMR($CDCl_3$) δ:3.15(2H,brs),3.73(2H,brs),4.53(2H,brs), 5.28 (2H, s),5.51(1H,brs), 7.16-8.12(8H,m),8.57(2H,brs), 8.81(1H,s)9.53(1H,s).

**Example 74**

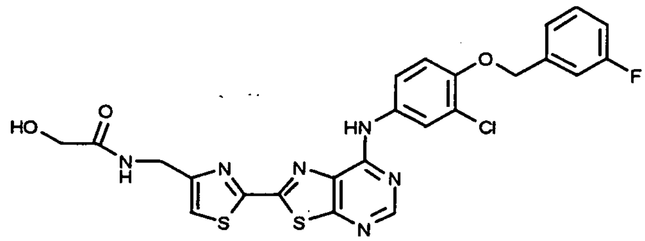

N-({2-[7-({3-chloro-4-[(3-fluorobenzyl)oxy]phenyl}amino)[1,3]thiazolo[5,4-d]pyrimidin-2-yl]-1,3-thiazol-4-yl}methyl)-2-hydroxyacetamide

The compound produced in Reference Example 7 (0.10g) was dissolved in tetrahydrofuran (10ml) and water (2ml), triphenylphosphine (1.1g) was added thereto, and the mixture was stirred at room temperature for 25 hours. To the reaction solution was added a 4N-HCl ethyl acetate solvent (10ml), and the precipitated powder was collected by filtration, and then dissolved in a 1N aqueous sodium hydroxide solution (10ml) and tetrahydrofuran (50ml). Ethyl acetate was added thereto, and the organic layer dried and concentrated. The residue was dissolved in dichloromethane (5ml), 1H-1,2,3-benzotriazol-1-ol (0.05g), N-[3-(dimethylamino)propyl]-N'-ethylcarbodiimide hydrochloride (0.24g) and hydroxyacetic acid (0.020g) were added thereto, and then the mixture was stirred at room temperature for 4 hours. A saturated aqueous sodium hydrogencarbonate was added thereto under an ice cooling condition, and the mixture was extracted with dichloromethane. The extract was dried and concentrated and the residue was purified with column chromatography (silica gel, developing solvent : methanol/ethyl acetate=1/10) to obtain the title compound (0.034g).
$^1$H-NMR($CDCl_3$) δ:4.22(2H,brs),4.69-4.71(2H,m),5.18 (2H,s),6.99-7.45(7H,m),7.63-7.66(1H,m),7.86(1H,brs),7.97 (1H, brs),8.63(1H,s).

**Example 75**

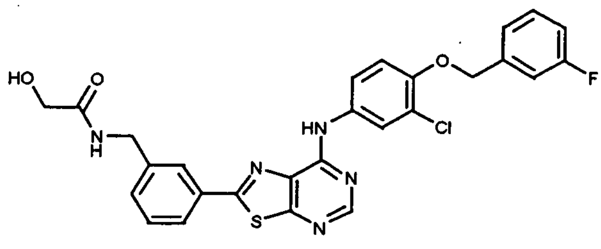

N-{3-[7-({3-chloro-4-[(3-fluorobenzyl)oxy]phenyl}amino)[1,3]thiazolo[5,4-d]pyrimidin-2-yl]benzyl}-2-hydroxyacetamide

The compound obtained by the same manner as shown in Example 1 from the compound produced in Reference Example 7 (1.00g) and 3-chloro-4-[(3-fluorobenzyl)oxy]aniline (0.80g) was dissolved in tetrahydrofuran (10ml) and water (2ml), triphenylphosphine (18.4g) was added thereto, and the mixture was stirred at room temperature for 25 hours. To the reaction solution was added a 4N-HCl ethyl acetate solvent (10ml) and the precipitated powder was collected by filtration, and dissolved in a 1N aqueous sodium hydroxide solution (10ml) and tetrahydrofuran (10ml). Ethyl acetate was added thereto, and the organic layer dried and concentrated. The residue (0.10g) was dissolved in dichloromethane (5ml), 1H-1,2,3-benzotriazol-1-ol (0.05g), N-[3-(dimethylamino)propyl]-N'-ethylcarbodiimide hydrochloride (0.25g) and hydroxyacetic acid (0.020g) were added thereto, and then the mixture was stirred at room temperature for 12 hours. A saturated aqueous sodium hydrogencarbonate was added thereto under an ice cooling condition, and the mixture was extracted with dichloromethane. The extract was dried and concentrated and the residue was purified with column chromatography (silica gel, developing solvent : ethyl acetate) to obtain the title compound (0.057g).
$^1$H-NMR($CDCl_3$) δ:4.24(2H,s),4.58(2H,d,J=6.OHz),5.14(2H,s), 6.95-7.09(3H,m),7.23-7.47(5H,m),7.60-7.65(1H,m),7.83-7.86(1H,m),7.98-8.01(3H,m),8.56(1H,s).

**Example 76**

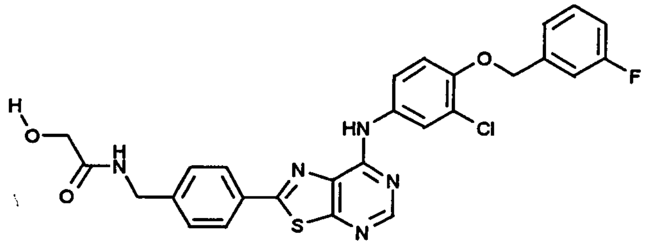

N-{4-[7-({3-chloro-4-[(3-fluorobenzyl)oxy]phenyl}amino)[1,3]thiazolo[5,4-d]pyrimidin-2-yl]benzyl}-2-hydroxyacetamide

In the same manner as shown in Example 75, the title compound (0.061g) was obtained from the compound produced in Reference Example 29 (0.22g), 3-chloro-4-[(3-fluorobenzyl) oxy]aniline (0.20g) and hydroxyacetic acid (0.087g).

$^1$H-NMR ($CDCl_3$) δ:4.24(2H,d,J=5.1Hz),4.60(2H,d,J=6.1Hz), 5.17(2H,s),6.91(1H,brs),6.98-7.06(2H,m),7.21-7.47(5H, m), 7.63-7.65(1H,m),7.89(1H,s), 7.97-8.05(3H,m),8.59(1H,s).

**Example 77**

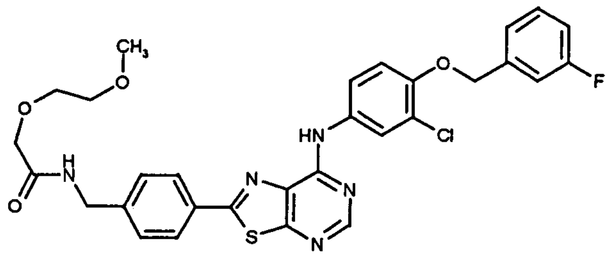

N-{4-[7-({3-chloro-4-[(3-fluorobenzyl)oxy]phenyl}amino)[1,3]thiazolo[5,4-d]pyrimidin-2-yl]benzyl}-2-(2-methoxyethoxy) acetamide

The compound obtained by the same manner as shown in Example 1 from the compound produced in Reference Example 29 (1.00g) and 3-chloro-4-[(3-fluorobenzyl)oxy]aniline (0.80g) was dissolved in tetrahydrofuran (10ml) and water (2ml), triphenylphosphine (18.4g) was added thereto, and the mixture was stirred at room temperature for 25 hours. To the reaction solution was added a 4N-HCl ethyl acetate solvent (10ml) and the precipitated powder was collected by filtration, and dissolved in a 1N aqueous sodium hydroxide solution (10ml) and tetrahydrofuran (10ml). Ethyl acetate was added thereto, and the organic layer dried and concentrated. The residue (0.10g) was dissolved in dichloromethane (5ml), and triethylamine (0.1ml) was added thereto. Under an ice cooling condition, (2-methoxyethoxy)acetyl chloride (0.10ml) was added thereto, and the mixture was stirred for 10 minutes. To the reaction solution was added a 5% aqueous sodium hydrogencarbonate, and the mixture was extracted with ethyl acetate. The extract was dried and concentrated and the residue was purified with column chromatography (silica gel, developing solvent : ethyl acetate/n-hexane=1/2 - methanol/ethyl acetate=2/98) to obtain the title compound (0.017g).

$^1$H-NMR (DMSO-$d_6$) δ:3.25(3H,s),3.49-3.52(2H,m),3.62-3.64(2H,m), 3.98(2H,s), 4.41-4.42(2H,m), 5.26(2H,s),7.16-7.50 (7H,m), 7.79-7.82(1H,m),8.11-8.13(3H,m), 8.35-8.40(1H,m), 8.54(1H,s), 10.00 (1H, s) .

**Example 78**

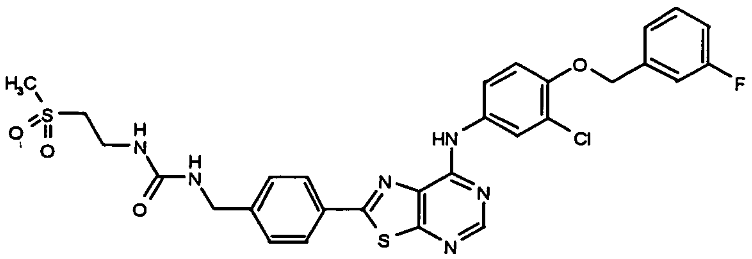

N-{4-[7-({3-chloro-4-[(3-fluorobenzyl)oxy]phenyl}amino)[1,3]thiazolo[5,4-d]pyrimidin-2-yl]benzyl}-N'-[2-(methylsulfonyl)ethyl]urea

The compound obtained by the same manner as shown in Example 1 from the compound produced in Reference Example 29 (1.00g) and 3-chloro-4-[(3-fluorobenzyl)oxy]aniline (0.80g) was dissolved in tetrahydrofuran (10ml) and water (2ml), triphenylphosphine (18.4g) was added thereto, and the mixture was stirred at room temperature for 25 hours. To the reaction solution was added a 4N-HCl ethyl acetate solvent (10ml) and the precipitated powder was collected by filtration, and dissolved in a 1N aqueous sodium hydroxide solution (10ml) and tetrahydrofuran (10ml). Ethyl acetate was added thereto, and the organic layer dried and concentrated. The residue (0.050g) was dissolved in a mixed solvent of tetrahydrofuran (3ml) and N,N-dimethylacetamide (2ml), 4-dimethylaminopyridine (0.0020g) and 1,1'-carbonylbis(1H-imidazole) (0.018g) were added thereto, and then the mixture was stirred for 1 hour. 2-(Methylsulfonyl)ethylamine was added thereto, and the mixture was stirred at 60°C for 30 minutes. To the reaction solution was added a 5% aqueous sodium hydrogencarbonate, and the mixture was extracted with ethyl acetate. The extract was dried and concentrated and the residue was purified with column chromatography (silica gel, developing solvent: methanol/ethyl acetate=2/98) to obtain the title compound (0.029g).

$^1$H-NMR(DMSO-$d_6$) δ:3.00(3H,s),3.23-3.28(2H,m),3.45-3.50(2H,m), 4.30-4.32(2H,m),5.26(2H,s),6.27-6.30(1H,m), 6.78-6.81(1H,m), 7.16-7.21(1H,m),7.25-7.48(6H,m),7.79-7.81(1H,m), 8.10-8.12 (3H,m),8.54(1H,s),9.99(1H,s).

**Example 79**

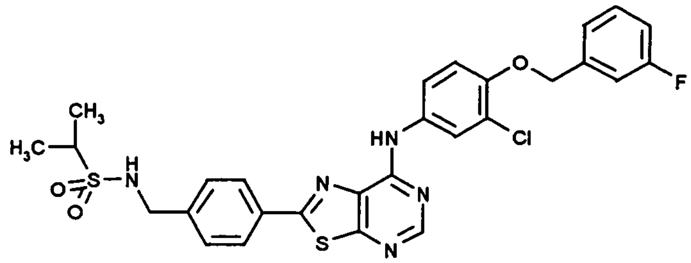

N-{4-[7-({3-chloro-4-[(3-fluorobenzyl)oxy]phenyl}amino)[1,3]thiazolo[5,4-d]pyrimidin-2-yl]benzyl}propane-2-sulfonamide

In the same manner as shown in Example 75, the title compound (0.054g) was obtained from the compound produced in Reference Example 29 (0.10g), 3-chloro-4-[(3-fluorobenzyl)oxy]aniline (0.10g) and propane-2-sulfonyl chloride (0.10ml).

$^1$H-NMR(DMSO-$d_6$) δ:1.23(6H,d,J=6.8Hz),2.95-2.99(1H,m), 4.25-4.28(2H,m),5.26(2H,s),7.17-7.82(9H,m),8.11-8.16 (3H,m), 8.55(1H,s),10.00(1H,s) .

**Example 80**

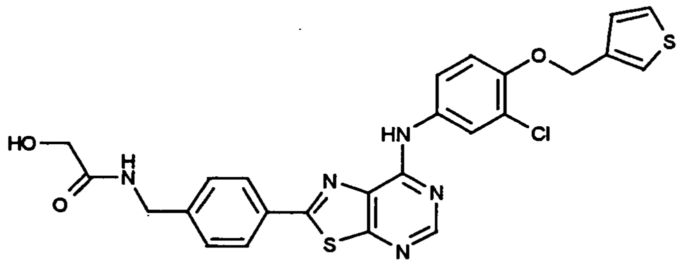

N-[4-(7-{[3-chloro-4-(3-thienylmethoxy)phenyl]amino}[1,3]thiazolo[5,-d]pyrimidin-2-yl)benzyl]-2-hydroxyacetamide

In the same manner as shown in Example 75, the title compound (0.11g) was obtained from the compound produced in Reference Example 29 (0.34g), 3-chloro-4-(3-thienylmethoxy) aniline (0.38g) and hydroxyacetic acid (0.34g).
$^1$H-NMR(DMSO-$d_6$) δ:3.90(2H,d,J=5.8Hz),4.40(2H,d,J=6.2Hz), 5.21(2H,s),5.58(1H,t,J=5.8Hz),7.21-7.31(2H,m), 7.48-7.61(4H,m),7.79-7.82(1H,m),8.09-8.12(3H,m),8.42-8.46 (1H,m),8.54(1H,s),9.99(1H,s).

**Example 81**

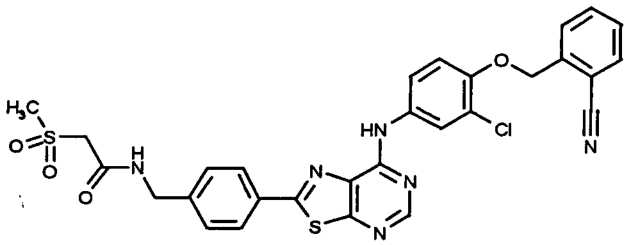

N-{4-[7-({3-chloro-4-[(2-cyanobenzyl)oxy]phenyl}amino)[1,3]thiazolo[5,4-d]pyrimidin-2-yl]benzyl}-2-(methylsulfonyl) acetamide

In the same manner as shown in Example 75, the title compound (0.025g) was obtained from the compound produced in Reference Example 29 (0.12g), 2-[(4-amino-2-chlorophenoxy) methyl]benzonitrile (0.11g) and (methylsulfonyl)acetic acid (0.22g).
$^1$H-NMR(DMSO-$d_6$) δ:3.15(3H,s),4.18(2H,s),4.45(2H,d,J=5.8Hz), 5.36(2H,s),7.34-8.16(11H,m),8.55(1H,s),8.99(1H, brs), 10.02 (1H, s).

**Preparation Example 1** (Dosage per a tablet)

| | | |
|---|---|---|
| (1) | Compound obtained in Example 57 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0mg of the compound obtained in Example 57, 60.0mg of lactose and 35.0mg of corn starch is granulated through a 1 mm-mesh sieve using 0.03ml of a 10% by weight aqueous solution of gelatin (3.0mg as gelatin), and then dried at 40°C and sieved. The obtained granules are mixed with 2.0mg of magnesium stearate and compressed. The obtained core tablets are sugar-coated with a suspension of sucrose, titanium dioxide, talc and gum Arabic. The thus-coated tablets are glazed with bees wax to obtain sugar-coated tablets.

**Preparation Example 2** (Dosage per a tablet)

| | | |
|---|---|---|
| (1) | Compound obtained in Example 57 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0mg of the compound obtained in Example 57 and 3.0mg of magnesium stearate are granulated with 0.07ml of an aqueous soluble starch solution (7.0mg as soluble starch), dried, and mixed with 70.0mg of lactose and 50.0mg of corn starch. The mixture is compressed to obtain tablets.

**Test Example 1A** Cloning of Human HER-2 Gene and Preparation of recombinant baculovirus

Cloning of Human HER2 Gene was performed with RT-PCR templating total RNA prepared from MCF 7 cell. To attach Flag tag in N-terminal of HER2, a primer used in RT-PCR was manufactured by adding flag peptide coding sequence and restricted enzyme recognition sequence to the base sequence (2176-3918 in Genbank Accession M11730) which codes a part of intracellular domain of the protein using an information of base sequence of HER2 Gene (Genbank Accession M11730). Base sequence of the primer was shown as below.
HER2-U:
5'-AATTAAGTCGACATGGACTACAAAGACGATGACGACAAGCGACGGCAGCAGAAGATCCGG AAGTAC-3' (base sequence: 1) and
HER2-L:
5'-AATTAAGCATGCTCACACTGGCACGTCCAGACCCAGGTACTC-3' (base sequence: 2)

RT reaction was performed using a SuperScript First-Strand Synthesis system for RT-PCR (Invitrogen), and PCR reaction was performed using a KOD-plus kit (TOYOBO). The obtained PCR product was subjected to electrophoresis on agarose gel (1%), and digested with Sal I and Sph I after collecting a DNA fragment amplified by PCR from the gel. The DNA treated with restricted enzymes was subjected to electrophoresis on agarose gel (1%), the obtained DNA fragment was collected, and ligated to a plasmid pFASTBAC1 (Invitrogen) digested with restricted enzymes Sal I and Sph I, resulting that expression plasmid pFB-HER2 was manufactured. Base sequence of inserted fragment was confirmed. As a result, the base sequence of inserted fragment was corresponded with base sequence of the intracellular domain of the HER2 (pp. 2176-3918 in Genbank Accession M11730). Further, a recombinant baculovirus BAC-HER2 was prepared using BAC-TO-BAC Baculovirus Expression System (Invitrogen).

**Test Example 1B** Preparation of a intracellular domain protein of HER2

A SF-21 cell was plated with $1\times10^6$ cells/ml in a Sf-900II SFM medium (Invitrogen) containing 10% of fetal bovine serum (Trace), 50mg/L of Gentamicin (Invitrogen) and 0.1% of Pluronic F-68 (Invitrogen), and cultured with shaking at 27°C at 100rpm using a 2L meyer. After culturing for 24 hours, 13.4ml of a recombinant baculovirus BAC-HER2 was added thereto, and further cultured for 3 days. The cultured solution was centrifuged at 2,000rpm for 5 minutes, and a virus-infected cell was obtained. The cell was washed with phosphate buffered saline (Invitrogen), centrifuged under the same condition, and preserved at -80°C. The freezing preserved cell was melted on ice, suspended with 30ml of a buffer A (50mM of tris-buffer containing 20% of glycerol and 0.15M of NaCl (pH 7.4)) to which was added Complete Protease Inhibitor (Behringer), and crushed at 20,000rpm for 30 seconds three times using Polytron homogenizer (Kinematica). The crushed solution was clarified by centrifugation at 40,000rpm for 30 minutes, and filtered using a 45μm filter. The filtrate was passed through a column filled with Anti-FLAG M2 Affinity Gel (Sigma Corp.) in a flow rate of about 0.5mL/min. The column was washed with a buffer A, and eluted with a buffer A containing 100μg/mL of FLAG peptide. The eluted solution was concentrated by Vivaspin 20 of MWCO (molecular weight of cut-off) 30K (Vivascience). This concentrated solution was gel-filtrated and purified by a Hi Load Superdex 200pg 16/60 (Amersham Biosciences) equilibrated with the buffer A. The fraction containing the intracellular domain of HER2 was collected, and glycerol was added thereto to be a 50% of terminal concentration, and then freezing preserved at -80°C. **Test Example 1C** Measurement of inhibition activity of HER2 Kinase

The test compound dissolved in dimethylsulfoxide (DMSO) was diluted with buffer for measuring the kinase reaction (50mM of Tris-HCl (pH 7.5), 5mM of $MgCl_2$, 5mM $MnCl_2$, 2mM of dithiothreitol, 0.01% of Tween-20). To the solution of test compound (10μl) was added 20 μl of buffer containing 5μg/mL of the intracellular domain of HER2 obtained in Test Example 1B and 12.5μg/mL of the polypeptide substrate (poly-Glu:Tyr(4:1), Sigma Corp.), and added 20μl of ATP solution (1.25μM of ATP, 0.05μCi [$\gamma$-$^{32}$P] ATP), and the reaction was initiated at 25°C for 10 minutes. Then,

the reaction was terminated by adding 50μl of a 20% TCA solution. The reacting solution was subjected at 4°C for 20 minutes, the acid-insoluble fraction was transferred to GF/C filter (PerkinElmer) using a cell harvester (PerkinElmer), and washed with 250mM of a phosphate buffer solution. After washing, the plate was dried at 45°C for 60 minutes, and added 35μl of a microscinti 0 (PerkinElmer) thereto, and then radioactivity of the test compound was measured using a Topcount (PerkinElmer). The inhibition rate (%) of the test compound to the HER2 kinase was determined by the formula represented below.

Inhibition rate (%) = (1-(count of the test compound - blank)/(control-blank)]× 100

wherein, the "control" was defined as the count of the reacted solution without adding the test compound, the "blank" was defined as the count of the solution without adding the test compound and the intracellular domain of HER2. The result of the inhibition rate of the test compound was shown in Table 1.

The result indicated that the compound of the present invention inhibits excellently the activity of HER2 kinase.

[Table 1]

| No. of Example (No. of the compound) | Inhibition rate (%) in 1.0 μM |
|---|---|
| 26 | 92 |
| 39 | 96 |
| 41 | 89 |
| 58 | 90 |
| 80 | 87 |

**Test Example 2A** Cloning of Human EGF Receptor Gene and Preparation of recombinant baculovirus

Cloning of Human EGF Receptor Gene was performed with RT-PCR templating total RNA prepared from A431 cell. To attach Flag tag in N-terminal of EGF, a primer used in RT-PCR was manufactured by adding flag peptide coding sequence and restricted enzyme recognition sequence to the base sequence (2182-3810 in Genbank Accession XM_ 167493) which codes a part of intracellular domain of the protein using an information of base sequence of EGF receptor Gene (Genbank Accession XM_167493). The Base sequence of the primer was shown as below.
EGFR-U:
5'-AATTAAGTCGACATGGACTACAAAGACGATGACGACCGAAGGCGCCACATCGTTCGGAAG CGCACG-3' (base sequence: 3) and
EGFR-L:
5'-AATTAAGCATGCTCATGCTCCAATAAATTCACTGCTTTGTGG-3' (base sequence: 4)

RT reaction was performed using a SuperScript First-Strand Synthesis system for RT-PCR (Invitrogen), and PCR reaction was performed using a KOD-plus kit (TOYOBO). The obtained PCR product was subjected to electrophoresis on agarose gel (1%), and digested with Sal I and Sph I after collecting a DNA fragment amplified by PCR from the gel. The DNA treated with restricted enzymes was subjected to electrophoresis on agarose gel (1%), the obtained DNA fragment was collected, and ligated to a plasmid pFASTBAC1 (Invitrogen) digested with restricted enzymes Sal I and Sph I, and expression plasmid pFB-HER2 was manufactured. Base sequence of inserted fragment was confirmed. As a result, the base sequence of inserted fragment was corresponded with base sequence of the intracellular domain of EGFR (pp. 2182-3810 in Genbank Accession XM_167493). Further, a recombinant baculovirus BAC-EGFR was prepared using BAC-TO-BAC Baculovirus Expression System (Invitrogen).

**Test Example 2B** Preparation of a intracellular domain protein of EGF Receptor

A SF-21 cell was plated with $1\times10^6$ cells/ml in a Sf-900II SFM medium (Invitrogen) containing 10% of a fetal bovine serum (Trace), 50mg/L of Gentamicin (Invitrogen) and 0.1% of Pluronic F-68 (Invitrogen), and cultured with shaking at 27°C at 100rpm using a 2L meyer. After culturing for 24 hours, 13.4mL of a recombinant baculovirus EGFR was added thereto, and further cultured for 3 days. The cultured solution was centrifuged in 2,000rpm for 5 minutes, and a virus-infected cell was obtained. The cell was washed with phosphate buffered saline (Invitrogen), centrifuged under the same condition, and then preserved at -80°C. The freezing preserved cell was melted on ice, suspended with

30mL of a buffer A (50mM of tris-buffer containing 20% of glycerol and 0.15M of NaCl (pH 7.4)) to which was added Complete Protease Inhibitor (Behringer), and crushed at 20,000rpm for 30 seconds three times using Polytron homogenizer (Kinematica). The crushed solution was clarified by centrifugation at 40,000rpm for 30 minutes, and filtered using a 45μm filter. The filtrate was passed through a column which is filled with Anti-FLAG M2 Affinity Gel (Sigma Corp.) in a flow rate of about 0.5mL/min. The column was washed with a buffer A, and eluted with a buffer containing 100μg/ml of FLAG peptide. The eluted solution was concentrated by Vivaspin 20 of MWCO (molecular weight of cut-off) 30K (Vivascience). This concentrated solution was gel-filtrated and purified by NAP™ 25 Column (Amersham Biosciences) equilibrated with the buffer A. The fraction containing the intracellular domain of EGF receptor was collected, and glycerol was added thereto to be a 50% of terminal concentration, and then freezing preserved at -80°C. **Test Example 2C** Measurement of inhibition activity of EGF Receptor Kinase

The test compound dissolved in dimethylsulfoxide (DMSO) was diluted with buffer for measuring the kinase reaction (50mM of Tris-HCl (pH 7.5) , 5mM of $MgCl_2$, 5mM $MnCl_2$, 2mM of dithiothreitol, 0.01% of Tween-20). To the solution of test compound (5μl) was added 10 μl of buffer containing 250ng/ml of the intracellular domain protein of EGFR receptor and 250ng/ml of the Biotin-recognition polypeptide (biotinyl-poly-Glu:Tyr (4:1), CIS bio International). To the obtained mixture was added 10μl of buffer containing 5μM of ATP, and the mixture was reacted at 25°C for 10 minutes. Then, the reaction was terminated by adding 25μl of a stop solution (100mM of EDTA·2sodium salt, 62.5mM of HEPES buffer (pH 7.4), 250mM of NaCl, 0.1% of a bovine serum albumin, 10μg/ml of streptavidine donor beads for α-screen assay, 10 μg/ml of anti-phosphotyrosin phosphorylation recognition antibody PY-100 conjugated acceptor beads for α-screen assay (Anti-phosphotyrisine(P-Tyr-100) Acceptor beads: PerkinElmer). The reacting solution was subjected at 25°C for 16 hours, and the count was measured by using plate reader· Fusion™ (PerkinElmer). The inhibition rate of the kinase in the test compound was determined by the formula represented below.

Inhibition rate (%) = (1-(count of the test compound - blank)/(control-blank)]× 100

wherein, the "control" was defined as the count of the reacted solution without adding the test compound, the "blank" was defined as the count of the solution without adding the test compound and ATP. The result of the inhibition rate of the test compound was shown in Table 2.

The result indicated that the compound of the present invention inhibits excellently the activity of EGFR kinase.

[Table 2]

| No. of Example (No. of the compound) | Inhibition rate (%) in 1.0 μM |
|---|---|
| 20 | 91 |
| 25 | 88 |
| 46 | 81 |
| 52 | 81 |

**Test Example 3** Antiproliferative activity of breast cancer cell BT-474 in vitro

100μl of a cell suspension of human breast cancer cell BT-474 (6,000 cells) was plated in 96 well microplate, and cultured at 37°C in 5% carbon dioxide gas incubator. The next day, 100μl of each test compound solution which was diluted 2 times was added thereto, and the culture of cell was continued for 5 days. After the culture solution containing the test compound was expected and the cell was washed, the cell was fixed with 50% trichloroacetic acid solution, and then dye SRB 0.4% (W/V) solution (dissolved in 1% acetic acid) was added thereto, result that the cell protein was fixed and dyed (Skehan et al., Journal of the National Cancer Institute, 82 Ed., pp. 1107-1112, 1990). After washing with 1% acetic acid solution, 100μl of the extract (10mM of tris-buffer solution) was added thereto and the dye was extracted, and the absorbance at absorption wavelength of 550nm was measured, and then the amount of cell was measured as the amount of protein. When 100% was defined as the protein amount of the control group without adding the test compound solution, the percentage of the remaining protein amount of each treating group was determined, and then the $IC_{50}$ values, which is a concentration of the test compound to inhibit the amount of the remaining cell so as to be 50% of that of the control, was calculated.

## Industrial Applicability

The present invention provides a thiazolo[5,4-d]pyrimidine derivative, a process for producing the same, and use thereof. The thiazolo[5,4-d]pyrimidine derivative has tyrosine kinase inhibitory action, and toxicity thereof is low, further it is useful as a pharmacological product.

The present application is based on patent application No. 2004-109393 (filing date: 2004-04-01) filed in Japan. Accordingly the whole of their contents is encompassed in the application.

## Claims

**1.** A compound represented by Formula:

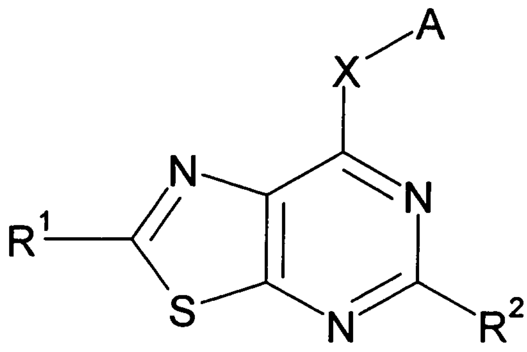

(I)

wherein A is an aryl group or a heteroaryl group, each of which may be substituted; $R^1$ is a group which is bonded through carbon; $R^2$ is a hydrogen atom or an aliphatic hydrocarbon group; and X is $-NR^3-$, -O-, -S-, -SO-, $-SO_2-$, or $-CHR^3-$ (wherein $R^3$ is a hydrogen atom or an aliphatic hydrocarbon group),
or a salt thereof (provided that 2-methyl-N-phenyl[1,3]thiazolo[5,4-d]pyrimidin-7-amine, N-(4-fluorophenyl)-2-methyl [1,3]thiazolo[5,4-d]pyrimidin-7-amine, 2,5-dimethyl-N-phenyl[1,3]thiazolo[5,4-d]pyrimidin-7-amine, N-(4-chlorophenyl)-2,5-dimethyl[1,3]thiazolo[5,4-d]pyrimidin-7-amine, N-(4-fluorophenyl)-2,5-dimethyl[1,3]thiazolo[5,4-d]pyrimidin-7-amine, 2,5-dimethyl-N-(4-methylphenyl)[1,3]thiazolo[5,4-d]pyrimidin-7-amine, 2,5-dimethyl-N-[3-(trifluoromethyl)phenyl][1,3]thiazolo[5,4-d]pyrimidin-7-amine, N-(3,4-dimethylphenyl)-2,5-dimethyl[1,3]thiazolo[5,4-d]pyrimidin-7-amine, N-(3,5-dimethylphenyl)-2,5-dimethyl[1,3]thiazolo[5,4-d]pyrimidin-7-amine, and N-(2,6-dimethylphenyl)-2,5-dimethyl[1,3]thiazolo[5,4-d]pyrimidin-7-amine are excluded).

**2.** A prodrug of the compound according to Claim 1.

**3.** The compound according to Claim 1, wherein A is an aryl group which is substituted with a group represented by Formula: -Y-B (wherein, Y is a single bond or a spacer, and B is an aryl group or a heterocyclic group, each of which may be substituted), and which may be further substituted.

**4.** The compound according to Claim 1, wherein $R^1$ is a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, or a heterocyclyl-$C_{1-4}$ alkyl group, each of which may be substituted.

**5.** The compound according to Claim 1, wherein $R^2$ is a hydrogen atom.

**6.** The compound according to Claim 1, wherein A is an aryl group which is substituted with a group represented by Formula: -Y-B (wherein, Y is a single bond or a spacer, and B is an aryl group or a heterocyclic group, each of which may be substituted), and which may be further substituted; $R^1$ is a $C_{1-8}$ alkyl group, a $C_{2-8}$ alkenyl group, a $C_{2-8}$ alkynyl group, a carbamoyl group, a $C_{3-8}$ cycloalkyl group, a $C_{4-12}$ bridged cyclic hydrocarbon group, a $C_{6-18}$ aryl group, a $C_{6-18}$ aryl-$C_{1-4}$ alkyl group, a heterocyclic group, or a heterocyclyl-$C_{1-4}$ alkyl group, each of which may be substituted; and $R^2$ is a hydrogen atom.

**7.** The compound according to Claim 1, wherein A is:

(i) an aryl group which is substituted with a group having an aromatic ring, or
(ii) a heteroaryl group which may be substituted.

**8.** A compound represented by Formula:

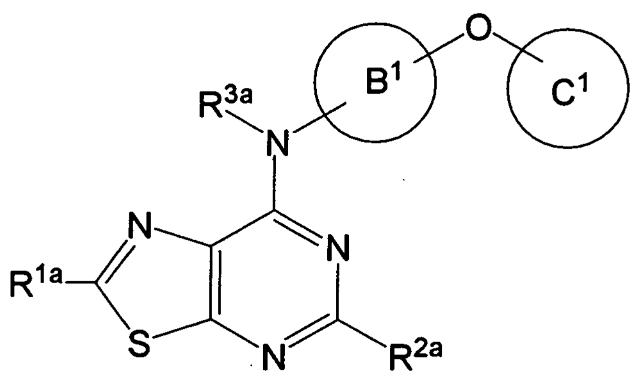

(I-I)

wherein $B^1$ is a $C_{6-18}$ aryl ring which may be substituted; $C^1$ is a $C_{6-18}$ aryl group which may be substituted; $R^{1a}$ is a group which is bonded through carbon; $R^{2a}$ is a hydrogen atom or an aliphatic hydrocarbon group; and $R^{3a}$ is a hydrogen atom or an aliphatic hydrocarbon group,
or a salt thereof.

**9.** A compound represented by Formula:

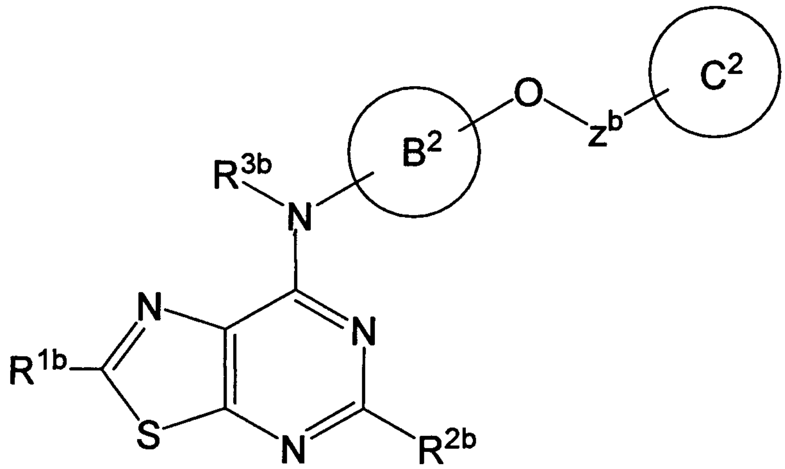

(I-II)

wherein $B^2$ is a $C_{6-18}$ aryl ring which may be substituted; $C^2$ is a $C_{6-18}$ aryl group which may be substituted; $R^{1b}$ is a group which is bonded through carbon; $R^{2b}$ is a hydrogen atom or an aliphatic hydrocarbon group; $R^{3b}$ is a hydrogen atom or an aliphatic hydrocarbon group; and $Z^b$ is a $C_{1-4}$ alkylene group which may be substituted,
or a salt thereof.

**10.** A compound represented by Formula:

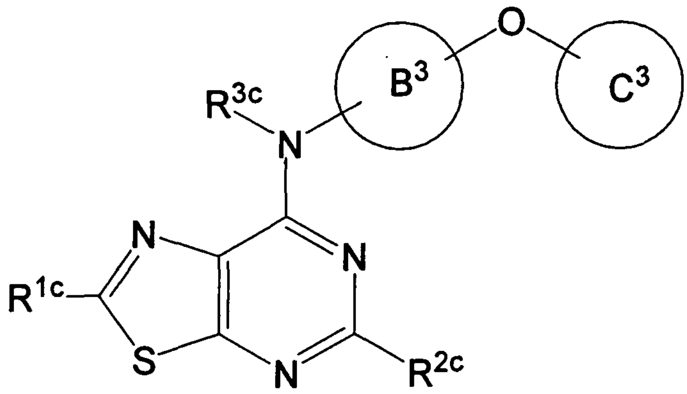

(I-III)

wherein $B^3$ is a $C_{6-18}$ aryl ring which may be substituted; $C^3$ is a heterocyclic group which may be substituted; $R^{1c}$ is a group which is bonded through carbon; $R^{2c}$ is a hydrogen atom or an aliphatic hydrocarbon group; and $R^{3c}$ is a hydrogen atom or an aliphatic hydrocarbon group,
or a salt thereof.

**11.** A compound represented by Formula:

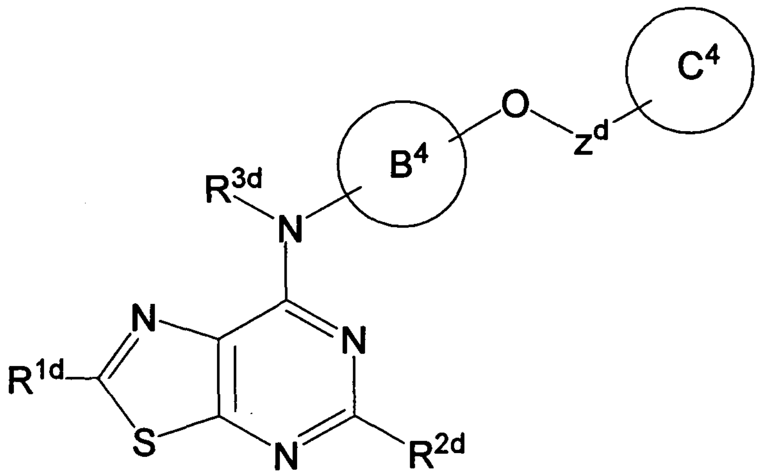

(I-IV)

wherein $B^4$ is a $C_{6-18}$ aryl ring which may be substituted; $C^4$ is a heterocyclic group which may be substituted; $R^{1d}$ is a group which is bonded through carbon; $R^{2d}$ is a hydrogen atom or an aliphatic hydrocarbon group; $R^{3d}$ is a hydrogen atom or an aliphatic hydrocarbon group; and $Z^d$ is a $C_{1-4}$ alkylene group which may be substituted,
or a salt thereof.

**12.** The compound according to Claim 1, wherein $R^1$ is a $C_{6-18}$ aryl group which may be substituted, or a $C_{6-18}$ aryl-$C_{1-4}$ alkyl group which may be substituted.

**13.** The compound according to Claim 1, wherein
A is a $C_{6-18}$ aryl group which may be substituted with 1 to 5 substituents selected from the group consisting of:

(i) a $C_{6-18}$ aryl-oxy group which may be substituted with substituent(s) selected from the group consisting of:

(a) a halogen atom,
(b) $C_{1-4}$ alkyl which may be halogenated, and
(c) $C_{1-4}$ alkyloxy which may be halogenated,

(ii) a $C_{6-18}$ aryl-$C_{1-4}$ alkyl-oxy group (said $C_{1-4}$ alkyl may be substituted with $C_{1-4}$ alkyl which may be halogenated) which may be substituted with substituent(s) selected from the group consisting of:

(a) a halogen atom,
(b) $C_{1-4}$ alkyl which may be halogenated, and
(c) cyano,

(iii) a 5- to 8-membered heterocyclyl-oxy group having 1 to 3 heteroatoms selected from nitrogen, oxygen and sulfur atoms, which may be substituted with $C_{1-4}$ alkyl,
(iv) a 5- to 8- membered heterocyclyl-$C_{1-3}$ alkyl-oxy group having 1 to 3 heteroatoms selected from nitrogen, oxygen and sulfur atoms,
(v) a $C_{6-8}$ aryl-$C_{1-4}$ alkyl group,
(vi) a halogen atom,
(vii) a $C_{1-4}$ alkyl group which may be substituted with substituent(s) selected from the group consisting of:

(a) a halogen atom,
(b) hydroxy, and
(c) $C_{1-4}$ alkyloxy, and

(viii) a $C_{1-4}$ alkyloxy group,

$R^1$ is :

(i) a $C_{1-8}$ alkyl group which may be substituted with substituent(s) selected from the group consisting of:

(a) hydroxy,
(b) $-NR^8-(CH_2)_n-O-C_{1-4}$ alkyl,
(C) $-NR^8-CO-C_{1-4}$ alkyl, and
(d) $-NR^8-CO-(CH_2)_n-O-C_{1-4}$ alkyl,

wherein n is an integer from 1 to 4, and $R^8$ is a hydrogen atom or a $C_{1-4}$ alkyl group,
(ii) a $C_{3-8}$ cycloalkyl group,
(iii) a $C_{6-18}$ aryl group which may be substituted with substituent(s) selected from the group consisting of:

(a) a halogen atom,
(b) nitro,
(c) $-NR^6R^7$,
(d) $-O-C_{1-4}$ alkyl,
(e) $-(CH_2)_m-NR^8-(CH_2)_n-OH$,
(f) $-(CH_2)_m-NR^8-(CH_2)_n-O-C_{1-4}$ alkyl,
(g) $-(CH_2)_m-NR^8-(CH_2)_n-SO_2-C_{1-4}$ alkyl,
(h) $-(CH_2)_m-NR^8-(CH_2)_n-NR^8-CO-C_{1-4}$ alkyl,
(i) $-(CH_2)_m-O-(CH_2)_n-O-C_{1-4}$ alkyl,
(J) $-(CH_2)_m-NR^8-CO-(CH_2)_n-OH$,
(k) $-(CH_2)_m-NR^8-CO-(CH_2)_n-O-C_{1-4}$ alkyl,
(1) $-(CH_2)_m-NR^8-CO-(CH_2)_n-SO_2-C_{1-4}$ alkyl,
(m) $-(CH_2)_m-NR^8-CO-NR^8-(CH_2)_n-SO_2-C_{1-4}$alkyl,
(n) $-(CH_2)_m-NR^8-SO_2-C_{1-4}$ alkyl,
(o) $-(CH_2)_m-NR^8-CO-(CH_2)_n-O-(CH_2)_n-O-C_{1-4}$ alkyl,
(p) $-(CH_2)_m-NR^8-CO-(CH_2)_n-NR^6R^7$, and
(q) a 5- to 8- membered heterocyclyl-$C_{1-4}$ alkyl group having 1 to 3 heteroatoms selected from nitrogen, oxygen and sulfur atoms, which may be substituted with $-(CH_2)_n-OH$,

wherein m is an integer from 0 to 4, n is an integer from 1 to 4, when m or n is two or more, a part of $-CH_2-CH_2-$ moieties thereof may be replaced by -CH=CH-; $R^6$ and $R^7$ are identical or different and are each a hydrogen atom or a $C_{1-4}$ alkyl group; and $R^8$ is a hydrogen atom, a $C_{1-4}$ alkyl group, or a $C_{1-4}$ alkyl-carbonyl group,
(iv) a $C_{6-18}$ aryl-$C_{1-4}$ alkyl group which may be substituted with substituent(s) selected from the group consisting of:

(a) nitro, and
(b) -O-$C_{1-4}$ alkyl,

(v) a carbamoyl group which may be substituted with substituent(s) selected from the group consisting of:

(a) - $(CH_2)_n$-OH,
(b) -$(CH_2)_n$-$NR^6R^7$,
(c) -$(CH_2)_n$-O-$C_{1-4}$ alkyl,
(d) -$(CH_2)_m$-O-$(CH_2)_n$-OH,
(d) -$(CH_2)_n$-$NR^8$-$(CH_2)_n$-$SO_2$-$C_{1-4}$ alkyl,
(e) -$(CH_2)_n$-$NR^8$-CO-$(CH_2)_n$-OH,
(f) -$(CH_2)_n$-$NR^8$-CO-$(CH_2)_n$-$NR^6R^7$, and
(g) -$(CH_2)_n$-$NR^8$-CO-$(CH_2)_n$-heterocyclic group,

wherein m is an integer from 0 to 4, n is an integer from 1 to 4, and when m or n is two or more, a part of -$CH_2$-$CH_2$- moieties thereof may be replaced by -CH=CH-; $R^6$ and $R^7$ are identical or different and are each a hydrogen atom or a $C_{1-4}$ alkyl group which may have hydroxy; and $R^8$ is a hydrogen atom or a $C_{1-4}$ alkyl group,
(vi) a 5- to 8- membered cyclic amino-carbonyl group which may be substituted with substituent(s) selected from the group consisting of:

(a) hydroxy,
(b)-$(CH_2)_n$-$NR^6R^7$, and
(c) -CO- $(CH_2)_n$-O- $C_{1-4}$ alkyl,

wherein n is an integer from 1 to 4, and when n is two or more, a part of -$CH_2$-$CH_2$- moieties thereof may be replaced by -CH=CH-; and $R^6$ and
$R^7$ are identical or different and are each a hydrogen atom or a $C_{1-4}$ alkyl group,
(vii) a 5- to 8- membered heterocyclic group having 1 to 3 heteroatoms selected from nitrogen, oxygen and sulfur atoms, which may be substituted with substituent(s) selected from the group consisting of:

(a) -$(CH_2)_m$-$NR^8$-CO- $(CH_2)_n$-OH, and
(b) -$(CH_2)_m$-$NR^8$-CO-$(CH_2)_n$-$SO_2$-$C_{1-4}$ alkyl,

wherein m is an integer from 0 to 4, n is an integer from 1 to 4, and $R^8$ is a hydrogen atom or a $C_{1-4}$ alkyl group, or
(viii) a 5- to 8- membered heterocyclyl-$C_{1-4}$ alkyl group having 1 to 3 heteroatoms selected from nitrogen, oxygen and sulfur atoms, which may be substituted with hydroxy,

$R^2$ is a hydrogen atom,
X is -$NR^3$-, and
$R^3$ is a hydrogen atom or a $C_{1-3}$ alkyl group.

**14.** The compound according to Claim 8, wherein
$B^1$ is a benzene ring which may be substituted with halogen atom(s),
$C^1$ is a $C_{6-18}$ aryl group which may be substituted with substituent(s) selected from the group consisting of:

(a) a halogen atom,
(b) $C_{1-4}$ alkyl which may be halogenated, and
(c) $C_{1-4}$ alkyloxy which may be halogenated,

$R^{1a}$ is:

(i) a $C_{6-18}$ aryl group which may be substituted with -$(CH_2)_m$-$NR^8$-$(CH_2)_n$-OH [wherein m is an integer from 0 to 4, n is an integer from 1 to 4, and $R^8$ is a hydrogen atom or $C_{1-4}$ alkyl], or
(ii) a carbamoyl group which may be substituted with -$(CH_2)_m$-O-$(CH_2)_n$-OH [wherein m is an integer from 0 to 4, and n is an integer from 1 to 4],

$R^{2a}$ is a hydrogen atom, and

$R^{3a}$ is a hydrogen atom.

**15.** The compound according to Claim 9, wherein
$B^2$ is a benzene ring which may be substituted with substituent(s) selected from the group consisting of:

(a) a halogen atom,
(b) $C_{1-4}$ alkyl which may be substituted with hydroxy or $C_{1-4}$ alkyloxy, and
(c) $C_{1-4}$ alkyloxy,

$C^2$ is a $C_{6-18}$ aryl group which may be substituted with substituent(s) selected from the group consisting of:

(a) a halogen atom,
(b) $C_{1-4}$ alkyl which may be halogenated, and
(c) cyano,

$R^{1b}$ is:

(i) a $C_{1-8}$ alkyl group which may be substituted with substituent(s) selected from the group consisting of:

(a) $-NR^8-CO-C_{1-4}$ alkyl, and
(b) $-NR^8-CO-(CH_2)_n-O-C_{1-4}$ alkyl,

wherein n is an integer from 1 to 4, and $R^8$ is a hydrogen atom or a $C_{1-4}$ alkyl group,
(ii) a $C_{3-8}$ cycloalkyl group,
(iii) a $C_{6-18}$ aryl group which may be substituted with substituent(s) selected from the group consisting of:

(a) a halogen atom,
(b) $-O-C_{1-4}$ alkyl,
(c) $-(CH_2)_m-NR^8-(CH_2)_n-OH$,
(d) $-(CH_2)_m-NR^8-(CH_2)_n-O-C_{1-4}$ alkyl,
(e) $-(CH_2)_m-NR^8-(CH_2)_n-SO_2-C_{1-4}$ alkyl,
(f) $-(CH_2)_m-NR^8-(CH_2)_n-NR^8-CO-C_{1-4}$ alkyl,
(g) $-(CH_2)_m-O-(CH_2)_n-O-C_{1-4}$ alkyl,
(h) $-(CH_2)_m-NR^8-CO-(CH_2)_n-OH$,
(i) $-(CH_2)_m-NR^8-CO-(CH_2)_n-O-C_{1-4}$ alkyl,
(j) $-(CH_2)_m-NR^8-CO-(CH_2)_n-SO_2-C_{1-4}$ alkyl,
(k) $-(CH_2)_m-NR^8-CO-NR^8-(CH_2)_n-SO_2-C_{1-4}$ alkyl,
(1) $-(CH_2)_m-NR^8-SO_2-C_{1-4}$ alkyl,
(m) $-(CH_2)_m-NR^8-CO-(CH_2)_n-O-(CH_2)_n-O-C_{1-4}$ alkyl, and
(n) a 5- to 8- membered heterocyclyl-$C_{1-4}$ alkyl group having 1 to 3 heteroatoms selected from nitrogen, oxygen and sulfur atoms, which may be substituted with $-(CH_2)_n-OH$,

wherein m is an integer from 0 to 4, n is an integer from 1 to 4, and $R^8$ is a hydrogen atom or a $C_{1-4}$ alkyl group,
(iv) a $C_{6-18}$ aryl-$C_{1-4}$ alkyl group which may be substituted with $-O-C_{1-4}$ alkyl,
(v) a carbamoyl group which may be substituted with substituent(s) selected from the group consisting of:

(a) $-(CH_2)_n-OH$,
(b) $-(CH_2)_n-NR^6R^7$,
(c) $-(CH_2)_n-O-C_{1-4}$ alkyl,
(d) $-(CH_2)_n-NR^8-(CH_2)_n-SO_2-C_{1-4}$ alkyl,
(e) $-(CH_2)_n-NR^8-CO-(CH_2)_n-OH$,
(f) $-(CH_2)_n-NR^8-CO-(CH_2)_n-NR^6R^7$, and
(g) $-(CH_2)_n-NR^8-CO-(CH_2)_n$-heterocyclic group

wherein n is an integer from 1 to 4, and when n is two or more, a part of $-CH_2-CH_2-$ of moieties thereof may be replaced by $-CH=CH-$;
$R^6$ and $R^7$ are identical or different and are each a hydrogen atom or a $C_{1-4}$ alkyl group which may have hydroxy; and $R^8$ is a hydrogen atom or a $C_{1-4}$ alkyl group,

(vi) a 5- to 8- membered cyclic amino-carbonyl group which may be substituted with substituent(s) selected from the group consisting of:

(a) hydroxy,
(b) $-(CH_2)_n-NR^6R^7$, and
(c) -CO- $(CH_2)_n$-O- $C_{1-4}$ alkyl,

wherein n is an integer from 1 to 4, and when n is two or more, a part of $-CH_2-CH_2-$ moieties thereof may be replaced by -CH=CH-; and $R^6$ and $R^7$ are identical or different and are each a hydrogen atom or a $C_{1-4}$ alkyl group,
(vii) a 5- to 8- membered heterocyclic group having 1 to 3 heteroatoms selected from nitrogen, oxygen and sulfur atoms, which may be substituted with substituent(s) selected from the group consisting of:

(a) - $(CH_2)_m-NR^8-CO-(CH_2)_n-OH$,
(b) - $(CH_2)_m-NR^8-CO-(CH_2)-SO_2-C_{1-4}$ alkyl, and
(c) $-(CH_2)_m-NR^8-(CH_2)_n-OH$,

wherein m is an integer from 0 to 4, n is an integer from 1 to 4, and $R^8$ is a hydrogen atom or a $C_{1-4}$ alkyl group, or
(viii) a 5- to 8- membered heterocyclyl-$C_{1-4}$ alkyl group having 1 to 3 heteroatoms selected from nitrogen, oxygen and sulfur atoms, which may be substituted with $-(CH_2)_n-OH$ [wherein n is an integer from 1 to 4],

$R^{2b}$ is a hydrogen atom,
$R^{3b}$ is a hydrogen atom, and
$Z^b$ is a $C_{1-4}$ alkylene group which may be substituted with $C_{1-4}$ alkyl which may be halogenated.

**16.** The compound according to Claim 10, wherein
$B^3$ is a benzene ring which may be substituted with $C_{1-4}$ alkyl,
$C^3$ is a 5- to 8- membered heterocyclic group having 1 to 3 heteroatoms selected from nitrogen, oxygen and sulfur atoms, which may be substituted with $C_{1-4}$ alkyl,
$R^{1c}$ is:

(i) a $C_{1-8}$ alkyl group which may be substituted with substituent(s) selected from the group consisting of:

(a) hydroxy,
(b) $-NR^8-(CH_2)_n-O-C_{1-4}$ alkyl, and
(C) $-NR^8-CO-(CH_2)_n-O-C_{1-4}$ alkyl,

wherein n is an integer from 1 to 4, and $R^8$ is a hydrogen atom or a $C_{1-4}$ alkyl group,
(ii) a $C_{6-18}$ aryl group which may be substituted with substituent(s) selected from the group consisting of:

(a) $-O-C_{1-4}$ alkyl,
(b) $-(CH_2)_m-NR^8-(CH_2)_n-OH$,
(c) $-(CH_2)_m-NR^8-(CH_2)_n-O-C_{1-4}$ alkyl,
(d) $-(CH_2)_m-NR^8-(CH_2)_n-NR^8-CO-C_{1-4}$ alkyl,
(e) $-(CH_2)_m-NR^8-(CH_2)_n-SO_2-C_{1-4}$ alkyl,
(f) $-(CH_2)_m-NR^8-CO-(CH_2)_n-O-C_{1-4}$ alkyl,
(g) $-(CH_2)_m-NR^8-CO-(CH_2)_n-NR^6R^7$,
(h) $-NR^6R^7$, and
(i) a 5- to 8- membered heterocyclyl-$C_{1-4}$ alkyl group having 1 to 3 heteroatoms selected from nitrogen, oxygen and sulfur atoms, which may be substituted with $-(CH_2)_n-OH$,

wherein m is an integer from 0 to 4, n is an integer from 1 to 4, and when m or n is two or more, a part of $-CH_2-CH_2-$ moieties thereof may be replaced by -CH=CH-; $R^6$ and $R^7$ are identical or different and are each a hydrogen atom or a $C_{1-4}$ alkyl group; and $R^8$ is a hydrogen atom, a $C_{1-4}$ alkyl group, or a $C_{1-4}$ alkyl-carbonyl group,
(iii) a $C_{6-18}$ aryl-$C_{1-4}$ alkyl group which may be substituted with substituent(s) selected from the group consisting of:

(a) nitro, and
(b) -O-$C_{1-4}$ alkyl,

(iv) a carbamoyl group which may be substituted with $-(CH_2)_n$-O-$C_{1-4}$ alkyl [wherein n is an integer from 1 to 4],
(v) a 5- to 8- membered cyclic amino-carbonyl group which may be substituted with hydroxy, or
(vi) a 5- to 8- membered heterocyclyl-$C_{1-4}$ alkyl group having 1 to 3 heteroatoms selected from nitrogen, oxygen and sulfur atoms, which may be substituted with hydroxy,
$R^{2c}$ is a hydrogen atom, and
$R^{3c}$ is a hydrogen atom.

**17.** The compound according to Claim 11, wherein
$B^4$ is a benzene ring which may be substituted with halogen atom(s),
$C^4$ is a 5- to 8- membered heterocyclic group having 1 to 3 heteroatoms selected from nitrogen, oxygen and sulfur atoms,
$R^{1d}$ is a $C_{6-18}$ aryl group which may be substituted with - $(CH_2)_m$-$NR^8$-CO-$(CH_2)_n$-OH [wherein m is an integer from 0 to 4, n is an integer from 1 to 4, and $R^8$ is a hydrogen atom or $C_1$-$_4$ alkyl],
$R^{2d}$ is a hydrogen atom,
$R^{3d}$ is a hydrogen atom, and
$Z^d$ is a $C_{1-4}$ alkylene group.

**18.** The compound according to Claim 1, which is N-{3-chloro-4-[(3-fluorobenzyl)oxy]phenyl}-2-[3-({[2-(methylsulfonyl)ethyl]amino}methyl)phenyl][1,3]thiazolo[5,4-d]pyrimidin-7-amine,
7-({3-chloro-4-[(3-fluorobenzyl)oxy]phenyl}amino)-N-(2-hydroxyethyl)[1,3]thiazolo[5,4-d]pyrimidine-2-carboxamide, 2-({4-[7-({3-chloro-4-[(3-fluorobenzyl)oxy]phenyl}amino)[1,3]thiazolo[5,4-d]pyrimidin-2-yl]benzyl}amino)ethanol,
N-{3-chloro-4-[(3-fluorobenzyl)oxy]phenyl}-2-(4-{[(2-methoxyethyl)amino]methyl}phenyl)[1,3]thiazolo[5,4-d]pyrimidin-7-amine,
N-{3-chloro-4-[(3-fluorobenzyl)oxy]phenyl}-2-(4-{[(3-methoxypropyl)amino]methyl}phenyl)[1,3]thiazolo[5,4-d]pyrimidin-7-amine,
N-[2-({4-[7-({3-chloro-4-[(3-fluorobenzyl)oxy]phenyl}amino)[1,3]thiazolo[5,4-d]pyrimidin-2-yl]benzyl}amino)ethyl]acetamide,
N-{3-chloro-4-[(3-fluorobenzyl)oxy]phenyl}-2-[4-({[2-(methylsulfonyl)ethyl]amino}methyl)phenyl][1,3]thiazolo[5,4-d]pyrimidin-7-amine,
N-{2-[{4-[7-({3-chloro-4-[(3-fluorobenzyl)oxy]phenyl}amino)[1,3]thiazolo[5,4-d]pyrimidin-2-yl]benzyl}(methyl)amino]ethyl}acetamide,
2-methoxy-N-{4-[7-({3-methyl-4-[(6-methylpyridin-3-yl)oxy]phenyl}amino)[1,3]thiazolo[5,4-d]pyrimidin-2-yl]benzyl}acetamide,
7-({3-chloro-4-[(3-fluorobenzyl)oxy]phenyl}amino)-N-(2-{[(2E)-4-(dimethylamino)but-2-enoyl]amino}ethyl)[1,3]thiazolo[5,4-d]pyrimidine-2-carboxamide,
N-{2-[bis(2-hydroxyethyl)amino]ethyl}-7-({3-chloro-4-[(3-fluorobenzyl)oxy]phenyl}amino)[1,3]thiazolo[5,4-d]pyrimidine-2-carboxamide,
7-({3-chloro-4-[(3-fluorobenzyl)oxy]phenyl}amino)-N-{2-[(3-hydroxypropanoyl)amino]ethyl}[1,3]thiazolo[5,4-d]pyrimidine-2-carboxamide,
N-{3-chloro-4-[(3-fluorobenzyl)oxy]phenyl}-2-({4-[(2E)-4-methoxybut-2-enoyl]piperazin-1-yl}carbonyl)[1,3]thiazolo[5,4-d]pyrimidin-7-amine,
N-{3-[7-({3-chloro-4-[(3-fluorobenzyl)oxy]phenyl}amino)[1,3]thiazolo [5,4-d]pyrimidin-2-yl]benzyl}-2-hydroxyacetamide, or
N-{4-[7-({3-chloro-4-[(3-fluorobenzyl)oxy]phenyl}amino)[1,3]thiazolo[5,4-d]pyrimidin-2-yl]benzyl}-N'-[2-(methylsulfonyl)ethyl]urea,
or a salt thereof.

**19.** A process for producing the compound represented by Formula (I) or a salt thereof, comprising reacting a compound represented by Formula:

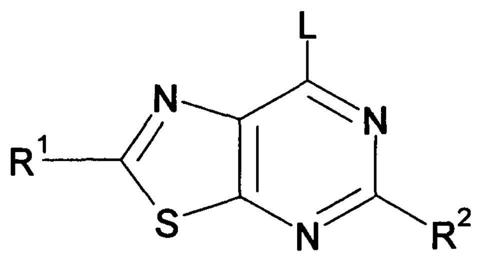

(II)

wherein L is a leaving group, and other symbols have the same meanings as defined in Claim 1, or a salt thereof, with a compound represented by Formula: G-X-A
wherein G is a hydrogen atom or a metal atom, and other symbols have the same meanings as defined in Claim 1, provided that when X is $-CHR^3-$ (the symbols in the formula have the same meanings as defined in Claim 1.), G is a metal atom, or a salt thereof.

**20.** A medicine containing a compound represented by Formula:

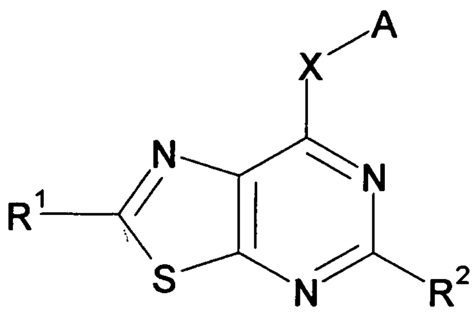

(Ia)

wherein A is aryl or heteroaryl, each of which may be substituted; $R^1$ is a group which is bonded through carbon; $R^2$ is hydrogen or an aliphatic hydrocarbon group; and X is - $NR^3$-, -O-, -S-, -SO-, $-SO_2-$, or $-CHR^3-$ (wherein $R^3$ is hydrogen or an aliphatic hydrocarbon group),
or a salt thereof or a prodrug thereof.

**21.** The medicine according to Claim 20, which is a tyrosine kinase inhibitor.

**22.** The medicine according to Claim 20, which is a prophylactic and/or therapeutic agent for cancer.

**23.** The medicine according to Claim 22, wherein the cancer is breast cancer, prostate cancer, lung cancer, pancreatic cancer, or kidney cancer.

**24.** A method of preventing and/or treating cancer by administering an effective amount of a compound represented by Formula:

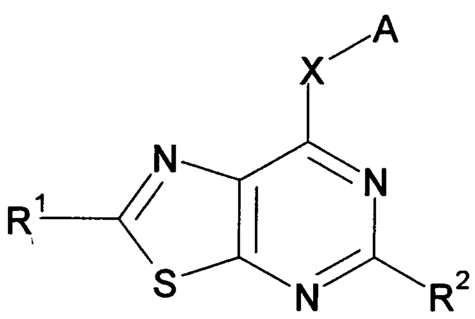

(Ia)

wherein A is aryl or heteroaryl, each of which may be substituted; $R^1$ is a group which is bonded through carbon; $R^2$ is hydrogen or an aliphatic hydrocarbon group; and X is - $NR^3$-, -O-, -S-, -SO-, $-SO_2-$, or $-CHR^3-$ (wherein $R^3$ is hydrogen or an aliphatic hydrocarbon group),
or a salt thereof or a prodrug thereof, to a mammal.

**25.** Use of a compound represented by Formula:

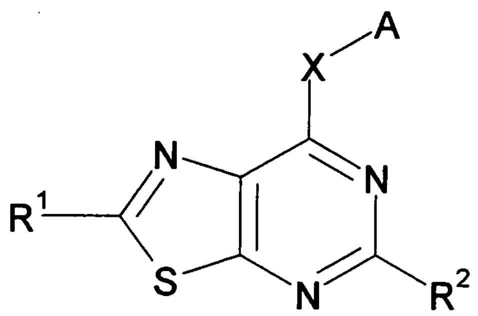

(Ia)

wherein A is aryl or heteroaryl, each of which may be substituted; $R^1$ is a group which is bonded through carbon; $R^2$ is hydrogen or an aliphatic hydrocarbon group; and X is - $NR^3$-, -O-, -S-, -SO-, $-SO_2-$, or $-CHR^3-$ (wherein $R^3$ is hydrogen or an aliphatic hydrocarbon group),
or a salt thereof, or a prodrug thereof, for the preparation of a prophylactic and/or therapeutic agent for cancer.

## INTERNATIONAL SEARCH REPORT

International application No.
PCT/JP2005/006832

A. CLASSIFICATION OF SUBJECT MATTER
Int.Cl[7] C07D513/04, A61K31/519, A61P13/08, 35/00, 43/00

According to International Patent Classification (IPC) or to both national classification and IPC

B. FIELDS SEARCHED

Minimum documentation searched (classification system followed by classification symbols)
Int.Cl[7] C07D513/04, A61K31/519, A61P13/08, 35/00, 43/00

Documentation searched other than minimum documentation to the extent that such documents are included in the fields searched

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 10-508875 A (Pfizer Inc.),<br>02 September, 1998 (02.09.98),<br>Full text; particularly, Claim 1; pages 48 to 50<br>& WO 96/40142 A1 & CZ 9601641 A3<br>& NO 9602386 A & AU 9654791 A<br>& SK 9600729 A3 & HU 9601559 A2<br>& KR 97001354 A & FI 9704443 A<br>& SG 45483 A1 & ZA 6304725 A<br>& EP 831829 A1 & NZ 286755 A<br>& TW 327636 A & BR 9602695 A<br>& MX 9709867 A1 & RU 2136683 C1<br>& CN 1141298 A & CA 2223081 C<br>& JP 03-290666 B2 & US 6395733 B1<br>& EP 831829 B1 & DE 69531558 E<br>& MX 217004 B & ES 2203642 T3 | 1,2,4,5,7,<br>13,19-23,25<br>3,6,8-12,<br>14-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 June, 2005 (27.06.05) | 12 July, 2005 (05.07.05) |
| Name and mailing address of the ISA/<br>Japanese Patent Office<br><br>Facsimile No. | Authorized officer<br><br><br>Telephone No. |

Form PCT/ISA/210 (second sheet) (January 2004)

INTERNATIONAL SEARCH REPORT

International application No.
PCT/JP2005/006832

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 2000-515136 A (Glaxo Group Ltd.),<br>14 November, 2000 (14.11.00),<br>Full text; particularly, Claim 1; table 1<br>& WO 98/02437 A1 & AU 9736936 A<br>& NO 9900123 A & ZA 9706145 A<br>& EP 912572 A1 & CZ 9900087 A3<br>& BR 9710362 A & CN 1230185 A<br>& MX 9900483 A1 & KR 2000023813 A<br>& US 6207669 B1 & EP 912572 B1<br>& DE 69718472 E & ES 2191187 T3 | 1-11,13,15,<br>19-23,25<br>12,14,16-18 |
| X<br><br>A | JP 11-513398 A (Glaxo Group Ltd.),<br>16 November, 1999 (16.11.99),<br>Full text; particularly, Claim 1; pages 19 to 20; table 1<br>& WO 97/13771 A1 & AU 9672896 A<br>& ZA 9608551 A & EP 861253 A1<br>& US 6169091 B1 | 1-11,13,15,<br>19-23,25<br>12,14,16-18 |
| Y<br><br>A | WO 2002/092603 A1 (NOVARTIS AG),<br>21 November, 2002 (21.11.02),<br>Full text; particularly, Claims 1, 5, 17; pages 7 to 8, 34 to 39<br>& CA 2449188 A & EP 1390371 A1<br>& BR 2002009645 A & JP 2005-508855 A<br>& US 2004/152892 A1 | 1-9,12-15,<br>19-23,25<br>10,11,16-18 |
| Y<br><br>A | WO 2003/053446 A1 (SMITHKLINE BEECHAM CORP.),<br>03 July, 2003 (03.07.03),<br>Full text; particularly, Claim 1; examples 81 to 102; pages 181 to 183; table 1<br>& EP 1463507 A1 & JP 2005-516023 A<br>& US 2005/009845 A1 | 1-9,12-15,<br>19-23,25<br>10,11,16-18 |
| Y<br><br>A | WO 2003/013541 A1 (NOVARTIS AG),<br>20 February, 2003 (20.02.03),<br>Full text; particularly, Claim 1; example compound; example 147<br>& CA 2453881 A & EP 1416935 A1<br>& BR 2002011801 A & JP 2005-501077 A<br>& ZA 2004000271 A & US 2004/242600 A1<br>& US 2004/248911 A1 | 1-9,12-15,<br>19-23,25<br>10,11,16-18 |
| X<br><br>A | INOUE, Shoji, Pyrimidine derivatives. VI.6, Chemical & Pharmaceutical Bulletin, 1958, Vol.6, p.352-5, full text; particularly, refer to the descriptions relating to the compound(VII) in the paragraph of "Experiments" | 1,4,5,19<br>2,3,6-18,<br>20-23,25 |

Form PCT/ISA/210 (continuation of second sheet) (January 2004)

INTERNATIONAL SEARCH REPORT

International application No.
PCT/JP2005/006832

C (Continuation). DOCUMENTS CONSIDERED TO BE RELEVANT

| Category* | Citation of document, with indication, where appropriate, of the relevant passages | Relevant to claim No. |
|---|---|---|
| A | ANDERSEN, Knud Erik et al., Diphosphorus pentoxide in organic synthesis XXVII. A one-step synthesis of thiazolo[5,4-d] pyrimidin-7-amines and purine-6-thiones from 5-(acylamino)-2-methylthiazole-4-carboxamides, Liebigs Annalen der Chemie, July, 1986, No. 7, p.1255 to 61, particularly, compound 2a to 2j | 1-23,25 |

Form PCT/ISA/210 (continuation of second sheet) (January 2004)

INTERNATIONAL SEARCH REPORT

International application No.
PCT/JP2005/006832

**Box No. II Observations where certain claims were found unsearchable (Continuation of item 2 of first sheet)**

This international search report has not been established in respect of certain claims under Article 17(2)(a) for the following reasons:

1. [x] Claims Nos.: 24
because they relate to subject matter not required to be searched by this Authority, namely:
Claim 24 pertains to methods for treatment of the human body by therapy and thus relates to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

INTERNATIONAL SEARCH REPORT

International application No.
PCT/JP2005/006832

<With respect to claims 2, 20-23, and 25>

With respect to the "prodrug" given in claims 20 and 25, examples of substituents to be incorporated for each functional group of the compound represented by the formula (Ia) are shown in pages 70-71 of the description.

However, the changeable substituents in the compound represented by the formula (Ia) given in claims 20 and 25 are defined by an expression which enables a wide range of substituent groups to be involved. It is hence difficult to grasp as to what functional groups the compound represented by the formula (Ia) has and as to what positions the functional groups are present.

Consequently, even if the substituents to be introduced for each functional group are defined in the description, the prodrug of the compound cannot be clearly grasped as to what chemical structure the compound has, because it is difficult to grasp the functional groups possessed by the compound represented by the formula (Ia).

Therefore, the subject matters of claims 20 and 25 are unclear.

Likewise, the subject matters of claims 21-23, in which claim 20 is cited, are not considered to be clear.

In claim 2 is cited the compound represented by the formula (I) of claim 1. However, in view of page 3 of the description, this compound is thought to be involved in the compound represented by the formula (Ia) of claim 20. With respect to the definition of the "prodrug" given in claim 2, the statement in the description concerning the prodrug of the compound represented by the formula (Ia) can be taken into account. Consequently, the subject matter of claim 2 also is unclear.

<With respect to claim 19>

There in no statement concerning a definition of the "compound represented by the formula (I)" given in claim 19. The "compound represented by the formula (I)" cannot be clearly grasped as to what chemical structure the compound has.

Therefore, the subject matter of claim 19 is unclear.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9713771 A
- WO 9802437 A
- WO 9640142 A
- WO 0044728 A
- US 20040242604 A
- WO 0202552 A
- WO 0198277 A
- WO 03049740 A
- WO 03050108 A
- WO 03053446 A
- WO 98003648 A
- WO 01077107 A
- WO 03031442 A
- WO 2004109393 A


### Non-patent literature cited in the description


- *Chemical & Pharmaceutical Bulletin,* 1958, vol. 6, 352-355
- *Liebigs Annalen der Chemie,* 1986, 1255-1261
- Molecular Design. Development of Medicine. Hirokawa Shoten, 1990, vol. 7, 163-198
- **MANIATIS et al.** Molecular Cloning. Cold Spring Harbor Laboratory, 1989
- **SKEHAN et al.** Journal of the National Cancer Institute. 1990, vol. 82, 1107-1112

SEQUENCE LISTING

<110> TAKEDA PHARMACEUTICAL COMPANY LIMITED

<120> Thiazolopyrimidine derivatives

<130> 09746

<150> JP2004-109393
<151> 2004-04-01

<160> 4

<170> PatentIn version 3.1

<210> 1
<211> 66
<212> DNA
<213> Artificial

<220>
<223> primer for cloning human HER2 gene

<400> 1
aattaagtcg acatggacta caaagacgat gacgacaagc gacggcagca gaagatccgg 60

aagtac 66

<210> 2
<211> 42
<212> DNA
<213> Artificial

<220>
<223> primer for cloning human HER2 gene

<400> 2
aattaagcat gctcacactg gcacgtccag acccaggtac tc 42

<210> 3
<211> 66
<212> DNA
<213> Artificial

<220>
<223> primer for cloning human EGFR gene

<400> 3
aattaagtcg acatggacta caaagacgat gacgaccgaa ggcgccacat cgttcggaag 60

cgcacg 66

<210> 4
<211> 42
<212> DNA
<213> Artificial

<220>
<223> primer for cloning human EGFR gene

<400> 4
aattaagcat gctcatgctc caataaattc actgctttgt gg 42